# EUROPEAN PATENT SPECIFICATION

(11) **EP 1 109 908 B1**
(45) Date of publication and mention of the grant of the patent: **17.08.2005**
(21) Application number: 99969119.9
(22) Date of filing: 10.09.1999
(51) Int. Cl.: C12N 15/12, C07K 14/47, C12N 9/10, C07K 16/18, C07K 16/40, G01N 33/53, C12Q 1/68, A61K 38/17, A61K 38/22, A61P 3/00

(54) **METHODS FOR DETERMINING COMPOUNDS FOR MODULATING THE BODY WEIGHT**
METHODEN ZUR BESTIMMUNG VON KOMPONENTEN ZUR MODULATION DES KÖRPERGEWICHTS
METHODES DE LA DETERMINATION DES COMPOSES POUR LA MODULATION DU POIDS CORPOREL

(30) Priority: 10.09.1998 US 150857; 29.10.1998 US 106378 P; 19.11.1998 US 195896; 15.04.1999 US 292228
(43) Date of publication of application: 27.06.2001
(73) Proprietor: Millennium Pharmaceuticals, Inc., Cambridge, MA 02139-4815 (US)
(72) Inventor: WHITE, David, Braintree, MA 02184 (US); ZHOU, Jianghong, Chestnut Hill, MA 02467 (US); TARTAGLIA, Louis, A., Newton, MA 02459 (US)
(74) Representative: Lock, Graham James
(86) International application number: PCT/US1999/020722
(87) International publication number: WO 2000/015826

(56) References cited:
- ZHOU ET AL.: "Induction by leptin of uncoupling protein-2 and enzymes of fatty acid oxidation" PROC. NATL. ACAD. SCI USA, vol. 94, June 1997 (1997-06), pages 6386-6390, XP002137954

### SEQUENCE LISTING

<110> Millennium Pharmaceuticals, Inc.
<120> LEPTIN INDUCED GENES
<130> 07334/126WO1
<140> PCT US99/20722
   <141> 1999-09-10
<150> US 09/292,228
   <151> 1999-04-15
<160> 17
<170> FastSEQ for Windows Version 3.0
<210> 1
   <211> 1196
   <212> DNA
   <213> Mus musculus
<400> 1
   agatgagtgt ggggcgtcga agagtcaagt tgctgggcat cctgatgatg gcaaatgtct     60
   tcatttattt gattgtggaa gtctccaaaa acagtagcca agacaaaaat ggaaagggag    120
   gagtaataat cccgaaagag aagttctgga agccacccag cactccccgg gcatactgga    180
   acagggaaca ggagaagctg aacaggtggt acaatcccat cttgaacagg gtggccaatc    240
   agacagggga gctagccaca tctccaaaca caagtcacct gagctattgt gaaccagact    300
   cgacggtcat gacagctgtg acagatttta ataatctgcc ggacagattt aaagactttc    360
   tcttgtattt gagatgccgg aattactcgc tgcttataga tcaaccgaag aaatgtgcaa    420
   agaagccctt cttactattg gcgataaagt ccctcattcc acattttgcc agaaggcaag    480
   caattcggga gtcttggggc cgagaaacca acgtagggaa ccagacagta gtgagggtct    540
   tcctgttggg caagacaccc ccagaggaca accaccctga cctttcggac atgcttaagt    600
   ttgagagtga caagcaccag gacatcctca tgtggaacta tagagacaca ttcttcaacc    660
   tgtccctgaa ggaagtgctg tttcttaggt gggtgagcac ttcctgtcca gacgcagagt    720
   ttgtcttcaa gggcgatgat gacgtgtttg tgaacaccca tcacatcctt aattacttga    780
   atagcttatc caagagcaaa gccaaagact tgttcatagg tgacgtgatc cacaatgctg    840
   ggcctcaccg ggataagaaa ctgaagtact acatcccaga agtcttctac accggcgtct    900
   acccaccgta tgccgggggt ggtggattcc tgtactccgg cccccttgcc ttgaggctgt     960
   acagtgcgac tagccgggtc catctctacc ctattgatga tgtttatacg ggaatgtgcc   1020
   ttcagaaact gggccttgtt ccagagaagc acaaaggctt caggacattt gatattgaag   1080
   agaaaaataa gaaaaatatt tgttcctata tagacctaat gttagtacat agcagaaaac   1140
   ctcaagagat gattgatatc tggtctcagt tgcaaagtcc taatttaaaa tgctga       1196
<210> 2
   <211> 397
   <212> PRT
   <213> Mus musculus
<400> 2
   Met Ser Val Gly Arg Arg Arg Val Lys Leu Leu Gly Ile Leu Met Met
   1               5                   10                  15
   Ala Asn Val Phe Ile Tyr Leu Ile Val Glu Val Ser Lys Asn Ser Ser
               20                  25                  30
   Gln Asp Lys Asn Gly Lys Gly Gly Val Ile Ile Pro Lys Glu Lys Phe
           35                  40                  45
   Trp Lys Pro Pro Ser Thr Pro Arg Ala Tyr Trp Asn Arg Glu Gln Glu
       50                  55                  60
   Lys Leu Asn Arg Trp Tyr Asn Pro Ile Leu Asn Arg Val Ala Asn Gln
   65                  70                  75                  80
   Thr Gly Glu Leu Ala Thr Ser Pro Asn Thr Ser His Leu Ser Tyr Cys
                   85                  90                  95
   Glu Pro Asp Ser Thr Val Met Thr Ala Val Thr Asp Phe Asn Asn Leu
               100                 105                 110
   Pro Asp Arg Phe Lys Asp Phe Leu Leu Tyr Leu Arg Cys Arg Asn Tyr
           115                 120                 125
   Ser Leu Leu Ile Asp Gln Pro Lys Lys Cys Ala Lys Lys Pro Phe Leu
       130                 135                 140
   Leu Leu Ala Ile Lys Ser Leu Ile Pro His Phe Ala Arg Arg Gln Ala
   145                 150                 155                 160
   Ile Arg Glu Ser Trp Gly Arg Glu Thr Asn Val Gly Asn Gln Thr Val
                   165                 170                 175
   Val Arg Val Phe Leu Leu Gly Lys Thr Pro Pro Glu Asp Asn His Pro
               180                 185                 190
   Asp Leu Ser Asp Met Leu Lys Phe Glu Ser Asp Lys His Gln Asp Ile
           195                 200                 205
   Leu Met Trp Asn Tyr Arg Asp Thr Phe Phe Asn Leu Ser Leu Lys Glu
       210                 215                 220
   Val Leu Phe Leu Arg Trp Val Ser Thr Ser Cys Pro Asp Ala Glu Phe
   225                 230                 235                 240
   Val Phe Lys Gly Asp Asp Asp Val Phe Val Asn Thr His His Ile Leu
                   245                 250                 255
   Asn Tyr Leu Asn Ser Leu Ser Lys Ser Lys Ala Lys Asp Leu Phe Ile
               260                 265                 270
   Gly Asp Val Ile His Asn Ala Gly Pro His Arg Asp Lys Lys Leu Lys
           275                 280                 285
   Tyr Tyr Ile Pro Glu Val Phe Tyr Thr Gly Val Tyr Pro Pro Tyr Ala
       290                 295                 300
   Gly Gly Gly Gly Phe Leu Tyr Ser Gly Pro Ala Leu Leu Arg Leu Tyr
   305                 310                 315                 320
   Ser Ala Thr Ser Arg Val His Leu Tyr Pro Ile Asp Asp Val Tyr Thr
                   325                 330                 335
   Gly Met Cys Leu Gln Lys Leu Gly Leu Val Pro Glu Lys His Lys Gly
               340                 345                 350
   Phe Arg Thr Phe Asp Ile Glu Glu Lys Asn Lys Lys Asn Ile Cys Ser
           355                 360                 365
   Tyr Ile Asp Leu Met Leu Val His Ser Arg Lys Pro Gln Glu Met Ile
       370                 375                 380
   Asp Ile Trp Ser Gln Leu Gln Ser Pro Asn Leu Lys Cys
   385                 390                 395
<210> 3
   <211> 1191
   <212> DNA
   <213> Mus musculus
<400> 3
   atgagtgtgg ggcgtcgaag agtcaagttg ctgggcatcc tgatgatggc aaatgtcttc     60
   atttatttga ttgtggaagt ctccaaaaac agtagccaag acaaaaatgg aaagggagga    120
   gtaataatcc cgaaagagaa gttctggaag ccacccagca ctccccgggc atactggaac     180
   agggaacagg agaagctgaa caggtggtac aatcccatct tgaacagggt ggccaatcag    240
   acaggggagc tagccacatc tccaaacaca agtcacctga gctattgtga accagactcg    300
   acggtcatga cagctgtgac agattttaat aatctgccgg acagatttaa agactttctc     360
   ttgtatttga gatgccggaa ttactcgctg cttatagatc aaccgaagaa atgtgcaaag    420
   aagcccttct tactattggc gataaagtcc ctcattccac attttgccag aaggcaagca    480
   attcgggagt cttggggccg agaaaccaac gtagggaacc agacagtagt gagggtcttc    540
   ctgttgggca agacaccccc agaggacaac caccctgacc tttcggacat gcttaagttt    600
   gagagtgaca agcaccagga catcctcatg tggaactata gagacacatt cttcaacctg    660
   tccctgaagg aagtgctgtt tcttaggtgg gtgagcactt cctgtccaga cgcagagttt    720
   gtcttcaagg gcgatgatga cgtgtttgtg aacacccatc acatccttaa ttacttgaat    780
   agcttatcca agagcaaagc caaagacttg ttcataggtg acgtgatcca caatgctggg    840
   cctcaccggg ataagaaact gaagtactac atcccagaag tcttctacac cggcgtctac    900
   ccaccgtatg ccgggggtgg tggattcctg tactccggcc cccttgcctt gaggctgtac    960
   agtgcgacta gccgggtcca tctctaccct attgatgatg tttatacggg aatgtgcctt   1020
   cagaaactgg gccttgttcc agagaagcac aaaggcttca ggacatttga tattgaagag   1080
   aaaaataaga aaaatatttg ttcctatata gacctaatgt tagtacatag cagaaaacct   1140
   caagagatga ttgatatctg gtctcagttg caaagtccta atttaaaatg c            1191
<210> 4
   <211> 365
   <212> PRT
   <213> Mus musculus
<400> 4
   Gln Asp Lys Asn Gly Lys Gly Gly Val Ile Ile Pro Lys Glu Lys Phe
   1               5                   10                  15
   Trp Lys Pro Pro Ser Thr Pro Arg Ala Tyr Trp Asn Arg Glu Gln Glu
               20                  25                  30
   Lys Leu Asn Arg Trp Tyr Asn Pro Ile Leu Asn Arg Val Ala Asn Gln
           35                  40                  45
   Thr Gly Glu Leu Ala Thr Ser Pro Asn Thr Ser His Leu Ser Tyr Cys
       50                  55                  60
   Glu Pro Asp Ser Thr Val Met Thr Ala Val Thr Asp Phe Asn Asn Leu
   65                  70                  75                  80
   Pro Asp Arg Phe Lys Asp Phe Leu Leu Tyr Leu Arg Cys Arg Asn Tyr
                   85                  90                  95
   Ser Leu Leu Ile Asp Gln Pro Lys Lys Cys Ala Lys Lys Pro Phe Leu
               100                 105                 110
   Leu Leu Ala Ile Lys Ser Leu Ile Pro His Phe Ala Arg Arg Gln Ala
           115                 120                 125
   Ile Arg Glu Ser Trp Gly Arg Glu Thr Asn Val Gly Asn Gln Thr Val
       130                 135                 140
   Val Arg Val Phe Leu Leu Gly Lys Thr Pro Pro Glu Asp Asn His Pro
   145                 150                 155                 160
   Asp Leu Ser Asp Met Leu Lys Phe Glu Ser Asp Lys His Gln Asp Ile
                   165                 170                 175
   Leu Met Trp Asn Tyr Arg Asp Thr Phe Phe Asn Leu Ser Leu Lys Glu
               180                 185                 190
   Val Leu Phe Leu Arg Trp Val Ser Thr Ser Cys Pro Asp Ala Glu Phe
           195                 200                 205
   Val Phe Lys Gly Asp Asp Asp Val Phe Val Asn Thr His His Ile Leu
       210                 215                 220
   Asn Tyr Leu Asn Ser Leu Ser Lys Ser Lys Ala Lys Asp Leu Phe Ile
   225                 230                 235                 240
   Gly Asp Val Ile His Asn Ala Gly Pro His Arg Asp Lys Lys Leu Lys
                   245                 250                 255
   Tyr Tyr Ile Pro Glu Val Phe Tyr Thr Gly Val Tyr Pro Pro Tyr Ala
               260                 265                 270
   Gly Gly Gly Gly Phe Leu Tyr Ser Gly Pro Ala Leu Leu Arg Leu Tyr
           275                 280                 285
   Ser Ala Thr Ser Arg Val His Leu Tyr Pro Ile Asp Asp Val Tyr Thr
       290                 295                 300
   Gly Met Cys Leu Gln Lys Leu Gly Leu Val Pro Glu Lys His Lys Gly
   305                 310                 315                 320
   Phe Arg Thr Phe Asp Ile Glu Glu Lys Asn Lys Lys Asn Ile Cys Ser
                   325                 330                 335
   Tyr Ile Asp Leu Met Leu Val His Ser Arg Lys Pro Gln Glu Met Ile
               340                 345                 350
   Asp Ile Trp Ser Gln Leu Gln Ser Pro Asn Leu Lys Cys
           355                 360                 365
<210> 5
   <211> 1203
   <212> DNA
   <213> Mus musculus
<400> 5
   atgatttgcc cttcagcttt actggttatt ttaagaaatt taatacggga agaaaaaatc      60
   atttctcaag agatcctcaa tttgattgaa ttaaggatga aaaaagggaa tattcagttg     120
   acaaactctg caatcagtga tgcattaaaa gaaatcgata gtagtgtgct caatgttgct     180
   gtcaccgggg agacgggatc agggaagtcc agcttcatca ataccctgag aggcattggg     240
   aatgaagaag aaggtgcagc taaaactggg gtggtggagg taaccatgga aagacatcca     300
   tacaaacacc ccaatatacc caatgtggtt ttttgggacc tgcctgggat tggaagcaca     360
   aatttcccac caaacactta cctggagaaa atgaagttct atgagtacga tttcttcatt     420
   attatttcgg ccacacgctt caagaaaaat gatatagaca ttgccaaagc aatcagcatg     480
   atgaagaagg aattctactt cgtgagaacc aaggtggact ctgacataac aaatgaagca     540
   gatggcaaac ctcaaacctt tgacaaagaa aaggtcctgc aggacatccg ccttaactgt     600
   gtgaacacct ttagggagaa tggcattgct gagccaccaa tcttcctgct ctctaacaaa     660
   aatgtttgtc actatgactt ccccgtcctg atggacaagc tgataagtga cctccctatc     720
   tacaggagac acaattttat ggtctcctta cccaatatca cagattcagt cattgaaaag     780
   aagcggcaat ttctgaagca raggatttgg ctggaaggat ttgctgctga cctagtgaat     840
   atcatccctt ctctgacctt tctcttggac agtgatttgg agactctgaa gaaaagcatg     900
   aaattctacc gcactgtgtt tggagtggat gaaacatctt tgcagagatt agctagggac     960
   tgggaaatag aggtggatca ggtggaggcc atgataaaat ctcctgctgt gttcaaacct    1020
   acagatgaag aaacaataca agaaaggctt tcaagatata ttcaggagtt ctgtttggct    1080
   aatgggtact tacttcctaa aaatagtttt cttaaagaaa tattttacct gaaatattat    1140
   ttccttgaca tggtgactga ggatgctaaa actcttctta aagagatatg tttaagaaac    1200
   tag                                                                   1203
<210> 6
   <211> 400
   <212> PRT
   <213> Mus musculus
<400> 6
   Met Ile Cys Pro Ser Ala Leu Leu Val Ile Leu Arg Asn Leu Ile Arg
   1               5                   10                  15
   Glu Glu Lys Ile Ile Ser Gln Glu Ile Leu Asn Leu Ile Glu Leu Arg
               20                  25                  30
   Met Lys Lys Gly Asn Ile Gln Leu Thr Asn Ser Ala Ile Ser Asp Ala
           35                  40                  45
   Leu Lys Glu Ile Asp Ser Ser Val Leu Asn Val Ala Val Thr Gly Glu
       50                  55                  60
   Thr Gly Ser Gly Lys Ser Ser Phe Ile Asn Thr Leu Arg Gly Ile Gly
   65                  70                  75                  80
   Asn Glu Glu Glu Gly Ala Ala Lys Thr Gly Val Val Glu Val Thr Met
                   85                  90                  95
   Glu Arg His Pro Tyr Lys His Pro Asn Ile Pro Asn Val Val Phe Trp
               100                 105                 110
   Asp Leu Pro Gly Ile Gly Ser Thr Asn Phe Pro Pro Asn Thr Tyr Leu
           115                 120                 125
   Glu Lys Met Lys Phe Tyr Glu Tyr Asp Phe Phe Ile Ile Ile Ser Ala
       130                 135                 140
   Thr Arg Phe Lys Lys Asn Asp Ile Asp Ile Ala Lys Ala Ile Ser Met
   145                 150                 155                 160
   Met Lys Lys Glu Phe Tyr Phe Val Arg Thr Lys Val Asp Ser Asp Ile
                   165                 170                 175
   Thr Asn Glu Ala Asp Gly Lys Pro Gln Thr Phe Asp Lys Glu Lys Val
               180                 185                 190
   Leu Gln Asp Ile Arg Leu Asn Cys Val Asn Thr Phe Arg Glu Asn Gly
           195                 200                 205
   Ile Ala Glu Pro Pro Ile Phe Leu Leu Ser Asn Lys Asn Val Cys His
       210                 215                 220
   Tyr Asp Phe Pro Val Leu Met Asp Lys Leu Ile Ser Asp Leu Pro Ile
   225                 230                 235                 240
   Tyr Arg Arg His Asn Phe Met Val Ser Leu Pro Asn Ile Thr Asp Ser
                   245                 250                 255
   Val Ile Glu Lys Lys Arg Gln Phe Leu Lys Gln Arg Ile Trp Leu Glu
               260                 265                 270
   Gly Phe Ala Ala Asp Leu Val Asn Ile Ile Pro Ser Leu Thr Phe Leu
           275                 280                 285
   Leu Asp Ser Asp Leu Glu Thr Leu Lys Lys Ser Met Lys Phe Tyr Arg
       290                 295                 300
   Thr Val Phe Gly Val Asp Glu Thr Ser Leu Gln Arg Leu Ala Arg Asp
   305                 310                 315                 320
   Trp Glu Ile Glu Val Asp Gln Val Glu Ala Met Ile Lys Ser Pro Ala
                   325                 330                 335
   Val Phe Lys Pro Thr Asp Glu Glu Thr Ile Gln Glu Arg Leu Ser Arg
               340                 345                 350
   Tyr Ile Gln Glu Phe Cys Leu Ala Asn Gly Tyr Leu Leu Pro Lys Asn
           355                 360                 365
   Ser Phe Leu Lys Glu Ile Phe Tyr Leu Lys Tyr Tyr Phe Leu Asp Met
       370                 375                 380
   Val Thr Glu Asp Ala Lys Thr Leu Leu Lys Glu Ile Cys Leu Arg Asn
   385                 390                 395                 400
<210> 7
   <211> 1200
   <212> DNA
   <213> Mus musculus
<400> 7
   atgatttgcc cttcagcttt actggttatt ttaagaaatt taatacggga agaaaaaatc      60
   atttctcaag agatcctcaa tttgattgaa ttaaggatga aaaaagggaa tattcagttg     120
   acaaactctg caatcagtga tgcattaaaa gaaatcgata gtagtgtgct caatgttgct     180
   gtcaccgggg agacgggatc agggaagtcc agcttcatca ataccctgag aggcattggg     240
   aatgaagaag aaggtgcagc taaaactggg gtggtggagg taaccatgga aagacatcca     300
   tacaaacacc ccaatatacc caatgtggtt ttttgggacc tgcctgggat tggaagcaca     360
   aatttcccac caaacactta cctggagaaa atgaagttct atgagtacga tttcttcatt     420
   attatttcgg ccacacgctt caagaaaaat gatatagaca ttgccaaagc aatcagcatg     480
   atgaagaagg aattctactt cgtgagaacc aaggtggact ctgacataac aaatgaagca     540
   gatggcaaac ctcaaacctt tgacaaagaa aaggtcctgc aggacatccg ccttaactgt     600
   gtgaacacct ttagggagaa tggcattgct gagccaccaa tcttcctgct ctctaacaaa     660
   aatgtttgtc actatgactt ccccgtcctg atggacaagc tgataagtga cctccctatc     720
   tacaggagac acaattttat ggtctcctta cccaatatca cagattcagt cattgaaaag     780
   aagcggcaat ttctgaagca raggatttgg ctggaaggat ttgctgctga cctagtgaat     840
   atcatccctt ctctgacctt tctcttggac agtgatttgg agactctgaa gaaaagcatg     900
   aaattctacc gcactgtgtt tggagtggat gaaacatctt tgcagagatt agctagggac     960
   tgggaaatag aggtggatca ggtggaggcc atgataaaat ctcctgctgt gttcaaacct    1020
   acagatgaag aaacaataca agaaaggctt tcaagatata ttcaggagtt ctgtttggct    1080
   aatgggtact tacttcctaa aaatagtttt cttaaagaaa tattttacct gaaatattat    1140
   ttccttgaca tggtgactga ggatgctaaa actcttctta aagagatatg tttaagaaac    1200
<210> 8
   <211> 326
   <212> PRT
   <213> Mus musculus
<400> 8
   Met Ala Ser Lys Val Ser Cys Leu Tyr Val Leu Ser Val Val Cys Trp
   1               5                   10                  15
   Ala Ser Ala Leu Trp Tyr Leu Ser Ile Thr Arg Pro Thr Ser Ser Tyr
               20                  25                  30
   Thr Gly Ser Lys Pro Phe Ser His Leu Thr Val Ala Arg Lys Asn Phe
           35                  40                  45
   Thr Phe Gly Asn Ile Arg Thr Arg Pro Ile Asn Pro His Ser Phe Glu
       50                  55                  60
   Phe Leu Ile Asn Glu Pro Asn Lys Cys Glu Lys Asn Ile Pro Phe Leu
   65                  70                  75                  80
   Val Ile Leu Ile Ser Thr Thr His Lys Glu Phe Asp Ala Arg Gln Ala
                   85                  90                  95
   Ile Arg Glu Thr Trp Gly Asp Glu Asn Asn Phe Lys Gly Ile Lys Ile
               100                 105                 110
   Ala Thr Leu Phe Leu Leu Gly Lys Asn Ala Asp Pro Val Leu Asn Gln
           115                 120                 125
   Met Val Glu Gln Glu Ser Gln Ile Phe His Asp Ile Ile Val Glu Asp
       130                 135                 140
   Phe Ile Asp Ser Tyr His Asn Leu Thr Leu Lys Thr Leu Met Gly Met
   145                 150                 155                 160
   Arg Trp Val Ala Thr Phe Cys Ser Lys Ala Lys Tyr Val Met Lys Thr
                   165                 170                 175
   Asp Ser Asp Ile Phe Val Asn Met Asp Asn Leu Ile Tyr Lys Leu Leu
               180                 185                 190
   Lys Pro Ser Thr Lys Pro Arg Arg Arg Tyr Phe Thr Gly Tyr Val Ile
           195                 200                 205
   Asn Gly Gly Pro Ile Arg Asp Val Arg Ser Lys Trp Tyr Met Pro Arg
       210                 215                 220
   Asp Leu Tyr Pro Asp Ser Asn Tyr Pro Pro Phe Cys Ser Gly Thr Gly
   225                 230                 235                 240
   Tyr Ile Phe Ser Ala Asp Val Ala Glu Leu Ile Tyr Lys Thr Ser Leu
                   245                 250                 255
   His Thr Arg Leu Leu His Leu Glu Asp Val Tyr Val Gly Leu Cys Leu
               260                 265                 270
   Arg Lys Leu Gly Ile His Pro Phe Gln Asn Ser Gly Phe Asn His Trp
           275                 280                 285
   Lys Met Ala Tyr Ser Leu Cys Arg Tyr Arg Arg Val Ile Thr Val His
       290                 295                 300
   Gln Ile Ser Pro Glu Glu Met His Arg Ile Trp Asn Asp Met Ser Ser
   305                 310                 315                 320
   Lys Lys His Leu Arg Cys
                   325
<210> 9
   <211> 331
   <212> PRT
   <213> Mus musculus
<400> 9
   Met Ala Pro Ala Val Leu Thr Ala Leu Pro Asn Arg Met Ser Leu Arg
   1               5                   10                  15
   Ser Leu Lys Trp Ser Leu Leu Leu Leu Ser Leu Leu Ser Phe Leu Val
               20                  25                  30
   Ile Trp Tyr Leu Ser Leu Pro His Tyr Asn Val Ile Glu Arg Val Asn
           35                  40                  45
   Trp Met Tyr Phe Tyr Glu Tyr Glu Pro Ile Tyr Arg Gln Asp Phe Arg
       50                  55                  60
   Phe Thr Leu Arg Glu His Ser Asn Cys Ser His Gln Asn Pro Phe Leu
   65                  70                  75                  80
   Val Ile Leu Val Thr Ser Arg Pro Ser Asp Val Lys Ala Arg Gln Ala
                   85                  90                  95
   Ile Arg Val Thr Trp Gly Glu Lys Lys Ser Trp Trp Gly Tyr Glu Val
               100                 105                 110
   Leu Thr Phe Phe Leu Leu Gly Gln Gln Ala Glu Arg Glu Asp Lys Thr
           115                 120                 125
   Leu Ala Leu Ser Leu Glu Asp Glu His Val Leu Tyr Gly Asp Ile Ile
       130                 135                 140
   Arg Gln Asp Phe Leu Asp Thr Tyr Asn Asn Leu Thr Leu Lys Thr Ile
   145                 150                 155                 160
   Met Ala Phe Arg Trp Val Met Glu Phe Cys Pro Asn Ala Lys Tyr Ile
                   165                 170                 175
   Met Lys Thr Asp Thr Asp Val Phe Ile Asn Thr Gly Asn Leu Val Lys
               180                 185                 190
   Tyr Leu Leu Asn Leu Asn His Ser Glu Lys Phe Phe Thr Gly Tyr Pro
           195                 200                 205
   Leu Ile Asp Asn Tyr Ser Tyr Arg Gly Phe Phe His Lys Asn His Ile
       210                 215                 220
   Ser Tyr Gln Glu Tyr Pro Phe Lys Val Phe Pro Pro Tyr Cys Ser Gly
   225                 230                 235                 240
   Leu Gly Tyr Ile Met Ser Gly Asp Leu Val Pro Arg Val Tyr Glu Met
                   245                 250                 255
   Met Ser His Val Lys Pro Ile Lys Phe Glu Asp Val Tyr Val Gly Ile
               260                 265                 270
   Cys Leu Asn Leu Leu Lys Val Asp Ile His Ile Pro Glu Asp Thr Asn
           275                 280                 285
   Leu Phe Phe Leu Tyr Arg Ile His Leu Asp Val Cys Gln Leu Arg Arg
       290                 295                 300
   Val Ile Ala Ala His Gly Phe Ser Ser Lys Glu Ile Ile Thr Phe Trp
   305                 310                 315                 320
   Gln Val Met Leu Arg Asn Thr Thr Cys His Tyr
                   325                 330
<210> 10
   <211> 325
   <212> PRT
   <213> Drosophilea melonogaster
<400> 10
   Met Gln Ser Lys His Arg Lys Leu Leu Leu Arg Cys Leu Leu Val Leu
   1               5                   10                  15
   Pro Leu Ile Leu Leu Val Asp Tyr Cys Gly Leu Leu Thr His Leu His
               20                  25                  30
   Glu Leu Asn Phe Glu Arg His Phe His Tyr Pro Leu Asn Asp Asp Thr
           35                  40                  45
   Gly Ser Gly Ser Ala Ser Ser Gly Leu Asp Lys Phe Ala Tyr Leu Arg
       50                  55                  60
   Val Pro Ser Phe Thr Ala Glu Val Pro Val Asp Gln Pro Ala Arg Leu
   65                  70                  75                  80
   Thr Met Leu Ile Lys Ser Ala Val Gly Asn Ser Arg Arg Arg Glu Ala
                   85                  90                  95
   Ile Arg Arg Thr Trp Gly Tyr Glu Gly Arg Phe Ser Asp Val His Leu
               100                 105                 110
   Arg Arg Val Phe Leu Leu Gly Thr Ala Glu Asp Ser Glu Lys Asp Val
           115                 120                 125
   Ala Trp Glu Ser Arg Glu His Gly Asp Ile Leu Gln Ala Asp Phe Thr
       130                 135                 140
   Asp Ala Tyr Phe Asn Asn Thr Leu Lys Thr Met Leu Gly Met Arg Trp
   145                 150                 155                 160
   Ala Ser Glu Gln Phe Asn Arg Ser Glu Phe Tyr Leu Phe Val Asp Asp
                   165                 170                 175
   Asp Tyr Tyr Val Ser Ala Lys Asn Val Leu Lys Phe Leu Gly Arg Gly
               180                 185                 190
   Arg Gln Ser His Gln Pro Glu Leu Leu Phe Ala Gly His Val Phe Gln
           195                 200                 205
   Thr Ser Pro Leu Arg His Lys Phe Ser Lys Trp Tyr Val Ser Leu Glu
       210                 215                 220
   Glu Tyr Pro Phe Asp Arg Trp Pro Pro Tyr Val Thr Ala Gly Ala Phe
   225                 230                 235                 240
   Ile Leu Ser Gln Lys Ala Leu Arg Gln Leu Tyr Ala Ala Ser Val His
                   245                 250                 255
   Leu Pro Leu Phe Arg Phe Asp Asp Val Tyr Leu Gly Ile Val Ala Leu
               260                 265                 270
   Lys Ala Gly Ile Ser Leu Gln His Cys Asp Asp Phe Arg Phe His Arg
           275                 280                 285
   Pro Ala Tyr Lys Gly Pro Asp Ser Tyr Ser Ser Val Ile Ala Ser His
       290                 295                 300
   Glu Phe Gly Asp Pro Glu Glu Met Thr Arg Val Trp Asn Glu Cys Arg
   305                 310                 315                 320
   Ser Ala Asn Tyr Ala
                   325
<210> 11
   <211> 422
   <212> PRT
   <213> Homo sapien
<400> 11
   Met Leu Gln Trp Arg Arg Arg His Cys Cys Phe Ala Lys Met Thr Trp
   1               5                   10                  15
   Asn Ala Lys Arg Ser Leu Phe Arg Thr His Leu Ile Gly Val Leu Ser
               20                  25                  30
   Leu Val Phe Leu Phe Ala Met Phe Leu Phe Phe Asn His His Asp Trp
           35                  40                  45
   Leu Pro Gly Arg Ala Gly Phe Lys Glu Asn Pro Val Thr Tyr Thr Phe
       50                  55                  60
   Arg Gly Phe Arg Ser Thr Lys Ser Glu Thr Asn His Ser Ser Leu Arg
   65                  70                  75                  80
   Asn Ile Trp Lys Glu Thr Val Pro Gln Thr Leu Arg Pro Gln Thr Ala
                   85                  90                  95
   Thr Asn Ser Asn Asn Thr Asp Leu Ser Pro Gln Gly Val Thr Gly Leu
               100                 105                 110
   Glu Asn Thr Leu Ser Ala Asn Gly Ser Ile Tyr Asn Glu Lys Gly Thr
           115                 120                 125
   Gly His Pro Asn Ser Tyr His Phe Lys Tyr Ile Ile Asn Glu Pro Glu
       130                 135                 140
   Lys Cys Gln Glu Lys Ser Pro Phe Leu Ile Leu Leu Ile Ala Ala Glu
   145                 150                 155                 160
   Pro Gly Gln Ile Glu Ala Arg Arg Ala Ile Arg Gln Thr Trp Gly Asn
                   165                 170                 175
   Glu Ser Leu Ala Pro Gly Ile Gln Ile Thr Arg Ile Phe Leu Leu Gly
               180                 185                 190
   Leu Ser Ile Lys Leu Asn Gly Tyr Leu Gln Arg Ala Ile Leu Glu Glu
           195                 200                 205
   Ser Arg Gln Tyr His Asp Ile Ile Gln Gln Glu Tyr Leu Asp Thr Tyr
       210                 215                 220
   Tyr Asn Leu Thr Ile Lys Thr Leu Met Gly Met Asn Trp Val Ala Thr
   225                 230                 235                 240
   Tyr Cys Pro His Ile Pro Tyr Val Met Lys Thr Asp Ser Asp Met Phe
                   245                 250                 255
   Val Asn Thr Glu Tyr Leu Ile Asn Lys Leu Leu Lys Pro Asp Leu Pro
               260                 265                 270
   Pro Arg His Asn Tyr Phe Thr Gly Tyr Leu Met Arg Gly Tyr Ala Pro
           275                 280                 285
   Asn Arg Asn Lys Asp Ser Lys Trp Tyr Met Pro Pro Asp Leu Tyr Pro
       290                 295                 300
   Ser Glu Arg Tyr Pro Val Phe Cys Ser Gly Thr Gly Tyr Val Phe Ser
   305                 310                 315                 320
   Gly Asp Leu Ala Glu Lys Ile Phe Lys Val Ser Leu Gly Ile Arg Arg
                   325                 330                 335
   Leu His Leu Glu Asp Val Tyr Val Gly Ile Cys Leu Ala Lys Leu Arg
               340                 345                 350
   Ile Asp Pro Val Pro Pro Pro Asn Glu Phe Val Phe Asn His Trp Arg
           355                 360                 365
   Val Ser Tyr Ser Ser Cys Lys Tyr Ser His Leu Ile Thr Ser His Gln
       370                 375                 380
   Phe Gln Pro Ser Glu Leu Ile Lys Tyr Trp Asn His Leu Gln Gln Asn
   385                 390                 395                 400
   Lys His Asn Ala Cys Ala Asn Ala Ala Lys Glu Lys Ala Gly Arg Tyr
                   405                 410                 415
   Arg His Arg Lys Leu His
               420
<210> 12
   <211> 229
   <212> PRT
   <213> Artificial sequence
<220>
   <221> VARIANT
   <222> (1)...(229)
   <223> Xaa = Any Amino Acid
<400> 12
   Met Ala Xaa Arg Arg Lys Val Leu Leu Arg Leu Leu Val Leu Ser Leu
    1               5                  10                  15
   Val Xaa Leu Xaa Xaa Xaa Phe Xaa Phe Leu Xaa His Trp Phe Phe Pro
               20                  25                  30
   Ile Trp Tyr Leu Ser Ile Pro Leu Arg Pro Gln Thr Gly Ser Xaa Ser
           35                  40                  45
   Xaa Ser Xaa Xaa Leu Ser His Leu Tyr Asn Thr Val Xaa Arg Xaa Asn
       50                  55                  60
   Xaa Xaa Phe Asn Asn Xaa Xaa Thr Arg Pro Ile Asn Ser Xaa Xaa Phe
   65                  70                  75                  80
   Glu Phe Leu Ile Asp Glu Pro Xaa Lys Cys Xaa Lys Lys Pro Phe Leu
                   85                  90                  95
   Val Leu Leu Ile Lys Ser Xaa Pro Gly Xaa Phe Xaa Ala Arg Gln Ala
               100                 105                 110
   Ile Arg Glu Thr Trp Gly Xaa Glu Xaa Asn Phe Xaa Gly Ile Xaa Val
           115                 120                 125
   Xaa Arg Val Phe Leu Leu Gly Lys Xaa Ala Glu Xaa Xaa Asp Pro Xaa
       130                 135                 140
   Leu Xaa Xaa Met Val Glu Xaa Glu Ser Arg Xaa His Gly Asp Ile Ile
   145                 150                 155                 160
   Gln Gln Asp Phe Leu Asp Thr Tyr Phe Asn Leu Thr Leu Lys Thr Leu
                   165                 170                 175
   Met Gly Met Arg Trp Val Ala Thr Phe Cys Pro Xaa Ala Glu Tyr Val
               180                 185                 190
   Met Lys Thr Asp Ser Asp Val Phe Val Asn Thr Xaa Asn Leu Leu Asn
           195                 200                 205
   Lys Leu Leu Lys Pro Ser Leu Ser His Arg Xaa Xaa Leu Phe Thr Gly
       210                 215                 220
   Tyr Val Ile Xaa Gly
   225
<210> 13
   <211> 1707
   <212> DNA
   <213> Homo sapiens
<220>
   <221> CDS
   <222> (246)...(1436)
<221> misc_feature
   <222> (1)...(1707)
   <223> n = A,T,C or G
<400> 13
   acgcgtccgc gcagcggcag cggcagcagc ggcaacaagt gccggaggct agcagagcca    60
   agccggagca gtccctgccg ccgacaccgc cgggccgccc gtccggggcg ccgcgcatgg   120
   agcgtgagct gcggcggtcg ccgggctgag ccgcgcggag cggccgggac gtggatgtgg   180
   ccgcgatctc ccgcccttgc ccccgccccg ccgagctgga gctgctcccg gacaagatat   240
   gagaa atg agt gtt gga cgt cga aga ata aag ttg ttg ggt atc ctg atg   290
         Met Ser Val Gly Arg Arg Arg Ile Lys Leu Leu Gly Ile Leu Met
           1               5                  10                  15
   atg gca aat gtc ttc att tat ttt att atg gaa gtc tcc aaa agc agt     338
   Met Ala Asn Val Phe Ile Tyr Phe Ile Met Glu Val Ser Lys Ser Ser
                   20                  25                  30
   agc caa gaa aaa aat gga aaa ggg gaa gta ata ata ccc aaa gag aag     386
   Ser Gln Glu Lys Asn Gly Lys Gly Glu Val Ile Ile Pro Lys Glu Lys
               35                  40                  45
   ttc tgg aag ata tct acc cct ccc gag gca tac tgg aac cga gag caa     434
   Phe Trp Lys Ile Ser Thr Pro Pro Glu Ala Tyr Trp Asn Arg Glu Gln
           50                  55                  60
   gag aag ctg aac cgg cag tac aac ccc atc ctg agc atg ctg acc aac     482
   Glu Lys Leu Asn Arg Gln Tyr Asn Pro Ile Leu Ser Met Leu Thr Asn
       65                  70                  75
   cag acg ggg gag gcg ggc agg ctc tcc aat ata agc cat ctg aac tac     530
   Gln Thr Gly Glu Ala Gly Arg Leu Ser Asn Ile Ser His Leu Asn Tyr
   80                  85                  90                  95
   tgc gaa cct gac ctg agg gtc acg tcg gtg gtt acg ggt ttt aac aac     578
   Cys Glu Pro Asp Leu Arg Val Thr Ser Val Val Thr Gly Phe Asn Asn
                   100                 105                 110
   ttg ccg gac aga ttt aaa gac ttt ctg ctg tat ttg aga tgc cgc aat     626
   Leu Pro Asp Arg Phe Lys Asp Phe Leu Leu Tyr Leu Arg Cys Arg Asn
               115                 120                 125
   tat tca ctg ctt ata gat cag ccg gat aag tgt gca aag aaa cct ttc     674
   Tyr Ser Leu Leu Ile Asp Gln Pro Asp Lys Cys Ala Lys Lys Pro Phe
           130                 135                 140
   ttg ttg ctg gcg att aag tcc ctc act cca cat ttt gcc aga agg caa      722
   Leu Leu Leu Ala Ile Lys Ser Leu Thr Pro His Phe Ala Arg Arg Gln
       145                 150                 155
   gca atc cgg gaa tcc tgg ggc caa gaa agc aac gca ggg aac caa acg      770
   Ala Ile Arg Glu Ser Trp Gly Gln Glu Ser Asn Ala Gly Asn Gln Thr
   160                 165                 170                 175
   gtg gtg cga gtc ttc ctg ctg ggc cag aca ccc cca gag gac aac cac      818
   Val Val Arg Val Phe Leu Leu Gly Gln Thr Pro Pro Glu Asp Asn His
                   180                 185                 190
   ccc gac ctt tca gat atg ctg aaa ttt gag agt gag aag cac caa gac      866
   Pro Asp Leu Ser Asp Met Leu Lys Phe Glu Ser Glu Lys His Gln Asp
               195                 200                 205
   att ctt atg tgg aac tac aga gac act ttc ttc aac ttg tct ctg aag      914
   Ile Leu Met Trp Asn Tyr Arg Asp Thr Phe Phe Asn Leu Ser Leu Lys
           210                 215                 220
   gaa gtg ctg ttt ctc agg tgg gta agt act tcc tgc cca gac act gag      962
   Glu Val Leu Phe Leu Arg Trp Val Ser Thr Ser Cys Pro Asp Thr Glu
       225                 230                 235
   ttt gtt ttc aag ggc gat gac gat gtt ttt gtg aac acc cat cac atc     1010
   Phe Val Phe Lys Gly Asp Asp Asp Val Phe Val Asn Thr His His Ile
   240                 245                 250                 255
   ctg aat tac ttg aat agt tta tcc aag acc aaa gcc aaa gat ctc ttc     1058
   Leu Asn Tyr Leu Asn Ser Leu Ser Lys Thr Lys Ala Lys Asp Leu Phe
                   260                 265                 270
   ata ggt gat gtg atc cac aat gct gga cct cat cgg gat aag aag ctg     1106
   Ile Gly Asp Val Ile His Asn Ala Gly Pro His Arg Asp Lys Lys Leu
               275                 280                 285
   aag tac tac atc cca gaa gtt gtt tac tct ggc ctc tac cca ccc tat     1154
   Lys Tyr Tyr Ile Pro Glu Val Val Tyr Ser Gly Leu Tyr Pro Pro Tyr
           290                 295                 300
   gca ggg gga ggg ggg ttc ctc tac tcc ggc cac ctg gcc ctg agg ctg     1202
   Ala Gly Gly Gly Gly Phe Leu Tyr Ser Gly His Leu Ala Leu Arg Leu
       305                 310                 315
   tac cat atc act gac cag gtc cat ctc tac ccc att gat gac gtt tat     1250
   Tyr His Ile Thr Asp Gln Val His Leu Tyr Pro Ile Asp Asp Val Tyr
   320                 325                 330                 335
   act gga atg tgc ctt cag aaa ctc ggc ctc gtt cca gag aaa cac aaa     1298
   Thr Gly Met Cys Leu Gln Lys Leu Gly Leu Val Pro Glu Lys His Lys
                   340                 345                 350
   ggc ttc agg aca ttt gat atc gag gag aaa aac aaa aat aac atc tgc     1346
   Gly Phe Arg Thr Phe Asp Ile Glu Glu Lys Asn Lys Asn Asn Ile Cys
               355                 360                 365
   tcc tat gta gat ctg atg tta gta cat agt aga aaa cct caa gag atg     1394
   Ser Tyr Val Asp Leu Met Leu Val His Ser Arg Lys Pro Gln Glu Met
           370                 375                 380
   att gat att tgg tct cag ttg cag agt gct cat tta aaa tgc             1436
   Ile Asp Ile Trp Ser Gln Leu Gln Ser Ala His Leu Lys Cys
       385                 390                 395
   taaaatagat acaaactcaa tttkgsatwg raaggggtwt tttgratwgg ycccatgttg   1496
   gggtctcaca ttagagtaat ttctatttna ancatgaaat tgcctttatg agtgataccc   1556
   atttanggcc tctaancctt catttgnact cacgtgaaga agggaaagcg ggagaaggta   1616
   atttntttat ggtgaatggc aggatattgg tctgacttac cgntagggga ntttaaaact   1676
   ggnccttttt gaatctgttt ggatggccct t                                     1707
<210> 14
   <211> 397
   <212> PRT
   <213> Homo sapiens
<400> 14
   Met Ser Val Gly Arg Arg Arg Ile Lys Leu Leu Gly Ile Leu Met Met
   1               5                   10                  15
   Ala Asn Val Phe Ile Tyr Phe Ile Met Glu Val Ser Lys Ser Ser Ser
               20                  25                  30
   Gln Glu Lys Asn Gly Lys Gly Glu Val Ile Ile Pro Lys Glu Lys Phe
           35                  40                  45
   Trp Lys Ile Ser Thr Pro Pro Glu Ala Tyr Trp Asn Arg Glu Gln Glu
       50                  55                  60
   Lys Leu Asn Arg Gln Tyr Asn Pro Ile Leu Ser Met Leu Thr Asn Gln
   65                  70                  75                  80
   Thr Gly Glu Ala Gly Arg Leu Ser Asn Ile Ser His Leu Asn Tyr Cys
                   85                  90                  95
   Glu Pro Asp Leu Arg Val Thr Ser Val Val Thr Gly Phe Asn Asn Leu
               100                 105                 110
   Pro Asp Arg Phe Lys Asp Phe Leu Leu Tyr Leu Arg Cys Arg Asn Tyr
           115                 120                 125
   Ser Leu Leu Ile Asp Gln Pro Asp Lys Cys Ala Lys Lys Pro Phe Leu
       130                 135                 140
   Leu Leu Ala Ile Lys Ser Leu Thr Pro His Phe Ala Arg Arg Gln Ala
   145                 150                 155                 160
   Ile Arg Glu Ser Trp Gly Gln Glu Ser Asn Ala Gly Asn Gln Thr Val
                   165                 170                 175
   Val Arg Val Phe Leu Leu Gly Gln Thr Pro Pro Glu Asp Asn His Pro
               180                 185                 190
   Asp Leu Ser Asp Met Leu Lys Phe Glu Ser Glu Lys His Gln Asp Ile
           195                 200                 205
   Leu Met Trp Asn Tyr Arg Asp Thr Phe Phe Asn Leu Ser Leu Lys Glu
       210                 215                 220
   Val Leu Phe Leu Arg Trp Val Ser Thr Ser Cys Pro Asp Thr Glu Phe
   225                 230                 235                 240
   Val Phe Lys Gly Asp Asp Asp Val Phe Val Asn Thr His His Ile Leu
                   245                 250                 255
   Asn Tyr Leu Asn Ser Leu Ser Lys Thr Lys Ala Lys Asp Leu Phe Ile
               260                 265                 270
   Gly Asp Val Ile His Asn Ala Gly Pro His Arg Asp Lys Lys Leu Lys
           275                 280                 285
   Tyr Tyr Ile Pro Glu Val Val Tyr Ser Gly Leu Tyr Pro Pro Tyr Ala
       290                 295                 300
   Gly Gly Gly Gly Phe Leu Tyr Ser Gly His Leu Ala Leu Arg Leu Tyr
   305                 310                 315                 320
   His Ile Thr Asp Gln Val His Leu Tyr Pro Ile Asp Asp Val Tyr Thr
                   325                 330                 335
   Gly Met Cys Leu Gln Lys Leu Gly Leu Val Pro Glu Lys His Lys Gly
               340                 345                 350
   Phe Arg Thr Phe Asp Ile Glu Glu Lys Asn Lys Asn Asn Ile Cys Ser
           355                 360                 365
   Tyr Val Asp Leu Met Leu Val His Ser Arg Lys Pro Gln Glu Met Ile
       370                 375                 380
   Asp Ile Trp Ser Gln Leu Gln Ser Ala His Leu Lys Cys
   385                 390                 395
<210> 15
   <211> 365
   <212> PRT
   <213> Homo sapiens
<400> 15
   Gln Glu Lys Asn Gly Lys Gly Glu Val Ile Ile Pro Lys Glu Lys Phe
   1               5                   10                  15
   Trp Lys Ile Ser Thr Pro Pro Glu Ala Tyr Trp Asn Arg Glu Gln Glu
               20                  25                  30
   Lys Leu Asn Arg Gln Tyr Asn Pro Ile Leu Ser Met Leu Thr Asn Gln
           35                  40                  45
   Thr Gly Glu Ala Gly Arg Leu Ser Asn Ile Ser His Leu Asn Tyr Cys
       50                  55                  60
   Glu Pro Asp Leu Arg Val Thr Ser Val Val Thr Gly Phe Asn Asn Leu
   65                  70                  75                  80
   Pro Asp Arg Phe Lys Asp Phe Leu Leu Tyr Leu Arg Cys Arg Asn Tyr
                   85                  90                  95
   Ser Leu Leu Ile Asp Gln Pro Asp Lys Cys Ala Lys Lys Pro Phe Leu
               100                 105                 110
   Leu Leu Ala Ile Lys Ser Leu Thr Pro His Phe Ala Arg Arg Gln Ala
           115                 120                 125
   Ile Arg Glu Ser Trp Gly Gln Glu Ser Asn Ala Gly Asn Gln Thr Val
       130                 135                 140
   Val Arg Val Phe Leu Leu Gly Gln Thr Pro Pro Glu Asp Asn His Pro
   145                 150                 155                 160
   Asp Leu Ser Asp Met Leu Lys Phe Glu Ser Glu Lys His Gln Asp Ile
                   165                 170                 175
   Leu Met Trp Asn Tyr Arg Asp Thr Phe Phe Asn Leu Ser Leu Lys Glu
               180                 185                 190
   Val Leu Phe Leu Arg Trp Val Ser Thr Ser Cys Pro Asp Thr Glu Phe
           195                 200                 205
   Val Phe Lys Gly Asp Asp Asp Val Phe Val Asn Thr His His Ile Leu
       210                 215                 220
   Asn Tyr Leu Asn Ser Leu Ser Lys Thr Lys Ala Lys Asp Leu Phe Ile
   225                 230                 235                 240
   Gly Asp Val Ile His Asn Ala Gly Pro His Arg Asp Lys Lys Leu Lys
                   245                 250                 255
   Tyr Tyr Ile Pro Glu Val Val Tyr Ser Gly Leu Tyr Pro Pro Tyr Ala
               260                 265                 270
   Gly Gly Gly Gly Phe Leu Tyr Ser Gly His Leu Ala Leu Arg Leu Tyr
           275                 280                 285
   His Ile Thr Asp Gln Val His Leu Tyr Pro Ile Asp Asp Val Tyr Thr
       290                 295                 300
   Gly Met Cys Leu Gln Lys Leu Gly Leu Val Pro Glu Lys His Lys Gly
   305                 310                 315                 320
   Phe Arg Thr Phe Asp Ile Glu Glu Lys Asn Lys Asn Asn Ile Cys Ser
                   325                 330                 335
   Tyr Val Asp Leu Met Leu Val His Ser Arg Lys Pro Gln Glu Met Ile
               340                 345                 350
   Asp Ile Trp Ser Gln Leu Gln Ser Ala His Leu Lys Cys
           355                 360                 365
<210> 16
   <211> 20
   <212> DNA
   <213> Homo sapiens
<220>
   <221> Artificial sequence
   <222> (1)...(20)
   <223> Synthetically generated primer
<400> 16
   cttcgacgcc ccacactcat 20
<210> 17
   <211> 20
   <212> DNA
   <213> Homo sapiens
<220>
   <221> Artificial sequence
   <222> (1)...(20)
   <223> Synthetically generated primer
<400> 17
   atgagtgtgg ggcgtcgaag 20

## Description

### Background of the Invention

The ob gene product, leptin, is an important circulating regulator of body weight. Leptin binds to and activates the long form of ObR, the leptin receptor (Tartaglia et al. (1995) *Cell* 83:1263-71). Leptin is thought to modulate body weight by influencing appetite and other factors. Compounds other than leptin, e.g., neuropeptide Y, melanocortins, CART, and orexins are also thought to play a role in modulation of body weight by influencing factors such as appetite and satiety, fat storage, and energy output,

### Summary of the Invention

The present invention is based, at least in part, on the identification of six genes whose expression is induced by leptin. Two of these genes, LIG46 and LIG56, are novel genes. Four of the genes have been previously identified. The previously identified genes are: Tgtp, encoding a T cell-specific GTP-binding protein; LRG-47, encoding an interferon (IFN) inducible protein; RC10-II, encoding a subunit of a 20S brain proteasome; and Stra13, encoding a retinoic acid inducible protein.

The six leptin-inducible genes and the proteins they encode represent targets for the development of therapeutic agents for use in modulating body weight. For example, agents that alter the expression or activity of one or more of the leptin-induced proteins can be used to modulate body weight. Such agents can be identified using cellular, *in vitro,* or *in vivo* assays which monitor the expression or activity of one or more of the six leptin-induced proteins. Potentially useful therapeutic agents can also be identified through the use of assays designed to identify agents that bind to one of the leptin-induced proteins. The leptin-induced genes and the proteins they encode may themselves be useful therapeutically and diagnostically.

### LIG46

The murine LIG46 cDNA described below (SEQ ID NO:1) has a 1191 nucleotide open reading frame (nucleotides 3 - 1193 of SEQ ID NO:1; SEQ ID NO:3) which encodes a 397 amino acid protein (SEQ ID NO:2). This protein includes a predicted signal sequence of about 32 amino acids (from amino acid 1 to about amino acid 32 of SEQ ID NO:2) and a predicted mature protein of about 365 amino acids (from about amino acid 33 to amino acid 397 of SEQ ID NO:2; SEQ ID NO:4). The extracellular domain of LIG46 extends from about amino acid 33 to about amino acid 302. LIG46 possesses one predicted transmembrane domain which extends from about amino acid 303 (extracellular end) to about 320 (intracellular end) of SEQ ID NO:2. The cytoplasmic domain of LIG46 extends from about amino acid 321 to about amino acid 397.

The human LIG46 cDNA described below (SEQ ID NO: ) has a 1191 nucleotide open reading frame which encodes a 397 amino acid protein (SEQ ID NO: ). This protein includes a predicted signal sequence of about 32 amino acids (from amino acid 1 to about amino acid 32 of SEQ ID NO:_) and a predicted mature protein of about 365 amino acids (from about amino acid 33 to amino acid 397 of SEQ ID NO:_; SEQ ID NO:_).

LIG46 protein has some sequence similarity to a number of galactosyltransferases. Galactosyltransferases have been implicated in developmental processes. In addition, galactosyltransferases may play a role in cell-to-cell signaling by modifying the carbohydrate repertoire on cell surface receptors to activate, inhibit or otherwise modify (e.g., by altering receptor affinity for a ligand) receptor activity. Thus, LIG46 may play a role body weight regulation by influencing cell-to-cell signaling mediated by molecules involved in body weight regulation, e.g., leptin.

The LIG46 polypeptide sequence of SEQ ID NO:2 includes potential N-glycosylation sites at amino acids 30-33, 79-82, 89-92, 127-173, and 219-222; potential protein kinase C phosphorylation sites at amino acids 54-56, 202-204, 221-223, 323-325, and 377-379; potential casein kinase II phosphorylation sites at amino acids 31-34, 94-97, 185-188, 221-224, 234-237, and 368-371; a potential tyrosine kinase phosphorylation site at amino acids 115-122; and a potential amidation site at amino acids 3-6.

The invention makes use of nucleic acid molecules encoding LIG46 proteins or biologically active portions thereof, as well as nucleic acid molecules suitable for use as primers or hybridization probes for the detection of LIG46-encoding nucleic acid molecules.

Nucleic acid molecules that are at least 45% (or 55%, 65%, 75%, 85%, 95%; or 98%) identical to the nucleotide sequence shown in SEQ ID NO:1, or SEQ ID NO:3, or a complement thereof are useful.

Nucleic acid molecules which include a fragment of at least 300 (325, 350, 375, 400, 425, 450, 500, 550, 600, 650, 700, 800, 900, 1000, 1200, 1300, or 1400) nucleotides of the nucleotide sequence shown in SEQ ID NO:1, or SEQ ID NO:3, or a complement thereof are useful.

Nucleic acid molecules which include a nucleotide sequence encoding a protein having an amino acid sequence that is at least 45% (or 55%, 65%, 75%, 85%, 95%; or 98%) identical to the amino acid sequence shown in SEQ ID NO:2, or SEQ ID NO:4, are useful.

A LIG46 nucleic acid molecule has the nucleotide sequence shown SEQ ID NO:1 or SEQ ID NO:3.

A useful nucleic acid molecule encodes a fragment of a polypeptide having the amino acid sequence of SEQ ID NO:2 or SEQ ID NO:4, the fragment including at least 15 (25, 30, 50, 100, 150, 300, or 390) contiguous amino acids of SEQ ID NO:2 or SEQ ID NO:4.

A useful nucleic acid molecule encodes a naturally occurring allelic variant of a polypeptide comprising the amino acid sequence of SEQ ID NO:2 or SEQ ID NO:4, wherein the nucleic acid molecule hybridizes to a nucleic acid molecule comprising SEQ ID NO:1 or SEQ ID NO:3 under stringent conditions.

Also useful are: an isolated LIG46 protein having an amino acid sequence that is at least about 65%, preferably 75%, 85%, 95%, or 98% identical to the amino acid sequence of SEQ ID NO:4 (mature murine LIG46) or the amino acid sequence of SEQ ID NO:2 (immature murine LIG46); and an isolated LIG46 protein having an amino acid sequence that is at least about 85%, 95%, or 98% identical to a portion of LIG46 having homology to a galactosyltransferase (e.g., amino acids 192-353, 142-184, 201-296, 289-347, 140-183, 367-391, 177-266, 299-343, or 140-184 of SEQ ID NO:2) or a neurogenic secreted signalling protein (e.g., amino acids 200-291, 270-354, 144-183, 380-394, or 211-248 of SEQ ID NO:2).

Also useful are: an isolated LIG46 protein which is encoded by a nucleic acid molecule having a nucleotide sequence that is at least about 65%, preferably 75%, 85%, or 95% identical to SEQ ID NO:3; and an isolated LIG46 protein which is encoded by a nucleic acid molecule having a nucleotide sequence which hybridizes under stringent hybridization conditions to a nucleic acid molecule having the nucleotide sequence of the complement of SEQ ID NO:3.

Also useful is a polypeptide which is a naturally occurring allelic variant of a polypeptide that includes the amino acid sequence of SEQ ID NO:2 or SEQ ID NO:4, wherein the polypeptide is encoded by a nucleic acid molecule which hybridizes to a nucleic acid molecule comprising the complement of SEQ ID NO:1 or SEQ ID NO:3 under stringent conditions.

LIG46 nucleic acid molecules specifically detect LIG46 nucleic acid molecules (e.g., a nucleic acid molecule encoding human LIG46) relative to nucleic acid molecules encoding other galactosyltransferases. For example, a LIG46 nucleic acid molecule hybridizes under stringent conditions to a nucleic acid molecule comprising the nucleotide sequence of SEQ ID NO:1, SEQ ID NO:3, or a complement thereof, but does not hybridize to unrelated galactosyltransferases.
The LIG46 nucleic acid molecule is at least 300 (325, 350, 375, 400, 425, 450, 500, 550, 600, 650, 700, 800, 900, 1000, or 1200) nucleotides in length and hybridizes under stringent conditions to a nucleic acid molecule comprising the nucleotide sequence shown in SEQ ID NO:1, SEQ ID NO:3, or a complement thereof.

A vector, e.g., a recombinant expression vector, comprising a LIG46 nucleic acid molecule is useful.

A host cell containing such a vector is useful. A method for producing LIG46 protein comprises culturing, in a suitable medium, a host cell containing a recombinant expression vector such that a LIG46 protein is produced.

Isolated or recombinant LIG46 proteins and polypeptides are useful.
Preferred LIG46 proteins and polypeptides possess at least one biological activity possessed by naturally-occurring LIG46 (e.g., the ability to act as a galactosyl transferase) and are induced by leptin.

The LIG46 proteins or biologically active portions thereof, can be operatively linked to a non-LIG46 polypeptide (e.g., heterologous amino acid sequences) to form LIG46 fusion proteins.

Antibodies that specifically bind LIG46 proteins, such as monoclonal or polyclonal antibodies are described herein. In addition, the LIG46 proteins or biologically active portions thereof can be incorporated into pharmaceutical compositions, which optionally include pharmaceutically acceptable carriers.

In one aspect, the present invention includes a method for detecting the presence of LIG46 activity or expression in a biological sample by contacting the biological sample with an agent capable of detecting an indicator of LIG46 activity such that the presence of LIG46 activity is detected in the biological sample.

A method for modulating LIG46 activity is described herein, the method comprising contacting a cell with an agent that modulates (inhibits or stimulates) LIG46 activity or expression such that LIG46 activity or expression in the cell is modulated. In one example, the agent is an antibody that specifically binds to LIG46 protein. In another example, the agent modulates expression of LIG46 by modulating transcription of a LIG46 gene, splicing of a LIG46 mRNA, or translation of a LIG46 mRNA. In yet another example, the agent is a nucleic acid molecule having a nucleotide sequence that is antisense to the coding strand of the LIG46 mRNA or the LIG46 gene.

The methods described herein are used to treat a subject having a disorder characterized by and undesirable level of LIG46 protein or nucleic acid expression or activity by administering an agent which is a LIG46 modulator to the subject. In one example, the LIG46 modulator is a LIG46 protein. In another example the LIG46 modulator is a LIG46 nucleic acid molecule. In other examples, the LIG46 modulator is a peptide, peptidomimetic, or other small molecule. In a preferred example, the disorder is obesity or cachexia.

For treatment of obesity it is desirable to administer an agent which reduces the expression or activity of LIG46 (an LIG46 antagonist). Such an agent can be administered in conjunction with leptin. Preferably the amount of leptin administered is sufficient, in combination with any endogenous leptin, to render the subect being treated sensitive to the effects of the LIG46 antagonist.

For treatment of low body weight it is desirable to administer an agent which increases the expression of activity of LIG46 (an LIG46 agonist).

A diagnostic assay for identifying the presence or absence of a genetic lesion or mutation is described herein, the assay characterized by identifying at least one of: (i) aberrant modification or mutation of a gene encoding a LIG46 protein; (ii) mis-regulation of a gene encoding a LIG46 protein; and (iii) aberrant post-translational modification of a LIG46 protein, wherein a wild-type form of the gene encodes a protein with a LIG46 activity.

In another aspect, the invention provides a method for identifying a compound that binds to or modulates the activity of a LIG46 protein. In general, such methods entail measuring a biological activity of a LIG46 protein in the presence and absence of a test compound and identifying those compounds which alter the activity of the LIG46 protein.

The invention also features methods for identifying a compound which modulates the expression of LIG46 by measuring the expression of LIG46 in the presence and absence of a compound.

### LIG56

The murine LIG56 cDNA described below (SEQ ID NO:5) has a 1200 nucleotide open reading frame (nucleotides 1 -1200 of SEQ ID NO:5; SEQ ID NO:7) which encodes a 400 amino acid protein (SEQ ID NO:6).

The LIG56 polypeptide sequence of SEQ ID NO:6 includes potential N-glycosylation sites at amino acids 252-255; potential protein kinase C phosphorylation sites at amino acids 67-69, 75-77, 203-205, 218-220, 295-297, and 299-301; potential casein kinase II phosphorylation sites at amino acids 126-129, 170-173, 203-206, 256-259, 291-294, 341-344, and 345-349; a potential tyrosine kinase phosphorylation site at amino acids 233-241; potential N-myristlation sites at amino acids 66-71, 85-90, 116-121, and 308-313; and a potential amidation site at amino acids 63-70.

LIG56 may be a GTP-binding protein. Portions of LIG56 protein are to similar to one or more murine GTP-binding proteins (Genbank Accession Numbers: L38444; U15636; M63630; U19119; and U53219).

LIG56 protein possesses a GTP-binding protein-like domain (amino acids 12 to 283 of SEQ ID NO:6) and an LRG-47-like domain (amino acids 24-177 of SEQ ID NO:6).

This invention makes use of nucleic acid molecules encoding LIG56 proteins or biologically active portions thereof, as well as nucleic acid fragments suitable as primers or hybridization probes for the detection of LIG56-encoding nucleic acids.

Nucleic acid molecules which are at least 45% (or 55%, 65%, 75%, 85%, 95%, or 98%) identical to the nucleotide sequence shown in SEQ ID NO:5 or SEQ ID NO:7, or a complement thereof are useful.

Nucleic acid molecules which include a fragment of at least 100 (200, 250, 300, 350, 400, 450, 500, 550, 600, 650, 700, 800, 900, 1000, 1100, or 1200) nucleotides of the nucleotide sequence shown in SEQ ID NO:5 or SEQ ID NO:7, or a complement thereof are useful.

Nucleic acid molecules which include a nucleotide sequence encoding a protein having an amino acid sequence that is at least 45% (or 55%, 65%, 75%, 85%, 95%, or 98%) identical to the amino acid sequence of SEQ ID NO:6 are useful.

A LIG56 nucleic acid molecule has the nucleotide sequence shown SEQ ID NO:5 or SEQ ID NO:7.

A useful nucleic acid molecule encodes a fragment of a polypeptide having the amino acid sequence of SEQ ID NO:6, the fragment including at least 15 (25, 30, 50, 100, 150, 300, or 400) contiguous amino acids of SEQ ID NO:6.

A useful nucleic acid molecule encodes a naturally occurring allelic variant of a polypeptide comprising the amino acid sequence of SEQ ID NO:6, wherein the nucleic acid molecule hybridizes to a nucleic acid molecule having the sequence of the complement of SEQ ID NO:5 or SEQ ID NO:7 under stringent conditions.

Also useful are: an isolated LIG56 protein having an amino acid sequence that is at least about 65%, preferably 75%, 85%, 95%, or 98% identical to the amino acid sequence of SEQ ID NO:6.

Also useful are: an isolated LIG56 protein which is encoded by a nucleic acid molecule having a nucleotide sequence that is at least about 65%, preferably 75%, 85%, or 95% identical to SEQ ID NO:7; and an isolated LIG56 protein which is encoded by a nucleic acid molecule having a nucleotide sequence which hybridizes under stringent hybridization conditions to a nucleic acid molecule having the nucleotide sequence of SEQ ID NO:7.

Also useful is a polypeptide which is a naturally occurring allelic variant of a polypeptide that includes the amino acid sequence of SEQ ID NO:6, wherein the polypeptide is encoded by a nucleic acid molecule which hybridizes to a nucleic acid molecule comprising SEQ ID NO:5 or SEQ ID NO:7 under stringent conditions.

LIG56 nucleic acid molecules specifically detect LIG56 nucleic acid molecules (e.g., human LIG56) relative to nucleic acid molecules encoding other unrelated nucleic acid molecules having sequence homology to GTP-binding proteins. For example, a LIG56 nucleic acid molecule hybridizes under stringent conditions to a nucleic acid molecule comprising the nucleotide sequence of SEQ ID NO:5 or SEQ ID NO:7, or a complement thereof. The LIG56 nucleic acid molecule is at least 300 (325, 350, 375, 400, 425, 450, 500, 550, 600, 650, 700, 800, 900, 1000, 1100 or 1200) nucleotides in length and hybridizes under stringent conditions to a nucleic acid molecule comprising the nucleotide sequence shown in SEQ ID NO:5 or SEQ ID NO:7, or a complement thereof.

An isolated nucleic acid molecule which is antisense to the coding strand of a LIG56 nucleic acid is useful.

A vector, e.g., a recombinant expression vector, comprising a LIG56 nucleic acid molecule is useful. A host cell containing such a vector is also useful. A method for producing LIG56 protein comprises culturing, in a suitable medium, a host cell of the invention containing a recombinant expression vector such that a LIG56 polypeptide is produced.

Isolated or recombinant LIG56 proteins and polypeptides are useful. Preferred LIG56 proteins and polypeptides possess at least one biological activity possessed by naturally occurring LIG56 and are induced by leptin.

The LIG56 proteins or biologically active portions thereof, can be operatively linked to a non-LIG56 polypeptide (e.g., heterologous amino acid sequences) to form LIG56 fusion proteins.

Antibodies that specifically bind LIG56 proteins, such as monoclonal or polyclonal antibodies are described herein. In addition, the LIG56 proteins or biologically active portions thereof can be incorporated into pharmaceutical compositions, which optionally include pharmaceutically acceptable carriers.

In one aspect, the present invention provides a method for detecting the presence of LIG56 activity or expression in a biological sample by contacting the biological sample with an agent capable of detecting an indicator of LIG56 activity such that the presence of LIG56 activity is detected in the biological sample.

A method for modulating LIG56 activity is described herein, the method comprising contacting a cell with an agent that modulates (inhibits or stimulates) LIG56 activity or expression such that LIG56 activity or expression in the cell is modulated. In one example the agent is an antibody that specifically binds to LIG56 protein. In another example, the agent modulates expression of LIG56 by modulating transcription of a LIG56 gene, splicing of a LIG56 mRNA, or translation of a LIG56 mRNA. In yet another example, the agent is a nucleic acid molecule having a nucleotide sequence that is antisense to the coding strand of the LIG56 mRNA or the LIG56 gene.

The methods described herein are used to treat a subject having a disorder characterized by an undesirable level of LIG56 protein or nucleic acid expression (e.g., a body weight disorder) or activity by administering an agent which is a LIG56 modulator to the subject. In one example, the LIG56 modulator is a LIG56 protein. In another example the LIG56 modulator is a LIG56 nucleic acid molecule. In other examples, the LIG56 modulator is a peptide, peptidomimetic, or other small molecule. In a preferred embodiment, the disorder is obesity or cachexia.

A diagnostic assay for identifying the presence or absence of a genetic lesion or mutation is described herein, the assay characterized by identifying at least one of: (i) aberrant modification or mutation of a gene encoding a LIG56 protein; (ii) mis-regulation of a gene encoding a LIG56 protein; and (iii) aberrant post-translational modification of a LIG56 protein, wherein a wild-type form of the gene encodes a protein with a LIG56 activity.

In another aspect, the invention provides a method for identifying a compound that binds to or modulates the activity of a LIG56 protein. In general, such methods entail measuring a biological activity of a LIG56 protein in the presence and absence of a test compound and identifying those compounds which alter the activity of the LIG56 protein.

The invention also features methods for identifying a compound which modulates the expression of LIG56 by measuring the expression of LIG56 in the presence and absence of a compound.

### Tgtp, LRG-47, RC10-II, and Stra13

Tgtp, LRG-47, RC10-II, and Stra13 are known genes. However, none of these genes has previously been implicated in body weight regulation. The present invention is based, in part, on the discovery that expression of each of these genes is induced by leptin. Because Tgtp, LRG-47, RC10-II, and Stra13 are induced by leptin, Tgtp, LRG-47, RC10-II, and Stra13 protein and the nucleic acid molecules encoding them are useful in the development of therapeutic compounds for the treatment or prevention of body weight disorders.

Tgtp (Genbank Accession Number L38444) encodes a T cell-specific guanine nucleotide triphosphate-binding protein (Carlow et al. (1994) *J. Immunol*. 154:1724-34). LRG-47 (Genbank Accession Number U19119) is induced by LPS, IFN-γ, and IFN-α/β and encodes a protein that has some homology to GTP-binding proteins (Sorace et al. (1995) *J. Leukocyte Biol*. 58:477-84).

LRG-47 (Genbank Accession Number U19119) is a LPS, IFN-γ, and IFN-α/β-inducible gene having homology to IRG-47 and Mg21, both of which are IFN-γ-inducible genes (Sorace et al. (1995) *J. Leukocyte Biol*. 58:477-484). LRG-47 also has homology to Tgtp and may be a GTP-binding protein (Sorace et al., supra).

RC10-II (Genbank Accession Number D21800) is gene that encodes the RC10-II subunit of the 20S proteasome of rat embryonic brain (Nishimura et al. (1993) *FEBS Lett.* 336:462-66). It has been suggested that RC10-II is a proteasomal subunit that is required for expression of tryptic activity (Nishimura et al., *supra*).

Stra13 (Genbank Accession Number AF010305) is a retinoic acid-inducible gene that encodes a basic helix-loop-helix protein (Boudjelal et al. (1997) *Genes Dev.* 11:2052-65). Stra13 may act as a repressor of activated transcription and is thought to play a role in neuronal differentiation (Boudjelal et al., *supra*).

The invention provides a method for identifying a compound that binds to or modulates the activity of a Tgtp, LRG-47, RC10-II, or Stra13 protein. In general, such methods entail measuring a biological activity of a Tgtp, LRG-47, RC10-II, or Stra13 protein in the presence and absence of a test compound and identifying those compounds which bind to or alter the activity of the Tgtp, LRG-47, RC10-II, or Stra13 protein.

The invention also features methods for identifying a compound which modulates the expression of Tgtp, LRG-47, RC10-II, or Stra13 by measuring the expression of Tgtp, LRG-47, RC10-II, or Stra13 in the presence and absence of a compound.

Thus, the invention can be used to provide a method for modulating Tgtp, LRG-47, RC10-II, or Stra13 activity comprising contacting a cell with an agent that modulates (inhibits or stimulates) Tgtp, LRG-47, RC10-II, or Stra13 activity or expression such that Tgtp, LRG-47, RC10-II, or Stra13 activity or expression in the cell is modulated. The agent can be an antibody that specifically binds to Tgtp, LRG-47, RC10-II, or Stra13 protein. In another example, the agent modulates expression of Tgtp, LRG-47, RC10-II, or Stra13 by modulating transcription of a Tgtp, LRG-47, RC10-II, or Stra13 gene; splicing of a Tgtp, LRG-47, RC10-II, or Stra13 mRNA; or translation of a Tgtp, LRG-47, RC10-II, or Stra13 mRNA. In yet another example, the agent is a nucleic acid molecule having a nucleotide sequence that is antisense to the coding strand of the Tgtp, LRG-47, RC10-II, or Stra13 mRNA or the Tgtp, LRG-47, RC10-II, or Stra13 gene.

The methods described herein are used to treat a subject having a disorder influenced by Tgtp, LRG-47, RC10-II, or Stra13 protein or nucleic acid expression or activity by administering an agent which is a Tgtp, LRG-47, RC10-II, or Stra13 modulator to the subject. In one example, the Tgtp, LRG-47, RC10-II, or Stra13 modulator is a Tgtp, LRG-47, RC10-II, or Stra13 protein. In another example the Tgtp, LRG-47, RC10-II, or Stra13 modulator is a Tgtp, LRG-47, RC10-II, or Stra13 nucleic acid molecule. In other examples, the Tgtp, LRG-47, RC10-II, or Stra13 modulator is a peptide, peptidomimetic, or other small molecule. In a preferred example, the disorder is obesity or cachexia.

A diagnostic assay for identifying the presence or absence of a genetic lesion or mutation is described herein, the assay characterized by identifying at least one of: (i) aberrant modification or mutation of a gene encoding a Tgtp, LRG-47, RC10-II, or Stra13 protein; (ii) mis-regulation of a gene encoding a Tgtp, LRG-47, RC10-II, or Stra13 protein; and (iii) aberrant post-translational modification of a Tgtp, LRG-47, RC10-II, or Stra13 protein, wherein a wild-type form of the gene encodes a protein with a Tgtp, LRG-47, RC10-II, or Stra13 activity, as such leasion are characterized by body weight disorders.

In another aspect, the present invention provides a method for detecting the presence of Tgtp, LRG-47, RC10-II, or Stra13 activity or expression in a biological sample by contacting the biological sample with an agent capable of detecting an indicator of Tgtp, LRG-47, RC10-II, or Stra13 activity such that the presence of Tgtp, LRG-47, RC10-II, or Stra13 activity is detected in the biological sample.

Other features and advantages of the invention will be apparent from the following detailed description and claims.

### Brief Description of the Drawings

Figure 1 depicts the cDNA sequence (SEQ ID NO:1) and predicted amino acid sequence (SEQ ID NO:2) of murine LIG46.

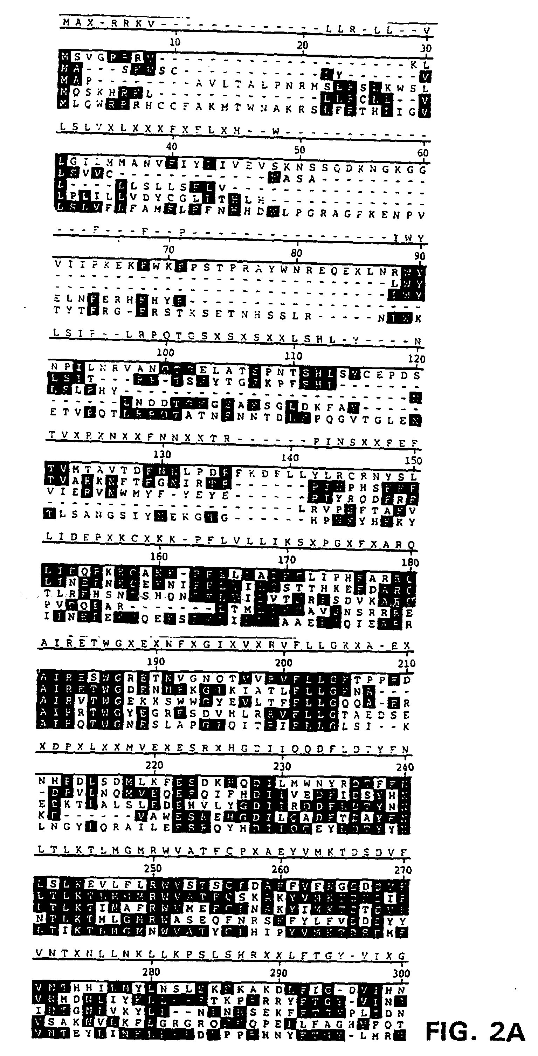

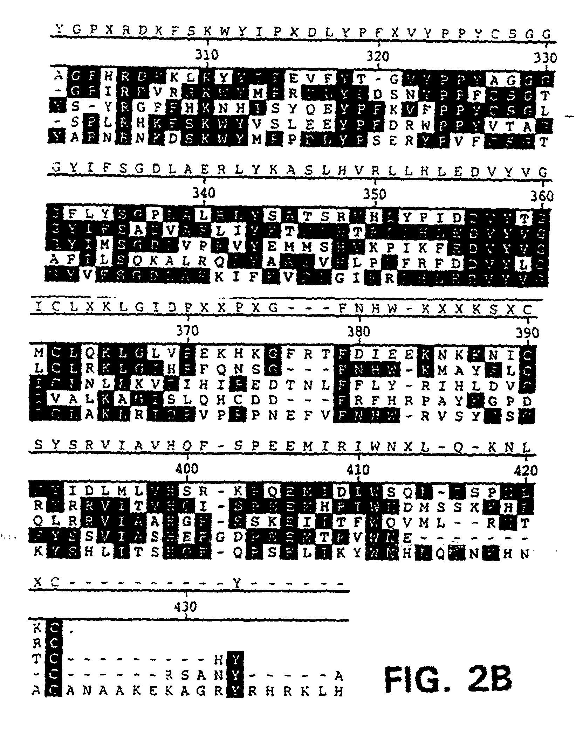

Figure 2 depicts a series of alignments of the amino acid sequence of LIG46 with portions of a number of galactosyltransferases, including (from top to bottom): *Mus musculus* UDP-Gal: betaGlcNAc beta 1,3-galactosyltransferase-I (Accession Number AF029790; SEQ ID NO: ); *Mus musculus* IPP-Gal: betaGlcNAc beta 1,3-galactosyltransferase-III (Accession Number AF029792); *Drosophila melanogaster* neurogenic secreted signalling protein ("Brainiac"; Accession Number U41449; SEQ ID NO: ); and *Homo sapiens* UDP-galactose: 2-acetamido-2-deoxy-D-glucose3beta-galactosyltransferase (Accession Number Y15014; SEQ ID NO: ). The amino acid sequence above the solid line is a majority sequence
Figure 3 is a hydropathy plot of LIG46. The location of the predicted transmembrane (TM), cytoplasmic (IN), and extracellular (OUT) domains are indicated as are the position of cysteines (cys; vertical bars immediately below the plot). Relative hydrophobicity is shown above the dotted line, and relative hydrophilicity is shown below the dotted line.
Figure 4 depicts the cDNA sequence (SEQ ID NO:5) and predicted amino acid sequence (SEQ ID NO:6) of murine LIG56.
Figure 5 is a hydropathy plot of LIG56. Relative hydrophobicity is shown above the dotted line, and relative hydrophilicity is shown below the dotted line.
Figure 6 is a graph depicting the effect of LIG46 sense and antisense oligonucleotides on food intake of male obese (ob/ob) mice in the presence and absence of leptin.
Figure 7 depicts the cDNA sequence of human LIG46
Figure 8 depicts the predicted amino acid sequence of human LIG46.
Figure 9 depicts an alignment of the cDNA sequences of human LIG46 (upper sequence) and murine LIG46 (lower sequence).
Figure 10 depicts an alignment of the predicted amino acid sequences of human LIG46 (upper sequence) and murine LIG46 (lower sequence).
Figure 11 is a graph depicting the effect of LIG46 sense and antisense oligonucleotides on food intake of male lean mice in the presence and absence of leptin.

### Detailed Description of the Invention

The present invention is based, in part, on the identification of six genes whose expression is induced by leptin. Four of the genes, Tgtp, LRG-47, RC10-II, and Stra13, are known genes. Two of the genes, LIG46 and LIG56, are novel.

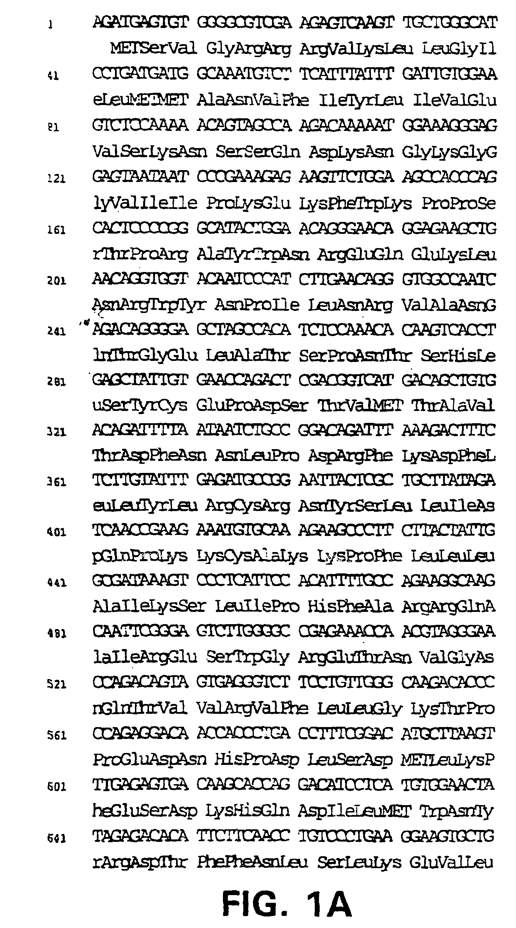

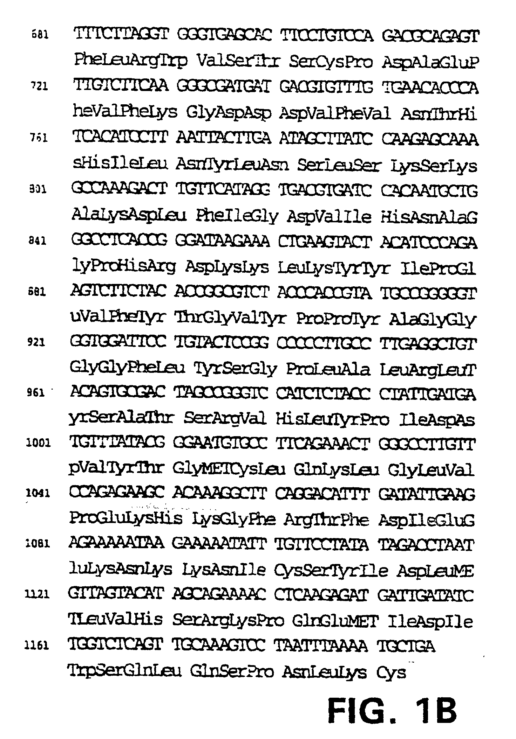

A nucleotide sequence encoding murine LIG46 protein is shown in Figure 1 (SEQ ID NO:1; SEQ ID NO:3 includes the open reading frame only). A predicted amino acid sequence of LIG46 protein is also shown in Figure 1 (SEQ ID NO: 2).

The murine LIG46 cDNA of Figure 1 (SEQ ID NO:1) encodes a 397 amino acid protein.

Murine LIG46 is one member of a family of molecules (the "LIG46 family") having certain conserved structural and functional features. The term "family" when referring to the protein and nucleic acid molecules of the invention is intended to mean two or more proteins or nucleic acid molecules having a common structural domain and having sufficient amino acid or nucleotide sequence identity as defined herein. Such family members can be naturally occurring and can be from either the same or different species. For example, a family can contain a first protein of murine origin and a homologue of that protein of human origin, as well as a second, distinct protein of human origin and a murine homologue of that protein. Members of a family may also have common functional characteristics.

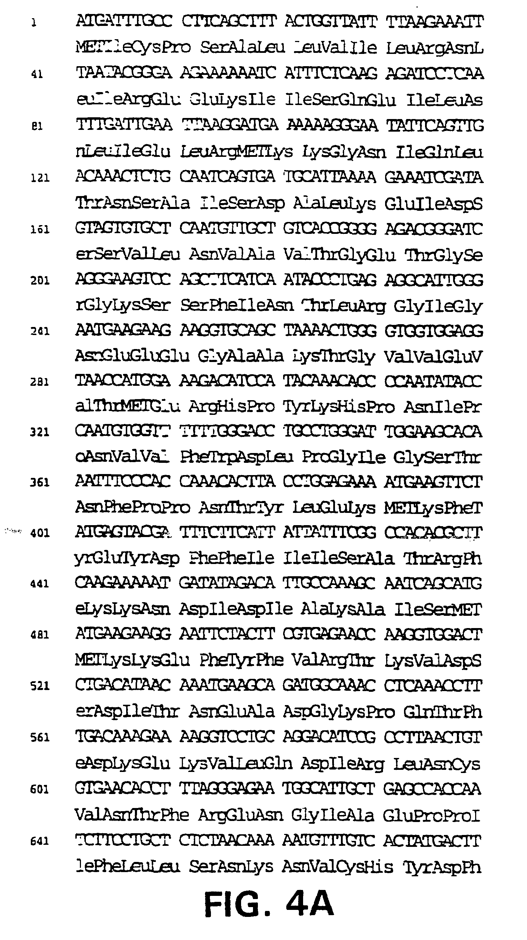

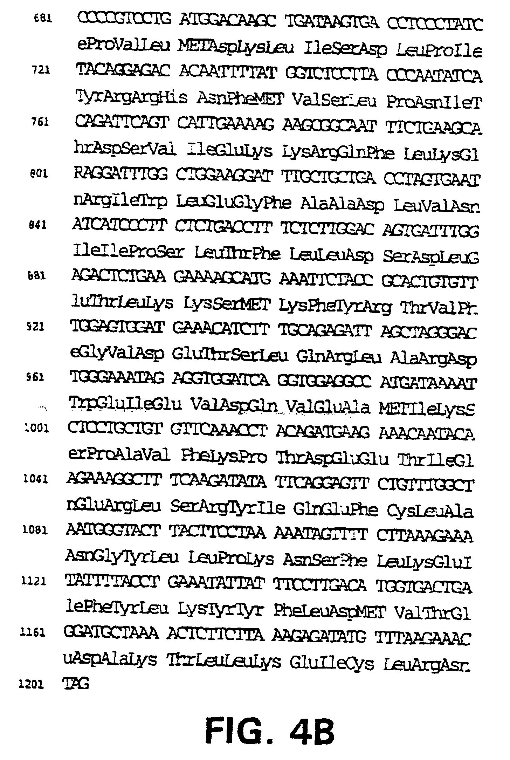

A nucleotide sequence encoding murine LIG56 protein is shown in Figure 4 (SEQ ID NO:5; SEQ ID NO:7 includes the open reading frame only). A predicted amino acid sequence of LIG46 protein is also shown in Figure 4 (SEQ ID NO:6).

The murine LIG56 cDNA of Figure 4 (SEQ ID NO:5) encodes a 400 amino acid protein.

Murine LIG56 is one member of a family of molecules (the "LIG56 family") having certain conserved structural and functional features. The term "family" when referring to the protein and nucleic acid molecules of the invention is intended to mean two or more proteins or nucleic acid molecules having a common structural domain and having sufficient amino acid or nucleotide sequence identity as defined herein. Such family members can be naturally occurring and can be from either the same or different species. For example, a family can contain a first protein of murine origin and a homologue of that protein of human origin, as well as a second, distinct protein of human origin and a murine homologue of that protein. Members of a family may also have common functional characteristics.

Tgtp (Genbank Accession Number L38444) encodes a T cell-specific guanine nucleotide triphosphate-binding protein (Carlow et al. (1994) *J. Immunol*. 154:1724-34). LRG-47 (Genbank Accession Number U19119) is induced by LPS, IFN-γ, and IFN-α/β and encodes a protein that has some homology to GTP-binding proteins (Sorace et al. (1995) *J. Leukocyte Biol*. 58:477-84).

LRG-47 (Genbank Accession Number U19119) is a LPS, IFN-γ, and IFN-α/β-inducible gene having homology to IRG-47 and Mg21, both of which are IFN-γ-inducible genes (Sorace et al. (1995) *J. Leukocyte Biol*. 58:477-484). LRG-47 also has homology to Tgtp and may be a GTP-binding protein (Sorace et al., *supra*).

RC10-II (Genbank Accession Number D21800) is gene that encodes the RC10-II subunit of the 20S proteasome of rat embryonic brain (Nishimura et al. (1993) *FEBS Lett.* 336:462-66). It has been suggested that RC10-II is a proteasomal subunit that is required for expression of tryptic activity (Nishimura et al., *supra).*

Stra13 (Genbank Accession Number AF010305) is a retinoic acid-inducible gene that encodes a basic helix-loop-helix protein (Boudjelal et al. (1997) *Genes Dev.* 11:2052-65). Stra13 may act as a repressor of activated transcription and is thought to play a role in neuronal differentiation (Boudjelal et al., *supra).*

Various aspects of the invention are described in further detail in the following subsections.

### I. Isolated Nucleic Acid Molecules

Isolated nucleic acid molecules that encode LIG46 or LIG56 proteins or biologically active portions thereof are described herein as well as nucleic acid molecules which can be used as hybridisation probes to identify LIG46 or LIG56-encoding nucleic acid molecules (e.g., human LIG46 or human LIG56) and fragments for use as PCR primers for the amplification or mutation of LIG46 or LIG56 nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

This section describes various LIG46 and LIG56 nucleic acid molecules. Of course, isolated nucleic acid molecules encoding all or part of Tgtp, LRG-47, RC10-II, and Stra13 are useful in the methods of the invention, e.g., methods for identifying compounds which modulate a body weight disorder. Thus, a nucleic acid molecule encompassing a sequence encoding all or part of Tgtp, LRG-47, RC10-II, or Stra13 (or a nucleic acid molecule encompassing all or part of the regulatory region of a Tgtp, LRG-47, RC10-II, or Stra13 gene) can be used to create recombinant cells that can be used in screening assays. In addition, nucleic acid molecules encoding Tgtp, LRG-47, RC10-II, and Stra13 can be used to create transgenic mice which overexpress one or more of Tgtp, LRG-47, RC10-II, and Stra13. Such transgenic mice are useful in elucidating the role of these genes in body weight regulation. Thus, the methods described in this section can be used to prepare and manipulate Tgtp, LRG-47, RC10-II, and Stra13 nucleic acid molecules as well as human homologues of Tgtp, LRG-47, RC10-II, and Stra13.

An "isolated" nucleic acid molecule is one which is separated from other nucleic acid molecules which are present in the natural source of the nucleic acid. Preferably, an "isolated" nucleic acid is free of sequences (preferably protein encoding sequences) which naturally flank the nucleic acid (i.e., sequences located at the 5.' and 3' ends of the nucleic acid) in the genomic DNA of the organism from which the nucleic acid is derived. For example, in various embodiments, the isolated LIG46 or LIG56 nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule described herein e.g., a nucleic acid molecule having the nucleotide sequence of SEQ ID NO:1, SEQ ID NO:3, SEQ ID NO:5 or SEQ ID NO:7, or a complement of any of these nucleotide sequences, can be isolated using standard molecular biology techniques and the sequence information provided herein. Using all or portion of the nucleic acid sequences of SEQ ID NO:1, SEQ ID NO:3, or all or a portion of the nucleic acid sequence of SEQ ID NO:5 or SEQ ID NO:7, as a hybridization probe, LIG46 and LIG56 nucleic acid molecules can be isolated using standard hybridization and cloning techniques (e.g., as described in Sambrook et al., eds., *Molecular Cloning: A Laboratory Manual. 2nd, ed., Cold Spring Harbor Laboratory*, Cold Spring Harbor Laboratory Press, Cold Spring Harbor, NY, 1989).

A nucleic acid can be amplified using cDNA, mRNA or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to LIG46 or LIG56 nucleotide sequences can be prepared by standard synthetic techniques, e.g., using an automated DNA synthesizer.

The isolated nucleic acid molecules comprise a nucleic acid molecule which is a complement of the nucleotide sequence shown in SEQ ID NO:1, SEQ ID NO:3, SEQ ID NO:5 or SEQ ID NO:7, or a portion thereof. A nucleic acid molecule which is complementary to a given nucleotide sequence is one which is sufficiently complementary to the given nucleotide sequence that it can hybridize to the given nucleotide sequence thereby forming a stable duplex.

Moreover, the nucleic acid molecule can comprise only a portion of a nucleic acid sequence encoding LIG46 or LIG56, for example, a fragment which can be used as a probe or primer or a fragment encoding a biologically active portion of LIG46 or LIG56. The nucleotide sequence determined from the cloning of the murine LIG46 gene and the murine LIG56 gene allows for the generation of probes and primers designed for use in identifying and/or cloning LIG46 or LIG56 homologues in other cell types, e.g., from other tissues, as well as LIG46 and LIG56 homologues from other mammals, e.g., humans. The probe/primer typically comprises substantially purified oligonucleotide. The oligonucleotide typically comprises a region of nucleotide sequence that hybridizes under stringent conditions to at least about 12, preferably about 25, more preferably about 50, 75, 100, 125, 150, 175, 200, 250, 300, 350 or 400 consecutive nucleotides of the sense or anti-sense sequence of SEQ ID NO:1 or SEQ ID NO:3, or of a naturally occurring mutant of SEQ ID NO:1 or SEQ ID NO:3, or sense or anti-sense sequence of SEQ ID NO:5 or SEQ ID NO:7, or of a naturally occurring mutant of SEQ ID NO:5 or SEQ ID NO:7.

Probes based on the LIG46 or LIG56 nucleotide sequence can be used to detect transcripts or genomic sequences encoding the same or related proteins (e.g., human homologues). The probe comprises a label group attached thereto, e.g., a radioisotope, a fluorescent compound, an enzyme, or an enzyme co-factor. Such probes can be used as a part of a diagnostic test kit for identifying cells or tissue which mis-express a LIG46 or LIG56 protein, such as by measuring a level of a LIG46 or LIG56-encoding nucleic acid in a sample of cells from a subject, e.g., detecting LIG46 or LIG56 mRNA levels or determining whether a genomic LIG46 or LIG56 gene has been mutated or deleted.

A nucleic acid fragment encoding a "biologically active portion of LIG46" can be prepared by isolating a portion of SEQ ID NO:1 or SEQ ID NO:3 which encodes a polypeptide having a LIG46 biological activity, expressing the encoded portion of LIG46 (e.g., by recombinant expression *in vitro*) and assessing the activity of the encoded portion of LIG46. For example, a nucleic acid fragment encoding a biologically active portion of LIG46 includes a galactosyltransferase-like domain, e.g., SEQ ID NO: .

Nucleic acid molecules may differ from the nucleotide sequence of SEQ ID NO:1 or SEQ ID NO:3 due to degeneracy of the genetic code and thus encode the same LIG46 protein as that encoded by the nucleotide sequence shown in SEQ ID NO:1 or SEQ ID NO:3. In addition to the LIG46 nucleotide sequences shown in SEQ ID NO:1 and SEQ ID NO:3, it will be appreciated by those skilled in the art that DNA sequence polymorphisms that lead to changes in the amino acid sequences of LIG46 may exist within a population. Such genetic polymorphism in the LIG46 gene may exist among individuals within a population due to natural allelic variation. As used herein, the terms "gene" and "recombinant gene" refer to nucleic acid molecules comprising an open reading frame encoding a LIG46 protein, preferably a mammalian LIG46 protein. Such natural allelic variations can typically result in 1-5% variance in the nucleotide sequence of the LIG46 gene. Any and all such nucleotide variations and resulting amino acid polymorphisms in LIG46 that are the result of natural allelic variation and that do not alter the functional activity of LIG46 are useful.

A nucleic acid fragment encoding a "biologically active portion of LIG56" can be prepared by isolating a portion of SEQ ID NO:5 or SEQ ID NO:7 which encodes a polypeptide having a LIG56 biological activity, expressing the encoded portion of LIG56 protein (e.g., by recombinant expression *in vitro*) and assessing the activity of the encoded portion of LIG56. For example, a nucleic acid fragment encoding a biologically active portion of LIG56 includes a GTP binding protein-like domain, e.g., SEQ ID NO: .

Nucleic acid molecules may differ from the nucleotide sequence of SEQ ID NO:5 or SEQ ID NO:7 due to degeneracy of the genetic code and thus encode the same LIG56 protein as that encoded by the nucleotide sequence shown in SEQ ID NO:5 or SEQ ID NO:7. In addition to the murine LIG56 nucleotide sequence shown in SEQ ID NO:5 and SEQ ID NO:7, it will be appreciated by those skilled in the art that DNA sequence polymorphisms that lead to changes in the amino acid sequences of LIG56 may exist within a population. Such genetic polymorphism in the LIG56 gene may exist among individuals within a population due to natural allelic variation. As used herein, the terms "gene" and "recombinant gene" refer to nucleic acid molecules comprising an open reading frame encoding a LIG56 protein, preferably a mammalian LIG56 protein. Such natural allelic variations can typically result in 1-5% variance in the nucleotide sequence of the LIG56 gene. Any and all such nucleotide variations and resulting amino acid polymorphisms in LIG56 that are the result of natural allelic variation and that do not alter the functional activity of LIG56 are useful.

Moreover, nucleic acid molecules encoding LIG46 or LIG56 proteins from other species (LIG46 or LIG56 homologues), which have a nucleotide sequence which differs from that of the murine gene, are intended to be within the scope of the invention. Nucleic acid molecules corresponding to natural allelic variants and homologues of the LIG46 or LIG56 cDNA of the invention can be isolated based on their identity to the LIG46 or LIG56 nucleic acids disclosed herein using the murine cDNAs, or a portion thereof, as a hybridization probe according to standard hybridization techniques under stringent hybridization conditions. For example, a soluble LIG46 cDNA can be isolated based on its identity to murine membrane-bound LIG46. Likewise, a membrane-bound human LIG56 cDNA can be isolated based on its identity to soluble LIG56.

Accordingly, a useful isolated nucleic acid molecule is at least 300 (325, 350, 375, 400, 425, 450, 500, 550, 600, 650, 700, 800, 900, 1000, or 1200) nucleotides in length and hybridizes under stringent conditions to the nucleic acid molecule comprising the nucleotide sequence, preferably the coding sequence, of SEQ ID NO:1, or SEQ ID NO:3, or SEQ ID NO:5 or SEQ ID NO:7. As used herein, the term "hybridizes under stringent conditions" is intended to describe conditions for hybridization and washing under which nucleotide sequences at least 60% (65%, 70%, preferably 75%) identical to each other typically remain hybridized to each other. Such stringent conditions are known to those skilled in the art and can be found in *Current Protocols in Molecular Biology*, John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, a useful isolated nucleic acid molecule hybridises under stringent conditions to the sequence of SEQ ID NO:1, SEQ ID NO:3, SEQ ID NO:5 or SEQ ID NO:7 and corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In addition to naturally-occurring allelic variants of the LIG46 or LIG56 sequence that may exist in the population, the skilled artisan will further appreciate that changes can be introduced by mutation into the nucleotide sequences disclosed herein, thereby leading to changes in the amino acid sequence of the encoded LIG46 or LIG56 protein, without altering the functional ability of the LIG46 or LIG56 protein. For example, one can make nucleotide substitutions leading to amino acid substitutions at "non-essential" amino acid residues. A "non-essential" amino acid residue is a residue that can be altered from the wild-type sequence of LIG46 or LIG56 without altering the biological activity, whereas an "essential" amino acid residue is required for biological activity. For example, amino acid residues that are conserved among the LIG46 or LIG56 proteins of various species are predicted to be particularly unamenable to alteration.

For example, preferred LIG46 proteins of the present invention retain amino acids that are conserved among galactosyltransferases. Such conserved domains are less likely to be amenable to mutation. Other amino acid residues, however, (e.g., those that are not conserved or only semi-conserved among LIG46 or LIG56 of various species) may not be essential for activity and thus are likely to be amenable to alteration.

Accordingly, useful nucleic acid molecules encoding LIG46 or LIG56 proteins may contain changes in amino acid residues that are not essential for activity. Such LIG46 or LIG56 proteins differ in amino acid sequence from those disclosed herein yet retain biological activity. In one embodiment, the isolated nucleic acid molecule includes a nucleotide sequence encoding a protein that includes an amino acid sequence that is at least about 45% identical, 65%, 75%, 85%, 95%, or 98% identical to the amino acid sequence of SEQ ID NO:2 or SEQ ID NO:6.

An isolated nucleic acid molecule encoding a LIG46 or LIG56 protein having a sequence which differs from that disclosed herein can be created by introducing one or more nucleotide substitutions, additions or deletions into the nucleotide sequence disclosed herein such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in LIG46 or LIG56 is preferably replaced with another amino acid residue from the same side chain family. Alternatively, mutations can be introduced randomly along all or part of a LIG46 or LIG56 coding sequence, such as by saturation mutagenesis, and the resultant mutants can be screened for LIG46 or LIG56 biological activity to identify mutants that retain activity. Following mutagenesis, the encoded protein can be expressed recombinantly and the activity of the protein can be determined.

Antisense nucleic acid molecules are described herein, i.e., molecules which are complementary to a sense nucleic acid encoding a protein, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire LIG46 or LIG56 coding strand, or to only a portion thereof, e.g., all or part of the protein coding region (or open reading frame). An antisense nucleic acid molecule can be antisense to a noncoding region of the coding strand of a nucleotide sequence encoding LIG46 or LIG56. The noncoding regions ("5' and 3' untranslated regions") are the 5' and 3' sequences which flank the coding region and are not translated into amino acids.

Given the coding strand sequences encoding LIG46 or LIG56 disclosed herein (e.g., SEQ ID NO:1, SEQ ID NO:3, SEQ ID NO:5, and SEQ ID NO:7), antisense nucleic acids of the invention can be designed according to the rules of Watson and Crick base pairing. The antisense nucleic acid molecule can be complementary to the entire coding region of LIG46 or LIG56 mRNA, but more preferably is an oligonucleotide which is antisense to only a portion of the coding or noncoding region of LIG46 or LIG56 mRNA. For example, the antisense oligonucleotide can be complementary to the region surrounding the translation start site of LIG46 or LIG56 mRNA. An antisense oligonucleotide can be, for example, about 5, 10, 15, 20, 25, 30, 35, 40, 45 or 50 nucleotides in length. An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules are typically administered to a subject or generated *in situ* such that they hybridize with or bind to cellular mRNA and/or genomic DNA encoding the protein of interest to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules include direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule can be an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) *Nucleic Acids. Res.* 15:6625-6641). The antisense nucleic acid molecule can also comprise a 2'-o-methylribonucleotide (Inoue et al. (1987) *Nucleic Acids Res*. 15:6131-6148) or a chimeric RNA-DNA analogue (Inoue et al. (1987) *FEBS Lett.* 215:327-330).

The description also encompasses ribozymes. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) Nature 334:585-591)) can be used to catalytically cleave LIG46 or LIG56 mRNA transcripts to thereby inhibit translation of LIG46 or LIG56 mRNA. A ribozyme having specificity for a LIG46 or LIG56-encoding nucleic acid can be designed based upon the nucleotide sequence of a LIG46 or LIG56 cDNA disclosed herein. For example, a derivative of a Tetrahymena L-19 IVS RNA can be constructed in which the nucleotide sequence of the active site is complementary to the nucleotide sequence to be cleaved in a LIG46 or LIG56-encoding mRNA. See, e.g., Cech et al. U.S. Patent No. 4,987,071; and Cech et al. U.S. Patent No. 5,116,742. Alternatively, LIG46 or LIG56 mRNA can be used to select a catalytic RNA having specific ribonuclease activity from a pool of RNA molecules. See, e.g., Bartel and Szostak (1993) *Science* 261:1411-1418.

Nucleic acid molecules which form triple helical structures are useful. For example, LIG46 or LIG56 gene expression can be inhibited by targeting nucleotide sequences complementary to the regulatory region of the LIG46 or LIG56 (e.g., the LIG46 or LIG56 promoter and/or enhancers) to form triple helical structures that prevent transcription of the LIG46 or LIG56 gene in target cells. See generally, Helene (1991) *Anticancer Drug Des*. 6(6):569-84; Helene (1992) *Ann. N.Y. Acad. Sci.* 660:27-36; and Maher (1992) *Bioassays* 14(12):807-15.

In preferred embodiments, the nucleic acid molecules can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (see Hyrup et al. (1996) *Bioorganic & Medicinal Chemistry* 4(1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al. (1996) *supra;* Perry-O'Keefe et al. (1996) *Proc. Natl. Acad. Sci. USA* 93: 14670-675.

PNAs of LIG46 or LIG56 can be used in therapeutic and diagnostic applications. For example, PNAs can be used as antisense or antigene agents for sequence-specific modulation of gene expression by, e.g., inducing transcription or translation arrest or inhibiting replication. PNAs of LIG46 or LIG56 can also be used, e.g., in the analysis of single base pair mutations in a gene by, e.g., PNA directed PCR clamping; as 'artificial restriction enzymes when used in combination with other enzymes, e.g., S1 nucleases (Hyrup (1996) supra; or as probes or primers for DNA sequence and hybridization (Hyrup (1996) supra; Perry-O'Keefe et al. (1996) *Proc*. *Natl*. *Acad. Sci. USA* 93: 14670-675).

PNAs of LIG46 or LIG56 can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of LIG46 or LIG56 can be generated which may combine the advantageous properties of PNA and DNA. Such chimeras allow DNA recognition enzymes, e.g., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996) *supra*). The synthesis of PNA-DNA chimeras can be performed as described in Hyrup (1996) supra and Finn et al. (1996) *Nucleic Acids Research* 24(17):3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, can be used as a between the PNA and the 5' end of DNA (Mag et al. (1989) *Nucleic Acid Res.* 17:5973-88). PNA monomers are then coupled in a stepwise manner to produce a chimeric molecule with a 5' PNA segment and a 3' DNA segment (Finn et al. (1996) *Nucleic Acids Research* 24(17):3357-63). Alternatively, chimeric molecules can be synthesized with a 5' DNA segment and a 3' PNA segment (Peterser et al. (1975) *Bioorganic Med. Chem. Lett.* 5:1119-11124).

In other embodiments, the oligonucleotide may include other appended groups such as peptides (e.g., for targeting host cell receptors *in vivo*), or agents facilitating transport across the cell membrane (see, e.g., Letsinger et al. (1989) *Proc. Natl. Acad. Sci. USA* 86:6553-6556; Lemaitre et al. (1987) *Proc. Natl. Acad. Sci. USA* 84:648-652; PCT Publication No. WO 88/09810) or the blood-brain barrier (see, e.g., PCT Publication No. WO 89/10134). In addition, oligonucleotides can be modified with hybridization-triggered cleavage agents (*see, e*.*g*., Krol et al. (1988) *Bio*/*Techniques* 6:958-976) or intercalating agents (*see, e.g*., Zon (1988) *Pharm*. *Res*. 5:539-549). To this end, the oligonucleotide may be conjugated to another molecule, e.g., a peptide, hybridization triggered cross-linking agent, transport agent, hybridization-triggered cleavage agent, etc.

### II. Isolated LIG46 Proteins, Isolated LIG56 Proteins, Anti-LIG46 antibodies, and Anti-LIG56 Antibodies

Isolated LIG46 or LIG56 proteins, and biologically active portions thereof are useful, as well as polypeptide fragments suitable for use as immunogens to raise anti-LIG46 or LIG56 antibodies. Native LIG46 or LIG56 proteins can be isolated from cells or tissue sources by an appropriate purification scheme using standard protein purification techniques. Alternatively, LIG46 or LIG56 proteins are produced by recombinant DNA techniques. Alternative to recombinant expression, a LIG46 or LIG56 protein or polypeptide can be synthesized chemically using standard peptide synthesis techniques.

This section focusses on LIG46 and LIG56 polypeptides, antibodies, and their use. However, Tgtp, LRG-47, RC10-II, and Stra13 polypeptides and antibodies (and fragments or variants thereof) are useful in the methods of the invention as are fusion proteins which include all or a portion of Tgtp, LRG-47, RC10-II, or Stra13. Thus, the methods described in this section for the production and use of LIG46 and LIG56 polypeptides and variants thereof apply to Tgtp, LRG-47, RC10-II, and Stra13. Antibodies directed against Tgtp, LRG-47, RC10-II, or Stra13 are useful in the method of the invention. For example, such antibodies can be used to measure expression of Tgtp, LRG-47, RC10-II, or Stra13 in screening assays designed to identify agents which modulate expression or activity of Tgtp, LRG-47, RC10-II, or Stra13. The description methods for preparing and characterizing anti-LIG46 and anti-LIG56 antibodies presented below can be applied to antibodies directed against Tgtp, LRG-47, RC10-II, or Stra13.

An "isolated" or "purified" protein or biologically active portion thereof is substantially free of cellular material or other contaminating proteins from the cell or tissue source from which the protein of interest is derived (e.g., LIG46 or LIG56), or substantially free from chemical precursors or other chemicals when chemically synthesized. The language "substantially free of cellular material" includes preparations in which the protein is separated from cellular components of the cells from which it is isolated or recombinantly produced- Thus, LIG46 or LIG56 protein that is substantially free of cellular material includes preparations of LIG46 or LIG56 protein having less than about 30%, 20%, 10%, or 5% (by dry weight) of non-LIG46 or LIG56 protein (also referred to herein as a "contaminating protein"). When the LIG46 or LIG56 protein or biologically active portion thereof is recombinantly produced, it is also preferably substantially free of culture medium, i.e., culture medium represents less than about 20%, 10%, or 5% of the volume of the protein preparation. When LIG46 or LIG56 protein is produced by chemical synthesis, it is preferably substantially free of chemical precursors or other chemicals, i.e., it is separated from chemical precursors or other chemicals which are involved in the synthesis of the protein. Accordingly such preparations of LIG46 or LIG56 protein have less than about 30%, 20%, 10%, 5% (by dry weight) of chemical precursors or non-LIG46 or LIG56 chemicals.

Biologically active portions of a LIG46 or LIG56 protein include peptides comprising amino acid sequences sufficiently identical to or derived from the amino acid sequence of the LIG46 or LIG56 protein, which include less amino acids than the full length LIG46 or LIG56 proteins, and exhibit at least one activity of a LIG46 or LIG56 protein. Typically, biologically active portions comprise a domain or motif with at least one activity of the LIG46 or LIG56 protein. A biologically active portion of a LIG46 or LIG56 protein can be a polypeptide which is, for example, 10, 25, 50, 100 or more amino acids in length. Preferred biologically active polypeptides include one or more identified LIG46 or LIG56 structural domains.

Moreover, other biologically active portions, in which other regions of the protein are deleted, can be prepared by recombinant techniques and evaluated for one or more of the functional activities of a native LIG46 or LIG56 protein.

Preferred LIG46 and LIG56 proteins have or are substantially identical to the amino acid sequences disclosed herein. Preferred proteins are substantially identical to those disclosed herein and retain the functional activity of the protein yet differ in amino acid sequence due to natural allelic variation or mutagenesis.

Accordingly, a useful LIG46 protein is a protein which includes an amino acid sequence at least about 45%, preferably 55%, 65%, 75%, 85%, 95%, or 99% identical to the amino acid sequence of SEQ ID NO:2 (or SEQ ID NO:4) and retains the functional activity of the LIG46 protein of SEQ ID NO:2 (or SEQ ID NO:4). In other instances, the LIG46 protein is a protein having an amino acid sequence 55%, 65%, 75%, 85%, 95%, or 98% identical to a portion of LIG46 having homology to a galactosyltransferase (e.g., amino acids 192-353, 142-184, 201-296, 289-347, 140-183, 367-391, 177-266, 299-343, or 140-184 of SEQ ID NO:2) or a neurogenic secreted signalling protein (e.g., amino acids 200-291, 270-354, 144-183, 380-394, or 211-248 of SEQ ID NO:2). In a preferred embodiment, the LIG46 protein retains a functional activity of the LIG46 protein of SEQ ID NO:2 (or SEQ ID NO:4).

A useful LIG56 protein is a protein which includes an amino acid sequence at least about 45%, preferably 55%, 65%, 75%, 85%, 95%, or 99% identical to the amino acid sequence of SEQ ID NO:6 and retains the functional activity of the LIG46 protein of SEQ ID NO:6.

To determine the percent identity of two amino acid sequences or of two nucleic acids, the sequences are aligned for optimal comparison purposes (e.g., gaps can be introduced in the sequence of a first amino acid or nucleic' acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity =.# of identical positions/total # of positions x 100) .

The determination of percent homology between two sequences can be accomplished using a mathematical algorithm. A preferred, non-limiting example of a mathematical algorithm utilized for the comparison of two sequences is the algorithm of Karlin and Altschul (1990) *Proc. Nat'l Acad. Sci. USA* 87:2264-2268, modified as in Karlin and Altschul (1993) *Proc. Nat'l Acad. Sci. USA* 90:5873-5877. Such an algorithm is incorporated into the NBLAST and XBLAST programs of Altschul, et al. (1990) *J. Mol. Biol*. 215:403-410. BLAST nucleotide searches can be performed with the NBLAST program, score = 100, wordlength = 12 to obtain nucleotide sequences homologous to LIG46 or LIG56 nucleic acid molecules of the invention. BLAST protein searches can be performed with the XBLAST program, score = 50, wordlength = 3 to obtain amino acid sequences homologous to LIG46 or LIG56 protein molecules of the invention. To obtain gapped alignments for comparison purposes, Gapped BLAST can be utilized as described in Altschul et al., (1997) *Nucleic Acids Res*. 25:3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithm utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, only exact matches are counted.

The invention also uses LIG46 or LIG56 chimeric or fusion proteins. As used herein, a LIG46 or LIG56 "chimeric protein" or "fusion protein" comprises a LIG46 or LIG56 polypeptide operatively linked to a non-LIG46 or LIG56 polypeptide. A "LIG46 or LIG56 polypeptide" refers to a polypeptide having an amino acid sequence corresponding to LIG46 or LIG56, whereas a "non-LIG46 or LIG56 polypeptide" refers to a polypeptide having an amino acid sequence corresponding to a protein which is not substantially identical to the LIG46 or LIG56 protein, e.g., a protein which is different from the LIG46 or LIG56 protein and which is derived from the same or a different organism. Within a LIG46 or LIG56 fusion protein the LIG46 or LIG56 polypeptide can correspond to all or a portion of a LIG46 or LIG56 protein, preferably at least one biologically active portion of a LIG46 or LIG56 protein. Within the fusion protein, the term "operatively linked" is intended to indicate that the LIG46 or LIG56 polypeptide and the non-LIG46 or LIG56 polypeptide are fused in-frame to each other. The non-LIG46 or LIG56 polypeptide can be fused to the N-terminus or C-terminus of the LIG46 or LIG56 polypeptide.

One useful fusion protein is a GST-LIG46 or LIG56 fusion protein in which the LIG46 or LIG56 sequences are fused to the C-terminus of the GST sequences. Such fusion proteins can facilitate the purification of recombinant LIG46 or LIG56.

In another embodiment, the fusion protein is a LIG46 protein containing a heterologous signal sequence at its N-terminus. For example, the native LIG46 signal sequence (i.e., about amino acids 1 to 32 of SEQ ID NO:2) can be removed and replaced with a signal sequence from another protein. In certain host cells (e.g., mammalian host cells), expression and/or secretion of LIG46 can be increased through use of a heterologous signal sequence. For example, the gp67 secretory sequence of the baculovirus envelope protein can be used as a heterologous signal sequence (*Current Protocols in Molecular Biology,* Ausubel et al., eds., John Wiley & Sons, 1992). Other examples of eukaryotic heterologous signal sequences include the secretory sequences of melittin and human placental alkaline phosphatase (Stratagene; La Jolla, California). In yet another example, useful prokaryotic heterologous signal sequences include the phoA secretory signal (*Molecular cloning*, Sambrook et al, second edition, Cold spring harbor laboratory press, 1989) and the protein A secretory signal (Pharmacia Biotech; Piscataway, New Jersey).

In yet another embodiment, the fusion protein is an LIG46 or LIG56-immunoglobulin fusion protein in which all or part of LIG46 or LIG56 is fused to sequences derived from a member of the immunoglobulin protein family. The LIG46-immunoglobulin fusion proteins can be incorporated into pharmaceutical compositions and administered to a subject to inhibit an interaction between a LIG56 ligand and a LIG56 protein on the surface of a cell, to thereby suppress LIG56-mediated signal transduction *in vivo*. The LIG56-immunoglobulin fusion proteins can be used to affect the bioavailability of a LIG56 cognate ligand. Moreover, the LIG56-immunoglobulin fusion proteins can be used as immunogens to produce LIG56 antibodies in a subject, to purify LIG56 ligands and in screening assays to identify molecules which inhibit the interaction of LIG56 with a LIG56 ligand. LIG46 fusion proteins can be used in an analogous manner.

Preferably, a LIG46 or LIG56 chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, e.g., *Current Protocols in Molecular Biology*, Ausubel et al. eds., John Wiley & Sons: 1992). Moreover, many expression vectors are commercially available that already encode a fusion moiety (e.g., a GST polypeptide). An LIG46- or LIG56-encoding nucleic acid can be cloned into such an expression vector such that the fusion moiety is linked in-frame to the LIG46 or LIG56 protein.

The present invention uses variants of the LIG46 or LIG56 proteins which function as either LIG46 or LIG56 agonists (mimetics) or as LIG46 or LIG56 antagonists. Variants of the LIG46 or LIG56 protein can be generated by mutagenesis, e.g., discrete point mutation or truncation of the LIG46 or LIG56 protein. An agonist of the LIG46 or LIG56 protein can retain substantially the same, or a subset, of the biological activities of the naturally occurring form of the LIG46 or LIG56 protein. An antagonist of the LIG46 or LIG56 protein can inhibit one or more of the activities of the naturally occurring form of the LIG46 or LIG56 protein by, for example, competitively binding to a downstream or upstream member of a cellular signaling cascade which includes the LIG46 or LIG56 protein. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the LIG46 or LIG56 proteins. Variants of the LIG46 or LIG56 protein which function as either LIG46 or LIG56 agonists (mimetics) or as LIG46 or LIG56 antagonists can be identified by screening combinatorial libraries of mutants, e.g., truncation mutants, of the LIG46 or LIG56 protein for LIG46 or LIG56 protein agonist or antagonist activity. In one embodiment, a variegated library of LIG46 or LIG56 variants is generated by combinatorial mutagenesis at the nucleic acid level and is encoded by a variegated gene library. A variegated library of LIG46 or LIG56 variants can be produced by, for example, enzymatically ligating a mixture of synthetic oligonucleotides into gene sequences such that a degenerate set of potential LIG46 or LIG56 sequences is expressible as individual polypeptides, or alternatively, as a set of larger fusion proteins (e.g., for phage display) containing the set of LIG46 or LIG56 sequences therein. There are a variety of methods which can be used to produce libraries of potential LIG46 or LIG56 variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential LIG46 or LIG56 sequences. Methods for synthesizing degenerate oligonucleotides are known in the art (*see*, *e.g.,* Narang (1983) *Tetrahedron* 39:3; Itakura et al. (1984) *Annu. Rev. Biochem.* 53:323; Itakura et al. (1984) *Science* 198:1056; Ike et al. (1983) *Nucleic Acid Res.* 11:477).

In addition, libraries of fragments of the LIG46 or LIG56 protein coding sequence can be used to generate a variegated population of LIG46 or LIG56 fragments for screening and subsequent selection of variants of a LIG46 or LIG56 protein. In one embodiment, a library of coding sequence fragments can be generated by treating a double stranded PCR fragment of a LIG46 or LIG56 coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the LIG46 or LIG56 protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of LIG46 or LIG56 proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify LIG46 or LIG56 variants (Arkin and Yourvan (1992) *Proc. Natl. Acad. Sci. USA 89*:7811-7815; Delgrave et al. (1993) Protein Engineering 6(3):327-331).

An isolated LIG46 or LIG56 protein, or a portion or fragment thereof, can be used as an immunogen to generate antibodies that bind LIG46 or LIG56 using standard techniques for polyclonal and monoclonal antibody preparation. The full-length LIG46 or LIG56 protein can be used or, alternatively, the invention provides antigenic peptide fragments of LIG46 or LIG56 for use as immunogens. The antigenic peptide of LIG46 or LIG56 comprises at least 8 (preferably 10, 15, 20, or 30) amino acid residues of the amino acid sequence shown in SEQ ID NO:2 and encompasses an epitope of LIG46 or LIG56 such that an antibody raised against the peptide forms a specific immune complex with LIG46 or LIG56.

Preferred epitopes encompassed by the antigenic peptide are regions of LIG46 or LIG56 that are located on the surface of the protein, e.g., hydrophilic regions. Hydrophilic regions and antigenic regions can be identified using standard analytical tools well-known to those skilled in the art.

A LIG46 or LIG56 immunogen typically is used to prepare antibodies by immunizing a suitable subject, (e.g., rabbit, goat, mouse or other mammal) with the immunogen. An appropriate immunogenic preparation can contain, for example, recombinantly expressed LIG46 or LIG56 protein or a chemically synthesized LIG46 or LIG56 polypeptide. The preparation can further include an adjuvant, such as Freund's complete or incomplete adjuvant, or similar immunostimulatory agent. Immunization of a suitable subject with an immunogenic LIG46 or LIG56 preparation induces a polyclonal anti-LIG46 or LIG56 antibody response.

Accordingly, anti-LIG46 or LIG56 antibodies are useful. The term "antibody" as used herein refers to immunoglobulin molecules and immunologically active portions of immunoglobulin molecules, i.e., molecules that contain an antigen binding site which specifically binds an antigen, such as LIG46 or LIG56. A molecule which specifically binds to LIG46 or LIG56 is a molecule which binds LIG46 or LIG56, but does not substantially bind other molecules in a sample, e.g., a biological sample, which naturally contains LIG46 or LIG56. Examples of immunologically active portions of immunoglobulin molecules include F(ab) and F(ab')₂ fragments which can be generated by treating the antibody with an enzyme such as pepsin.

Polyclonal and monoclonal antibodies that bind LIG46 or LIG56 are described herein. The term "monoclonal antibody" or "monoclonal antibody composition", as used herein, refers to a population of antibody molecules that contain only one species of an antigen binding site capable of immunoreacting with a particular epitope of LIG46 or LIG56. A monoclonal antibody composition thus typically displays a single binding affinity for a particular LIG46 or LIG56 protein with which it immunoreacts.

Polyclonal anti-LIG46 or LIG56 antibodies can be prepared as described above by immunizing a suitable subject with a LIG46 or LIG56 immunogen. The anti-LIG46 or LIG56 antibody titer in the immunized subject can be monitored over time by standard techniques, such as with an enzyme linked immunosorbent assay (ELISA) using immobilized LIG46 or LIG56. If desired, the antibody molecules directed against LIG46 or LIG56 can be isolated from the mammal (e.g., from the blood) and further purified by well-known techniques, such as protein A chromatography to obtain the IgG fraction. At an appropriate time after immunization, e.g., when the anti-LIG46 or LIG56 antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (Kozbor et al. (1983) *Immunol Today* 4:72), the EBV-hybridoma technique (Cole et al. (1985). *Monoclonal Antibodies and Cancer Therapy,* Alan R. Liss, Inc., pp. 77-96) or trioma techniques. The technology for producing various antibodies monoclonal antibody hybridomas is well known (see generally current Protocols in Immunology (1994) Coligan et al. (eds.) John Wiley & Sons, Inc. , New York, NY). Briefly, an immortal cell line (typically a myeloma) is fused to lymphocytes (typically splenocytes) from a mammal immunized with a LIG46 or LIG56 immunogen as described above, and the culture supernatants of the resulting hybridoma cells are screened to identify a hybridoma producing a monoclonal antibody that binds LIG46 or LIG56.

Any of the many well known protocols used for fusing lymphocytes and immortalized cell lines can be applied for the purpose of generating an anti-LIG46 or LIG56 monoclonal antibody (see, e.g., Current Protocols in Immunology, *supra*; Galfre et al. (1977) *Nature* 266:55052; R.H. Kenneth, in *Monoclonal Antibodies: A New Dimension In Biological Analyses*, Plenum Publishing Corp., New York, New York (1980); and Lerner (1981) Yale *J. Biol. Med.,* 54:387-402. Moreover, the ordinarily skilled worker will appreciate that there are many variations of such methods which also would be useful. Typically, the immortal cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line, e.g., a myeloma cell line that is sensitive to culture medium containing hypoxanthine, aminopterin and thymidine ("HAT medium"). Any of a number of myeloma cell lines can be used as a fusion partner according to standard techniques, e.g., the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind LIG46 or LIG56, e.g., using a standard ELISA assay.

Alternative to preparing monoclonal antibody-secreting hybridomas, a monoclonal anti-LIG46 or LIG56 antibody can be identified and isolated by screening a recombinant combinatorial immunoglobulin library (e.g., an antibody phage display library) with LIG46 or LIG56 to thereby isolate immunoglobulin library members that bind LIG46 or LIG56. Kits for generating and screening phage display libraries are commercially available (e.g., the Pharmacia *Recombinant Phage Antibody System*, Catalog No. 27-9400-01; and the Stratagene *SurfZAP™ Phage Display Kit*, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) *Bio*/*Technology* 9:1370-1372; Hay et al. (1992) *Hum. Antibod. Hybridomas* 3:81-85; Huse et al. (1989) *Science* 246:1275-1281; Griffiths et al. (1993) *EMBO J* 12:725-734.

Additionally, recombinant anti-LIG46 or LIG56 antibodies, such as chimeric and humanized monoclonal antibodies, comprising both human and non-human portions, can be made using standard recombinant DNA techniques' . Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al. (1988) *Science* 240:1041-1043; Liu et al. (1987) *Proc. Natl. Acad. Sci. USA* 84:3439-3443; Liu et al. (1987) *J. Immunol.* 139:3521-3526; Sun et al. (1987) *Proc. Natl. Acad. Sci. USA* 84:214-218; Nishimura et al. (1987) *Canc. Res.* 47:999-1005; Wood et al. (1985) Nature 314:446-449; and Shaw et al. (1988) *J. Natl. Cancer Inst.* 80:1553-1559); Morrison, (1985) *Science* 229:1202-1207; Oi et al. (1986) *Bio*/*Techniques* 4:214; U.S. Patent 5,225,539; Jones et al. (1986) *Nature* 321:552-525; Verhoeyan et al. (1988) *Science* 239:1534; and Beidler et al. (1988) *J. Immunol*. 141:4053-4060.

An anti-LIG46 or LIG56 antibody (e.g., monoclonal antibody) can be used to isolate LIG46 or LIG56 by standard techniques, such as affinity chromatography or immunoprecipitation. An anti-LIG46 or LIG56 antibody can facilitate the purification of natural LIG46 or LIG56 from cells and of recombinantly produced LIG46 or LIG56 expressed in host cells. Moreover, an anti-LIG46 or LIG56 antibody can be used to detect LIG46 or LIG56 protein (e.g., in a cellular lysate or cell supernatant) in order to evaluate the abundance and pattern of expression of the LIG46 or LIG56 protein. Anti-LIG46 or LIG56 antibodies can be used diagnostically to monitor protein levels in tissue as part of a clinical testing procedure, e.g., to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Completely human antibodies are particularly desirable for therapeutic treatment of human patients. Such antibodies can be produced using transgenic mice which are incapable of expressing endogenous immunoglobulin heavy and light chains genes, but which can express human heavy and light chain genes. The transgenic mice are immunized in the normal fashion with a selected antigen. Monoclonal antibodies directed against the antigen can be obtained using conventional hybridoma technology. The human immunoglobulin transgenes of harbored by the transgenic mice rearrange during B cell differentiation, subsequently undergo class switching and somatic mutation. Thus, using such a technique, it is possible to produce therapeutically useful IgG, IgA and IgE antibodies. For an overview of this technology for producing human antibodies, see Lonberg and Huszar (1995, *Int. Rev. Immunol*. 13:65-93). For a detailed discussion of this technology for producing human antibodies and human monoclonal antibodies and protocols for producing such antibodies, see, e.g., U.S. Patent 5.625,126; U.S. Patent 5,633,425; U.S. Patent 5,569,825; U.S. Patent 5,661,016; and U.S. Patent 5,545,806. Human antibodies directed against a selected antigen can be provided by Abgenix, Inc. (Fremont, CA) and GenPharm. Inc. (Palo Alto, CA).

### III. Recombinant Expression Vectors and Host cells

Vectors, preferably expression vectors, containing a nucleic acid encoding LIG46 or LIG56 (or a portion thereof) are useful.

The techniques described below can also be applied to host cells and vectors used to express Tgtp, LRG-47, RC10-II, and Stra13 for use in the production of recombinant protein or transgenic animals. Thus, although the this section refers to LIG46 and LIG56, the methods described can be applied to Tgtp, LRG-47, RC10-II, and Stra13.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome, Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operatively linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions.

The recombinant expression vectors comprise a nucleic acid in a form suitable for expression of the nucleic acid in a host cell, which means chat the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; *Gene Expression Technology: Methods in Enzymology* 185, Academic Press, San Diego, CA (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors can be introduced into host cells to thereby produce proteins or peptides, including fusion proteins or peptides, encoded by nucleic acids as described herein (e.g., LIG46 or LIG56 proteins, mutant forms of LIG46 or LIG56, fusion proteins, etc.).

The recombinant expression vectors can be designed for expression of LIG46 or LIG56 in prokaryotic or eukaryotic cells, e.g., bacterial cells such as *E. coli*, insect cells (using baculovirus expression vectors) yeast cells or mammalian cells. Suitable host cells are discussed further in Goeddel, *Gene Expression Technology: Methods in Enzymology* 185, Academic Press, San Diego, CA (1990). Alternatively, the recombinant expression vector can be transcribed and translated *in vitro*, for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Examples of suitable inducible non-fusion *E. coli* expression vectors include pTrc (Amann et al., (1988) Gene 69:301-315) and pET 11d (Studier et al., Gene *Expression Technology: Methods in Enzymology* 185, Academic Press, San Diego, California (1990) 60-69). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident λ prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, *Gene* Expression Technology: Methods in Enzymology 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada et al. (1992) Nucleic *Acids Res.* 20:2112-2118). Such alteration of nucleic acid sequences of the invention can be carried out by standard DNA synthesis techniques.

In another embodiment, the LIG46 or LIG56 expression vector is a yeast expression vector. Examples of vectors for expression in yeast S. cerivisae include pYepSecl (Baldari et al. (1987) *EMBO J*. 6:229-234), pMFa (Kurjan and Herskowitz, (1982) *Cell* 30:933-943), pJRY88 (Schultz et al. (1987) *Gene* 54:113-123). pYES2 (Invitrogen Corporation, San Diego, CA), and picZ (InVitrogen Corp, San Diego, CA).

Alternatively, LIG46 or LIG56 can be expressed in insect cells using baculovirus expression vectors. Baculovirus vectors available for expression of proteins in cultured insect cells (e.g., Sf 9 cells) include the pAc series (Smith et al. (1983) *Mol. Cell Biol*. 3:2156-216S) and the pVL series (Lucklow and Summers (1989) *Virology* 170:31-39).

In yet another embodiment, a nucleic acid of the invention is expressed in mammalian cells using a mammalian expression vector. Examples of mammalian expression vectors include pCDM8 (Seed (1987) Nature 329:840) and pMT2PC (Kaufman et al. (1987) *EMBO J*. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al. (*supra*).

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) *Genes* *Dev.* 1:268-277), lymphoid-specific promoters (Calame and Eaton (1988) *Adv. Immunol*. 43:235-275), in particular promoters of T cell receptors (Winoto and Baltimore (1989) *EMBO J*. 8:729-733) and immunoglobulins (Banerji et al. (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA* 86:5473-5477), pancreas-specific promoters (Edlund et al. (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss (1990) *Science* 249:374-379) and the α-fetoprotein promoter (Campes and Tilghman (1989) *Genes Dev*. 3:537-546).

A recombinant expression vector is described herein, the vector comprising a DNA molecule cloned into the expression vector in an antisense orientation. That is, the DNA molecule is operatively linked to a regulatory sequence in a manner which allows for expression (by transcription of the DNA molecule) of an RNA molecule which is antisense to LIG46 or LIG56 mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub et al. (Reviews - Trends in Genetics, Vol. 1(1) 1986).

Host cells are described herein in which a recombinant expression vector has been introduced The terms "host cell" and "recombinant host cell" are use interchangeably herein.

It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, LIG46 or LIG56 protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook, et al. (*supra*), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding LIG46 or LIG56 or can be introduced on a separate vector. Cells stably transfected with the introduced nucleic acid can be identified by drug selection (e.g., cells that have incorporated the selectable marker gene will survive, while the other cells die).

A host cell, such as a prokaryotic or eukaryotic host cell in culture, can be used to produce (i.e., express) LIG46 or LIG56 protein. Accordingly, methods for producing LIG46 or LIG56 protein a described herein, the methods using the host cells describe above. In one embodiment, the method comprises culturing the host cell (into which a recombinant expression vector encoding LIG46 or LIG56 has been introduced) in a suitable medium such that LIG46 or LIG56 protein is produced. In another embodiment, the method further comprises isolating LIG46 or LIG56 from the medium or the host cell.

The host cells can also be used to produce non-human transgenic animals which overexpress a protein of interest. For example, in one embodiment, a host cell is a fertilized oocyte or an embryonic stem cell into which a nucleic acid molecule which directs high level expression of LIG46, LIG56, Tgtp, LRG-47, RC10-II, or Stra13 has been introduced. Such host cells can then be used to create non-human transgenic animals in which LIG46, LIG56, Tgtp, LRG-47, RC10-II, or Stra13 sequences have been introduced into their genome or homologous recombinant animals in which endogenous LIG46, LIG56, Tgtp, LRG-47, RC10-II, or Stra13 sequences have been altered. Such animals are useful for studying the function and/or activity of LIG46, LIG56, Tgtp, LRG-47, RC10-II, or Stra13 and for identifying and/or evaluating modulators of LIG46 or LIG56 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, an "homologous recombinant animal" is a non-human animal, preferably a mammal, more preferably a mouse, in which an endogenous LIG46, LIG56, Tgtp, LRG-47, RC10-II, or Stra13 gene has been altered by homologous recombination between the endogenous gene and an exogenous DNA molecule introduced into a cell of the animal, e.g., an embryonic cell of the animal, prior to development of the animal.

A transgenic animal can be created by introducing a nucleic acid molecule encoding a desired protein into the male pronuclei of a fertilized oocyte, e.g., by microinjection, retroviral infection, and allowing the oocyte to develop in a pseudopregnant female foster animal. The cDNA sequence can be introduced as a transgene into the genome of a non-human animal. Alternatively, a human homologue of the LIG46, LIG56, Tgtp, LRG-47, RC10-II, or Stra13 gene can be isolated based on hybridization to the murine LIG46, LIG56, Tgtp, LRG-47, RC10-II, or Stra13 cDNA and used as a transgene. Intronic sequences and polyadenylation signals can also be included in the transgene to increase the efficiency of expression of the transgene. A tissue-specific regulatory sequence(s) can be operably linked to the transgene to direct expression of the protein to particular cells. Methods for generating transgenic animals via embryo manipulation and microinjection, particularly animals such as mice, have become conventional in the art and are described, for example, in U.S. Patent Nos. 4,736,866 and 4,870,009, U.S. Patent No. 4,873,191 and in Hogan, *Manipulating the Mouse Embryo*, (Cold Spring Harbor Laboratory Press, Cold Spring Harbor, N.Y., 1986). Similar methods are used for production of other transgenic animals. A transgenic founder animal can be identified based upon the presence of the transgene in its genome and/or expression of the mRNA in tissues or cells of the animals. A transgenic founder animal can then be used to breed additional animals carrying the transgene. Moreover, transgenic animals carrying a transgene encoding LIG46, LIG56, Tgtp, LRG-47, RC10-II, or Stra13 can further be bred to other transgenic animals carrying other transgenes.

To create an homologous recombinant animal, a vector is prepared which contains at least a portion of a LIG46, LIG56, Tgtp, LRG-47, RC10-II, or Stra13 gene into which a deletion, addition or substitution has been introduced to thereby alter, e.g., functionally disrupt, the gene. In a preferred embodiment, the vector is designed such that, upon homologous recombination, the endogenous gene is functionally disrupted (i.e., no longer encodes a functional protein; also referred to as a "knock out" vector). Alternatively, the vector can be designed such that, upon homologous recombination, the endogenous gene is mutated or otherwise altered but still encodes functional protein (e.g., the upstream regulatory region can be altered to thereby alter the expression of the endogenous protein). In the homologous recombination vector, the altered portion of the gene is flanked at its 5' and 3' ends by additional nucleic acids of the gene to allow for homologous recombination to occur between the exogenous gene carried by the vector and an endogenous gene in an embryonic stem cell. The additional flanking nucleic acid is of sufficient length for successful homologous recombination with the endogenous gene. Typically, several kilobases of flanking DNA (both at the 5' and 3' ends) are included in the vector (*see, e.g*., Thomas and Capecchi (1987) *Cell* 51:503 for a description of homologous recombination vectors). The vector is introduced into an embryonic stem cell line (e.g., by electroporation) and cells in which the introduced gene has homologously recombined with the endogenous gene are selected (*see, e*.*g*., Li et al. (1992) *Cell* 69:915). The selected cells are then injected into a blastocyst of an animal (e.g., a mouse) to form aggregation chimeras (*see, e.g*., Bradley in *Teratocarcinomas and Embryonic Stem Cells: A Practical Approach,* Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley (1991) *Current Opinion in Bio*/*Technology* 2:823-829 and in PCT Publication Nos. WO 90/11354, WO 91/01140, WO 92/0968, and WO 93/04169.

In another embodiment, transgenic non-human animals can be produced which contain selected systems which allow for regulated expression of the transgene. One example of such a system is the *cre*/*loxP* recombinase system of bacteriophage P1. For a description of the *cre*/*loxP* recombinase system, see, e.g., Lakso et al. (1992) *Proc. Natl. Acad. Sci. USA* 89:6232-6236. Another example of a recombinase system is the FLP recombinase system of *Saccharomyces cerevisiae* (O'Gorman et al. (1991) *Science* 251:1351-1355. If a *cre*/*loxP* recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut et al. (1997) *Nature* 385:810-813 and PCT Publication Nos. WO 97/07668 and WO 97/07669. In brief, a cell, e.g., a somatic cell, from the transgenic animal can be isolated and induced to exit the growth cycle and enter Gₒ phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, e.g., the somatic cell, is isolated.

### IV. Pharmaceutical Compositions

The LIG46 and LIG56 nucleic acid molecules, LIG46 and LIG56 proteins, and anti-LIG46 and anti- LIG56 antibodies (also referred to herein as "active compounds") can be incorporated into pharmaceutical compositions suitable for administration as can various modulators of LIG46, LIG56, Tgtp, LRG-47, RC10-II, or Stra13 expression or activity.

Therapeutic compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF; Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a LIG46 or LIG56 protein or anti-LIG46 or LIG56 antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For.the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means- For transmucosal or transdermal administration, penetrants appropriate to the barrier to be permeated are used in the formulation. Such penetrants are generally known in the art, and include, for example, for transmicosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No: 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules described above can be inserted into vectors and used as gene therapy vectors. Gene therapy vectors can be delivered to a subject by, for example, intravenous injection, local administration (U.S. Patent 5,328,470) or by stereotactic injection (*see, e.g.,* Chen et al. (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

### V. Uses and Methods of the Invention

The LIG46, LIG56, Tgtp, LRG-47, RC10-II, and Stra13 nucleic acid molecules, proteins, protein homologues, and antibodies described herein can be used in one or more of the following methods: a) screening assays; b) detection assays (e.g., chromosomal mapping, tissue typing, forensic biology); and c) methods of treatment (e.g., therapeutic and prophylactic). The isolated nucleic acid molecules of the invention can be used to express LIG46, LIG56, Tgtp, LRG-47, RC10-II, or Stra13 protein (e.g., via a recombinant expression vector in a host cell in gene therapy applications or transgenic animals), to detect LIG46, LIG56, Tgtp, LRG-47, RC10-II, or Stra13 mRNA (e.g., in a biological sample) or a genetic lesion in a LIG46, LIG56, Tgtp, LRG-47, RC10-II, or Stra13 gene, and to modulate LIG46, LIG56, Tgtp, LRG-47, RC10-II, or Stra13 activity or expression. In addition, LIG46, LIG56, Tgtp, LRG-47, RC10-II, or Stra13 proteins can be used to screen drugs or compounds which modulate LIG46, LIG56, Tgtp, LRG-47, RC10-II, or Stra13 activity or expression as well as to treat disorders characterized by insufficient or excessive production of LIG46, LIG56, Tgtp, LRG-47, RC10-II, or Stra13 protein or production of LIG46, LIG56, Tgtp, LRG-47, RC10-II, or Stra13 protein forms which have an undesirable level of activity compared to the wild type protein. In addition, the anti-LIG46, LIG56, Tgtp, LRG-47, RC10-II, or Stra13 antibodies of the invention can be used to detect and isolate LIG46, LIG56, Tgtp, LRG-47, RC10-II, or Stra13 proteins and modulate LIG46, LIG56, Tgtp, LRG-47, RC10-II, or Stra13 activity.

Novel agents identified by the above-described screening assays may be used for treatments as described above.

### A. Screening Assays

The invention provides a method (also referred to herein as a "screening assay") for identifying modulators, i.e., candidate or test compounds or agents (e.g., peptides, peptidomimetics, small molecules or other drugs) which bind to a LIG46, LIG56, Tgtp, LRG-47. RC10-II, or Stra13 protein and/or have a stimulatory or inhibitory effect on, for example, LIG46, LIG56, Tgtp, LRG-47, RC10-II, or Stra13 expression or activity.

The invention provides assays for screening candidate or test compounds which bind to or modulate the activity of the membrane-bound form of a LIG46 protein or polypeptide or biologically active portion thereof. Other embodiments entail the use of a soluble form of LIG46.

The test compounds of the present invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) Anticancer *Drug Des.* 12:145).

Examples of methods for the synthesis of molecular libraries can be found in the art, for example in: DeWitt et al. (1993) *Proc. Natl. Acad. Sci. U.S.A.* 90:6909; Erb et al. (1994) *Proc*. *Natl. Acad. Sci. USA* 91:11422; Zuckermann et al. (1994). *J. Med. Chem.* 37:2678; Cho et al. (1993) *Science* 261:1303; Carrell et al. (1994) *Angew. Chem. Int. Ed. Engl.* 33:2059; Carell et al. (1994) *Angew. Chem. Int. Ed. Engl*. 33:2061; and Gallop et al. (1994) *J. Med. Chem.* 37:1233.

Libraries of compounds may be presented in solution (e.g., Houghten (1992) *Bio*/*Techniques* 13:412-421), or on beads (Lam (1991) Nature 354:82-84), chips (Fodor (1993) Nature 364:555-556), bacteria (U.S. Patent No. 5,223,409), spores (Patent Nos. 5,571,698; 5,403,484; and 5,223,409), plasmids (Cull et al. (1992) *Proc*. *Natl. Acad. Sci. USA* 89:1865-1869) or on phage (Scott and Smith (1990) *Science* 249:386-390; Devlin (1990) *Science* 249:404-406; Cwirla et al. (1990) *Proc. Natl. Acad. Sci*. 87:6378-6382; and Felici (1991) *J. Mol. Biol*. 222:301-310).

The invention includes assays employing soluble LIG46, LIG56, Tgtp, LRG-47, RC10-II, or Stra13. Such assays, entail contacting a LIG46, LIG56, Tgtp, LRG-47, RC10-II, or Stra13 protein or biologically active portion thereof with a test compound and determining the ability of the test compound to bind to LIG46, LIG56, Tgtp, LRG-47, RC10-II, or Stra13 protein or biologically active portion thereof. Binding of the test compound to LIG46, LIG56, Tgtp, LRG-47, RC10-II, or Stra13 protein can be determined either directly or indirectly using the approaches described above. In a preferred embodiment, the assay includes contacting LIG46, LIG56, Tgtp, LRG-47, RC10-II, or Stra13 protein or biologically active portion thereof with a known compound which binds LIG46, LIG56, Tgtp, LRG-47, RC10-II, or Stra13 to form an assay mixture, contacting the assay mixture with a test compound, and determining the ability of the test compound to interact with LIG46, LIG56, Tgtp, LRG-47, RC10-II, or Stra13 protein, wherein determining the ability of the test compound to interact with LIG46, LIG56, Tgtp, LRG-47, RC10-II, or Stra13 protein comprises determining the ability of the test compound to preferentially bind to LIG46, LIG56, Tgtp, LRG-47, RC10-II, or Stra13 or biologically active portion thereof as compared to the known compound.

In another embodiment, an assay is a cell-free assay comprising contacting LIG46 or LIG56 protein or biologically active portion thereof with a test compound and determining the ability of the test compound to modulate (e.g., stimulate or inhibit) the activity of LIG46, LIG56, Tgtp, LRG-47, RC10-II, or Stra13 protein or a biologically active portion thereof. Determining the ability of the test compound to modulate the activity of LIG46, LIG56, Tgtp, LRG-47, RC10-II, or Stra13 can be accomplished, for example, by determining the ability of LIG46, LIG56, Tgtp, LRG-47, RC10-II, or Stra13 protein to bind to a LIG46, LIG56, Tgtp, LRG-47, RC10-II, or Stra13 by one of the methods described herein for determining direct binding. In an alternative embodiment, determining the ability of the teat compound to modulate the activity of LIG46, LIG56, Tgtp, LRG-47, RC10-II, or Stra13 can be accomplished by determining the ability of the agent to alter the activitry of LIG46, LIG56, Tgtp, LRG-47, RC10-II, or Stra13 target molecule. For example, the catalytic/enzymatic activity of the target molecule on an appropriate substrate can be determined.

In yet another embodiment, the cell-free assay comprises contacting the LIG46, LIG56, Tgtp, LRG-47, RC10-II, or Stra13 protein or biologically active portion thereof with a known compound which binds LIG46, LIG56, Tgtp, LRG-47, RC10-II, or Stra13 to form an assay mixture, contacting the assay mixture with a test compound, and determining the ability of the test compound to interact with a LIG46, LIG56, Tgtp, LRG-47, RC10-II, or Stra13 protein, wherein determining the ability of the test compound to interact with a LIG46, LIG56, Tgtp, LRG-47, RC10-II, or Stra13 protein comprises determining the ability of the LIG46, LIG56, Tgtp, LRG-47, RC10-II, or Stra13 protein to preferentially bind to or modulate the activity of a LIG46, LIG56, Tgtp, LRG-47, RC10-II, or Stra13 target molecule.

For membrane-bound proteins such as LIG46, in one embodiment, an assay is a cell-based assay in which a cell which expresses a membrane-bound form of LIG46 protein, or a biologically active portion thereof, on the cell surface is contacted with a test compound and the ability of the test compound to bind to a LIG46 protein determined. The cell, for example, can be a yeast cell or a cell of mammalian origin. Determining the ability of the test compound to bind to the LIG46 protein can be accomplished, for example, by coupling the test compound with a radioisotope or enzymatic label such that binding of the test compound to the LIG46 protein or biologically active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemmission or by scintillation counting. Alternatively, test compounds can be enzymatically labeled with, for example, horseradish peroxidase, alkaline phosphatase, or luciferase, and the enzymatic label detected by determination of conversion of an appropriate substrate to product. In a preferred embodiment, the assay comprises contacting a cell which expresses a membrane-bound form of LIG46 protein, or a biologically active portion thereof, on the cell surface with a known compound which binds LIG46 to form an assay mixture, contacting the assay mixture with a test compound, and determining the ability of the test compound to interact with a LIG46 protein, wherein determining the ability of the test compound to interact with a LIG46 protein comprises determining the ability of the test compound to preferentially bind to LIG46or a biologically active portion thereof as compared to the known compound.

In another embodiment, an assay is a cell-based assay comprising contacting a cell expressing a membrane-bound form of LIG46 protein, or a biologically active portion thereof, on the cell surface with a test compound and determining the ability of the test compound to modulate (e.g., stimulate or inhibit) the activity of the LIG46 protein or biologically active portion thereof. Determining the ability of the test compound to modulate the activity of LIG46 or a biologically active portion thereof can be accomplished, for example, by determining the ability of the LIG46 protein to bind to or interact with a LIG46 target molecule. As used herein, a "target molecule" is a molecule with which a LIG46 protein binds or interacts in nature, for example, a molecule on the surface of a cell which expresses a LIG46 protein, a molecule on the surface of a second cell, a molecule in the extracellular milieu, a molecule associated with the internal surface of a cell membrane or a cytoplasmic molecule. A LIG46 target molecule can be a non-LIG46 molecule or a LIG46 protein or polypeptide of the present invention. In one embodiment, a LIG46 target molecule is a component of a signal transduction pathway which facilitates transduction of an extracellular signal (e.g., a signal generated by binding of a compound to a membrane-bound LIG46 molecule) through the cell membrane and into the cell. The target, for example, can be a second intercellular protein which has catalytic activity or a protein which facilitates the association of downstream signaling molecules with LIG46.

Determining the ability of the membrane bound LIG46 protein to bind to or interact with a LIG46 target molecule can be accomplished by one of the methods described above for determining direct binding. In a preferred embodiment, determining the ability of the LIG46 protein to bind to or interact with a LIG46 target molecule can be accomplished by determining the activity of the target molecule. For example, the activity of the target molecule can be determined by detecting catalytic/enzymatic activity or detecting a cellular response.

The cell-free assays of the present invention are amenable to use of both the soluble form or the membrane-bound form of LIG46. In the case of cell-free assays comprising the membrane-bound form of LIG46, it may be desirable to utilize a solubilizing agent such that the membrane-bound form of LIG46 is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)n, 3-[(3-cholamidopropyl)dimethylaminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In more than one embodiment of the above assay methods of the present invention, it may be desirable to immobilize either LIG46, LIG56, Tgtp, LRG-47, RC10-II, or Stra13 or the corresponding target molecule to facilitate separation of complexed from uncomplexed forms of one or both of the proteins, as well as to accommodate automation of the assay. Binding of a test compound to LIG46, LIG56, Tgtp, LRG-47, RC10-II, or Stra13, or interaction of LIG46, LIG56, Tgtp, LRG-47, RC10-II, or Stra13 with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/ fusion proteins or glutathione-S-transferase/target fusion proteins can be adsorbed onto glutathione sepharose beads (Sigma Chemical; St. Louis, MO) or glutathione derivatized microtitre plates, which are then combined with the test compound or the test compound and either the non-adsorbed target protein or LIG46, LIG56, Tgtp, LRG-47, RC10-II, or Stra13 protein, and the mixture incubated under conditions conducive to complex formation (e.g., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of LIG46, LIG56, Tgtp, LRG-47, RC10-II, or Stra13 binding or activity determined using standard techniques.

Other techniques for immobilizing proteins on matrices can also be used in the screening assays of the invention. For example, either LIG46, LIG56, Tgtp, LRG-47, RC10-II, or Stra13 or the corresponding target molecule can be immobilized utilizing conjugation of biotin and streptavidin. Biotinylated LIG46, LIG56, Tgtp, LRG-47, RC10-II, or Stra13 or the corresponding target molecule can be prepared from biotin-NHS (N-hydroxy-succinimide) using techniques well known in the art (e.g., biotinylation kit, Pierce Chemicals; Rockford; IL), and immobilized in the wells of streptavidin-coated 96 well plates (Pierce Chemical). Alternatively, antibodies reactive with LIG46, LIG56, Tgtp, LRG-47, RC10-II, or Stra13 or the corresponding target molecule but which do not interfere with binding of the LIG46, LIG56, Tgtp, LRG-47, RC10-II, or Stra13 protein to its target molecule can be derivatized to the wells of the plate, and unbound target or LIG46, LIG56; Tgtp, LRG-47, RC10-II, or Stra13 trapped in the wells by antibody conjugation. Methods for detecting such complexes, in addition to those described above for the GST-immobilized complexes, include immunodetection of complexes using antibodies reactive with the LIG46, LIG56, Tgtp, LRG-47, RC10-II, or Stra13 or corresponding target molecule, as well as enzyme-linked assays which rely on detecting an enzymatic activity associated with the LIG46, LIG56, Tgtp, LRG-47, RC10-II, or Stra13 or corresponding target molecule.

In another embodiment, modulators of LIG46, LIG56, Tgtp, LRG-47, RC10-II, or Stra13 expression are identified in a cell-based assay in which a cell is contacted with a candidate compound and the expression of LIG46, LIG56, Tgtp, LRG-47, RC10-II, or Stra13 mRNA or protein in the cell is determined. The level of expression of LIG46, LIG56, Tgtp, LRG-47, RC10-II, or Stra13 mRNA or protein in the presence of the candidate compound is compared to the level of expression of LIG46, LIG56, Tgtp, LRG-47, RC10-II, or Stra13 mRNA or protein in the absence of the candidate compound. The candidate compound can then be identified as a modulator of LIG46, LIG56, Tgtp, LRG-47, RC10-II, or Stra13 expression based on this comparison. For example, when expression of LIG46 mRNA or protein is greater (statistically significantly greater) in the presence of the candidate compound than in its absence, the candidate compound is identified as a stimulator of LIG46 mRNA or protein expression. Alternatively, when expression of LIG46 mRNA or protein is less (statistically significantly less) in the presence of the candidate compound than in its absence, the candidate compound is identified as an inhibitor of LIG46 mRNA or protein expression. The level of LIG46 mRNA or protein expression in the cells can be determined by methods described herein for detecting LIG46 mRNA or protein.

In another embodiment, modulators of LIG46, LIG56, Tgtp, LRG-47, RC10-II, or Stra13 activity are identified in a cell-based assay in which a cell is contacted with a candidate compound and the activity of LIG46, LIG56, Tgtp, LRG-47, RC10-II, or Stra13 mRNA or protein in the cell is determined. The level of activity of LIG46, LIG56, Tgtp, LRG-47, RC10-II, or Stra13 mRNA or protein in the presence of the candidate compound is compared to the level of activity of LIG46, LIG56, Tgtp, LRG-47, RC10-II, or Stra13 mRNA or protein in the absence of the candidate compound. The candidate compound can then be identified as a modulator of LIG46, LIG56, Tgtp, LRG-47, RC10-II, or Stra13 activity based on this comparison.

For example, when activity of LIG46 is greater (statistically significantly greater) in the presence of the candidate compound than in its absence, the candidate compound is identified as a stimulator of LIG46 mRNA or protein expression. Alternatively, when the activity of LIG46 is less (statistically significantly less) in the presence of the candidate compound than in its absence, the candidate compound is identified as an inhibitor of LIG46 activity.

In yet another aspect of the invention, LIG46, LIG56, Tgtp, LRG-47, RC10-II, or Stra13 protein can be used a "bait protein" in a two-hybrid assay or three hybrid assay (see, e.g., U.S. Patent No. 5,283,317; Zervos et al. (1993) *Cell* 72:223-232; Madura et al. (1993) *J. Biol. Chem*. 268:12046-12054; Bartel et al. (1993) *Bio*/*Techniques* 14:920-924; Iwabuchi et al. (1993) *Oncogene* 8:1693-1696; and PCT Publication No. WO 94/10300), to identify other proteins, which bind to or interact with LIG46, LIG56, Tgtp, LRG-47, RC10-II, or Stra13 and modulate activity.

The two-hybrid system is based on the modular nature of most transcription factors, which consist of separable DNA-binding and activation domains. Briefly, the assay utilizes two different DNA constructs. In one construct, the gene that codes for the protein of interest, e.g., Stra13, is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo*, forming a complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with Stra13.

This invention further pertains to novel agents identified by the above-described screening assays and uses thereof for treatments as described herein.

### B. Detection Assays

Portions or fragments of the cDNA LIG46 and LIG56 sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. Accordingly, LIG46 or LIG56 nucleic acid molecules described herein or fragments thereof, can be used to map the location of LIG46 or LIG56 genes on a chromosome. The mapping of the LIG46 or LIG56 sequences to chromosomes is an important first step in correlating these sequences with genes associated with disease.

Briefly, LIG46 or LIG56 genes can be mapped to chromosomes by preparing PCR primers (preferably 15-25 bp in length) from the LIG46 or LIG56 sequences. Computer analysis of LIG46 or LIG56 sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the LIG46 or LIG56 sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but human cells can, the one human chromosome that contains the gene encoding the needed enzyme, will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. (D'Eustachio et al. (1983) *Science* 220:919-924). Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the LIG46 or LIG56 sequences to design oligonucleotide primers, sublocalization can be achieved with panels of fragments from specific chromosomes. Other mapping strategies which can similarly be used to map a LIG46 or LIG56 sequence to its chromosome include *in situ* hybridization (described in Fan et al. (1990) *Proc. Natl*. *Acad. Sci. USA* 87:6223-27), pre-screening with labeled flow-sorted chromosomes, and pre-selection by hybridization to chromosome specific cDNA libraries.

Fluorescence in situ hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., (Human Chromosomes: A Manual of Basic Techniques (Pergamon Press, New York, 1988)).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between genes and disease, mapped to the same chromosomal region, can then be identified through linkage analysis (co-inheritance of physically adjacent genes), described in, e.g., Egeland et al. (1987) *Nature,* 325:783-787.

Moreover, differences in the DNA sequences between individuals affected and unaffected with a disease associated with the LIG46 or LIG56 gene can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 2. Tissue Typing

The LIG46 or LIG56 sequences described herein can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the LIG46 or LIG56 sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The LIG46 or LIG56 sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SBQ ID NO:1 can comfortably provide positive individual identification with a panel of perhaps 10 to 1,000 primers which each yield a noncoding amplified sequence of 100 bases. If predicted coding sequences, such as those in SEQ ID NO:3 are used, a more appropriate number of primers for positive individual identification would be 500-2,000.

If a panel of reagents from LIG46 or LIG56 sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial LIG46 or LIG56 Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 are particularly appropriate for this use as greater numbers of polymorphisms occur in the noncoding regions, making it easier to differentiate individuals using this technique. Examples of polynucleotide reagents include the LIG46 or LIG56 sequences or portions thereof, e.g., fragments derived from the noncoding regions of SEQ ID NO:1 having a length of at least 20 or 30 bases.

The LIG46 or LIG56 sequences described herein can further be used to provide polynucleotide reagents, e.g., labeled or labelable probes which can be used in, for example, an in situ hybridization technique, to identify a specific tissue, e.g., brain tissue. This can be very useful in cases where a forensic pathologist is presented with a tissue of unknown origin. Panels of such LIG46 or LIG56 probes can be used to identify tissue by species and/or by organ type.

In a similar fashion, these reagents, e.g., LIG46 or LIG56 primers or probes can be used to screen tissue culture for contamination (i.e., screen for the presence of a mixture of different types of cells in a culture).

This invention is further illustrated by the following examples which should not be construed as limiting. The contents of all references, patents and published patent applications cited throughout this application are hereby incorporated by reference.

### EXAMPLES

### Example 1: Identification of Leptin Induced Genes

The leptin induced genes of the invention were identified by comparing the expression pattern of leptin-treated murine neuronal cells expressing OB-RL with the expression pattern of otherwise identical treated cells not expressing OB-RL.

### Preparation of Ob Receptor Expressing Neuronal Cells

An adenovirus vector expressing long form murine OB receptor (ObR-L) (Bauman et al. (1996) Proc. Nat'l. Acad. Sci. USA 93:8374-78) was prepared using standard techniques. A high titer viral stock carrying this vector was prepared and used to infect GT1-7 murine neuronal cells. The infected cells were incubated in standard growth medium for 48 hours and then tested for ObR-L expression by measuring binding of labelled leptin ((1995) *Cell* 83:1263-71). This assay demonstrated that the infected cells express ObR-L.

### Preparation of a Subtracted Library

The ObR-L expressing murine neuronal cells described above were starved were four hours by growth in serum-free medium. A sample of the starved cells was stimulated by incubation in the presence of 200 ng/ml murine leptin for three hours. A second sample of starved cells was mock-stimulated. Total RNA was isolated from both cell samples and used to create cDNA using the SMART PCR™ cDNA synthesis kit (Clontech, Inc.; Palo Alto, CA). The two cDNA pools (generated from total RNA harvested from untreated and leptin-treated cells) created as described above were used to create a subtracted library using the Clontech PCR-Select cDNA Subtraction Kit (Clontech, Inc.).

### Screening of the Subtracted Library and Analysis of Positive Clones

The clones in the subtracted library were cloned into T/A vector plasmid T-Adv (Advantage PCR Cloning Kit; Clontech, Inc.). Plasmid specific flanking primers were used to PCR amplify cDNA inserts from the library. The PCR products were then used to create microarrays on nylon filters. The microarrays were probed with labeled cDNA from the subtracted library. Positive clones identified on the the microarray were sequenced, and differential expression of the positive clones was confirmed by virtual Northern analysis on the original treated and untreated samples (pre-subtracted cDNA generated from from the original cell samples). Additionally, a subset of these clones were analyzed for brain and peripheral tissue distribution by Nothern blotting.

Two positive clones which appeared to represent novel genes were used to probe a murine whole brain library in order to identify full-length clones. This resulted in the identification of LIG46 and LIG56.

Six of the leptin induced genes identified as described above, LIG46, LIG56, Tgtp, LRG-47, RC10-II, and Stra13 are described in greater detail below.

### Example 2: Characterization of Murine and Human LIG46 cDNA and Protein

The LIG46 cDNA isolated as described above (SEQ ID NO:1) has a 1191 nucleotide open reading frame (nucleotides - of SEQ ID NO:1; SEQ ID NO:3) which encodes a 397 amino acid protein (SEQ ID NO:2). This protein includes a predicted signal sequence of about 32 amino acids (from amino acid 1 to about amino acid 32 of SEQ ID NO:2) and a predicted mature protein of about 365 amino acids (from about amino acid 33 to amino acid 397 of SEQ ID NO:2; SEQ ID NO:4). The extracellular domain of LIG46 extends from about amino acid 33 to about amino acid 302. LIG46 protein possesses one predicted transmembrane domain which extends from about amino acid 303 (extracellular end) to about 320 (intracellular end) of SEQ ID NO:2. The cytoplasmic domain of LIG46 extends from about amino acid 321 to about amino acid 397.

LIG46 protein has some sequence similarity to a number of galactosyltransferases. Galactosyltransferases have been implicated in developmental processes. In addition, galactosyltransferases may play a role in cell to cell signaling by modifying the carbohydrate repertoire on cell surface receptors to activate, inhibit or otherwise modify (e.g., by alter receptor affinity for a ligand) receptor activity. Thus, LIG46 may play a role body weight regulation by influencing cell to cell signaling mediated by molecules involved in body weight regulation, e.g., leptin.

The LIG46 polypeptide sequence of SEQ ID NO:2 includes potential N-glycosylation sites at amino acids 30-33, 79-82, 89-92, 127-173, and 219-222; potential protein kinase C phosphorylation sites at amino acids 54-56, 202-204, 221-223, 323-325, and 377-379; potential casein kinase II phosphorylation sites at amino acids 31-34, 94-97, 185-188, 221-224, 234-237, and 368-371; a potential tyrosine kinase phosphorylation site at amino acids 115-122; and a potential amidation site at amino acids 3-6.

Portions of LIG46 are similar to certain galactosyltransferases. Figure 2 depicts a series of alignments of portions of the amino acid sequence of LIG46 with portions of a number of galactosyltransferases, including: *Mus musculus* UDP-Gal: betaGlcNAc beta 1,3-galactosyltransferase-I (Accession Number AF029790; SEQ ID NO:_); *Mus musculus* IPP-Gal: betaGlcNAc beta 1,3-galactosyltransferase-III (Accession Number AF029792); *Drosophila melanogaster* neurogenic secreted signalling protein (Accession Number U41449; SEQ ID NO:_); and *Homo sapiens* UDP-galactose: 2-acetamido-2-deoxy-D-glucose3beta-galactosyltransferase (Accession Number Y15014; SEQ ID NO:_). A majority sequence is depicted above the solid line. Conserved residues are shaded. These residues are more likely conserved in functional variants of LIG46.

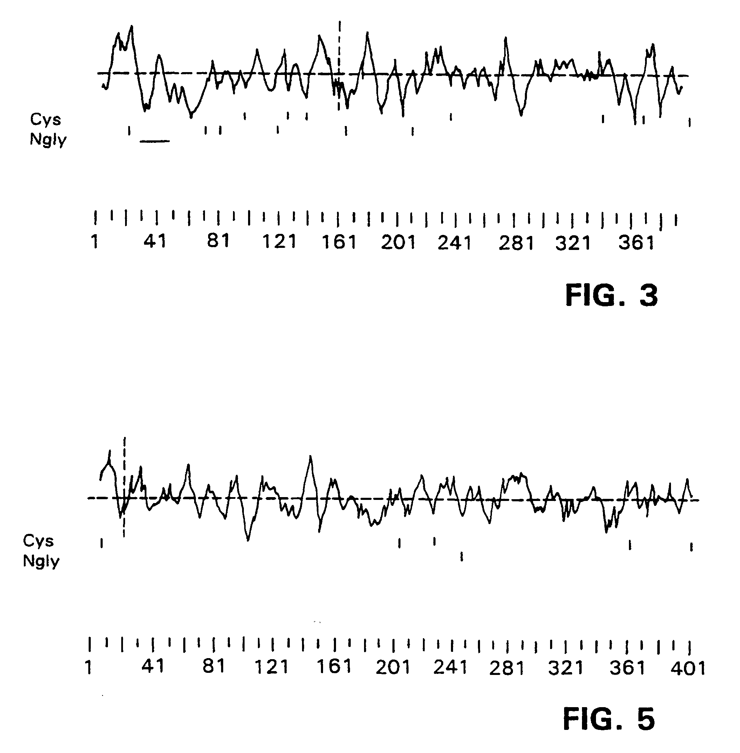

Figure 3 is a hydropathy plot of LIG46. Relative hydrophobicity is shown above the dotted line, and relative hydrophilicity is shown below the dotted line.

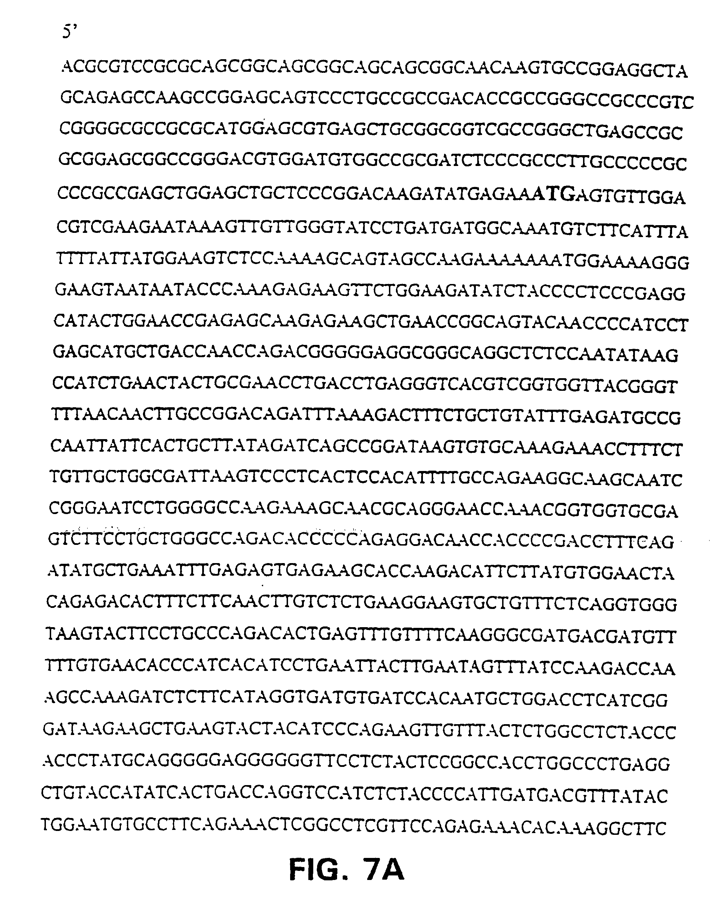

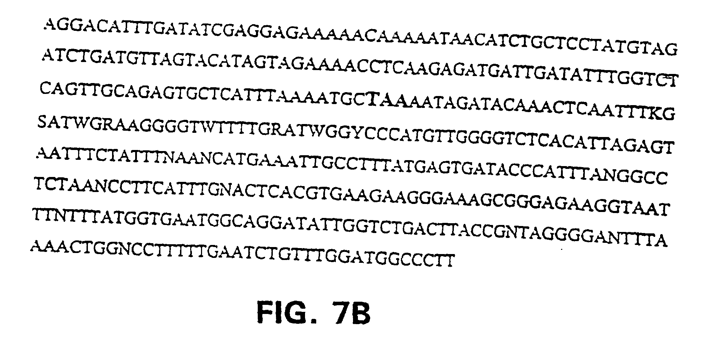

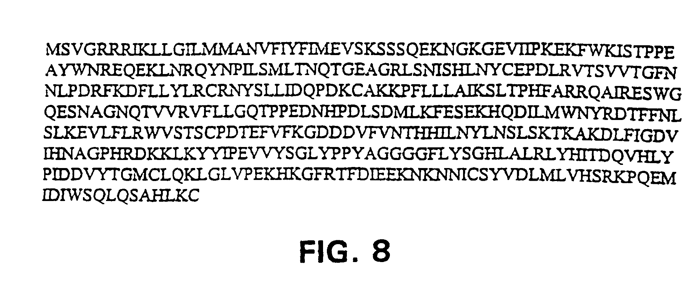

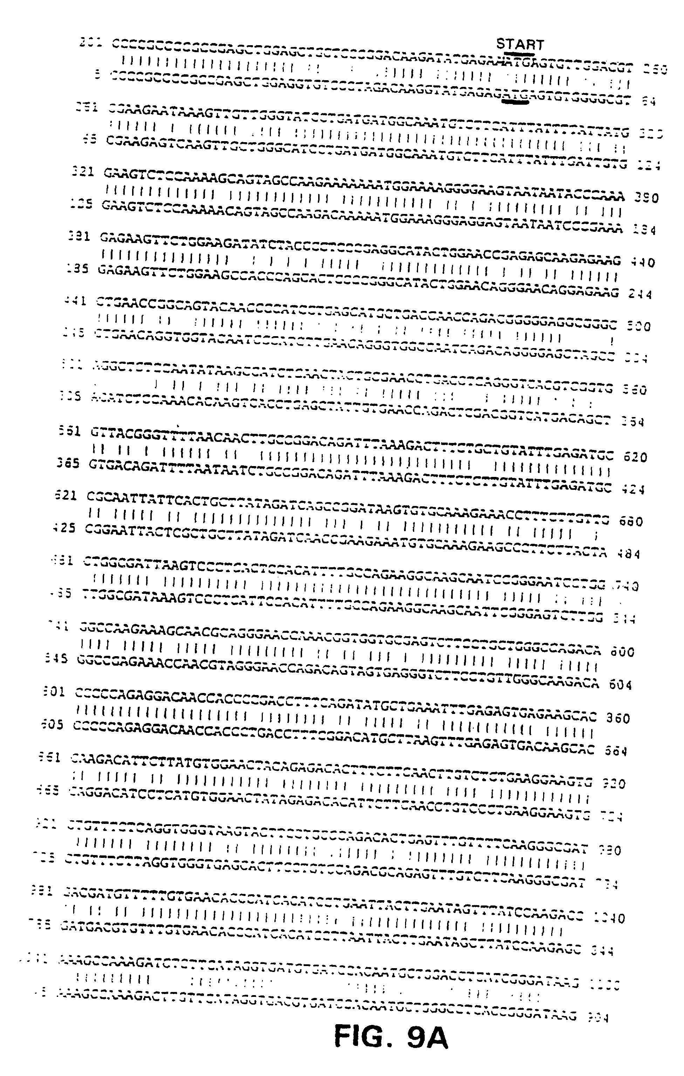

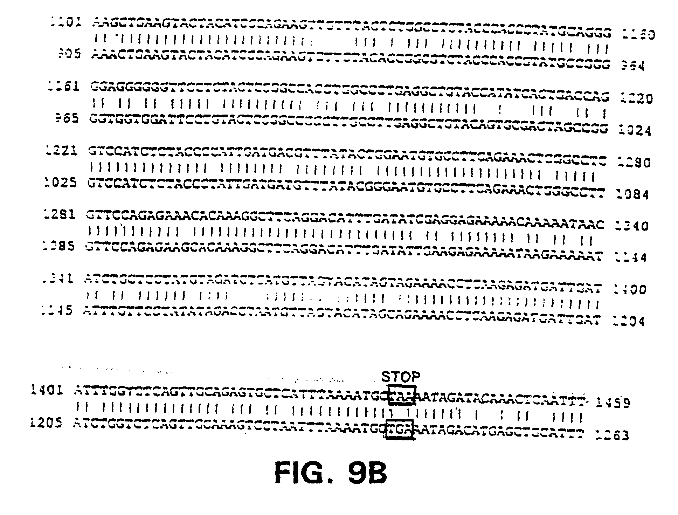

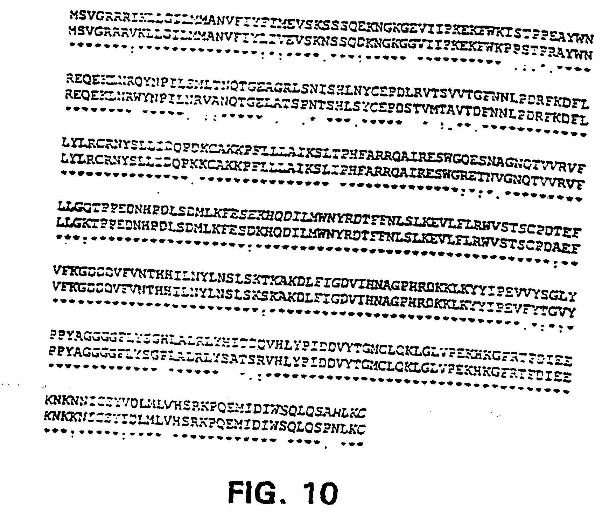

Figure 7 depicts the cDNA sequence of a full-length human LIG46 clone. Figure 8 depicts the predicted amino acid sequence of human LIG46. The human LIG46 cDNA depicted in Figure 7 (SEQ ID NO:_) has a 1191 nucleotide open reading frame which encodes a 397 amino acid protein (SEQ ID NO:_). This protein includes a predicted signal sequence of about 32 amino acids (from amino acid 1 to about amino acid 32 of SEQ ID NO:_) and a predicted mature protein of about 365 amino acids (from about amino acid 33 to amino acid 397 of SEQ ID NO:_; SEQ ID NO:_). Figure 9 depicts an alignment of the cDNA sequences of human LIG46 (upper sequence) and murine LIG46 (lower sequence). Figure 10 depicts an alignment of the predicted amino acid sequences of human LIG46 (upper sequence) and murine LIG46 (lower sequence).

### Genomic Mapping of LIG46

LIG46 was mapped to human chromosome 2, 17.9 cR₃₀₀₀ telomeric to the Whitehead Institute framework marker D2S290 (LOD score = 15.5) and 23.5 cR₃₀₀₀ centromeric of the Whitehead framework marker WI-6130 (LOD score = 13.6). This region corresponds to cytogenic location 2p12-13, within or just outside the minimal interval for Alström syndrome (Macari et al. (1998) Human Genet. 103:658-61). Alström syndrome is an autosomal recessive disorder characterized by childhood obesity, retinal pigment degeneration, neurogenic deafness, non-insulin dependent diabetes mellitus, chronic nephropathy, and hyperlipidemia. Other symptoms include: cardiomyopathy, acanthosis nigricans, hypothyroidism, growth hormone deficiency, progressive baldness, hyperuricemia, gynecomastia, and reduced fertility (Russell-Eggitt et al. (1998) Ophthalmology 105:1274-80).

Briefly, the LIG46 gene was mapped using the Genebridge 4 Radiation Hybrid Panel. A pair of primers within the 3' untranslated region of LIG46 (forward-CCATGTTGGGGTCTCACATTAGAG, SEQ ID NO:_; and reverse-GGTAAGTCAGACCAATATCCTGCC, SEQ ID NO:_) were used to amplify DNA from the Genebridge 4 panel. The PCR products were run on a 2% agarose gel, stained with SYBR Gold and scanned. Linkage analysis was performed using the Map Manager QT623 software package.

LIG46 nucleic acid molecules can be used in the diagnosis of Alström syndrome. Moreover, it is possible that mutations in LIG46 cause Alström syndrome. If so, LIG46 polypeptide and nucleic acid molecules as well as antibodies directed against LIG46 and modulators of LIG46 expression or activity can be used to treat Alström syndrome and/or various symptoms of Alström syndrome.

### Example 3: Distribution of LIG46 mRNA

The expression of LIG46 in murine tissue was analyzed using Northern blot hybridization. Analysis of total tissue blots revealed that LIG46 is expressed at the highest level in heart and liver followed by lung and kidney, then brain, then spleen testis, and skeletal muscle. Analysis of LIG46 expression in murine brain revealed that LIG46 is expressed at least in the hypothalamus (including: the arcuate nucleus, the ventral/medial hypothalamus, and the superchiasmatic nucleus, the hippocampus, the cortex, and the striatum.

### Example 4: Secretion of LIG46

LIG46 protein is homologous to *D. melanogaster* brainiac (Goode et al., (1996) Development 122:3863-79), a secreted protein (Fig. 2). As discussed above, LIG46 has a predicted signal sequence at its amino terminus. Therefore, to determine whether LIG46 protein is secreted, full-length LIG46 (amino acids 1-397) was fused to alkaline phosphatase using methods similar to those previously described (fusion at carboxy-terminus of LIG46; Cheng and Flanagan (1994) *Cell* 79:157-168; Tartaglia et al. (1995) *Cell* 83:1263-71). This construct was transiently transfected into human 293T cells.

At 48 hrs post transfection, the growth media was assayed for alkaline phosphatase activity (White et al., (1997) *Proc. Natl. Acad. Sci USA* 94:10657-10662) using the Great EscAPe alkaline phosphatase detection kit (Clontech, Inc.). A large increase in alkaline phosphatase activity was observed in the growth medium from transfected cells compared to mock tranfected cells, indicating that LIG46 protein is secreted and that the signal sequence of LIG46 is functional.

### Example 5: LIG46 Expression is Induced by Leptin in vivo

C57BL6 *ob*/*ob* mice were injected (via the interperitoneum (IP)) with 100 µl of either phosphate buffered saline (PBS) (sham injected) or PBS supplemented with 100 µg leptin (leptin injected) (R&D Systems Inc., Minneapolis, MN). Following a 1 or 3 hr treatment, the animals were euthanized by CO₂ asphyxiation, the brains were harvested, sliced, and the hypothalamus analyzed by in situ hybridization using a 386 base pair radiolabeled antisense probe to the coding region of LIG46. Comparative analysis of hypothalamic slices from sham injected and leptin injected animals indicates that LIG46 transcript is induced in the arcuate nucleus and the ventromedial hypothalamus by leptin.

### Example 6: The Effect of LIG46 Antisence

### Oligodeoxynucleotides on Feeding of Obese (ob/ob) Male Mice

For this study, a phosphothioate-protected antisense oligodeoxynucleotide and its respective control sequence (sense) were synthesized. The antisense oligodeoxynucleotide targets the LIG46 start codon mRNA at position 39. Male obese *ob*/*ob* C57BL/6J (45 g) mice were individually housed in macrolon cages (22±2° C; 12:12 h light/dark cycle with lights off at 6 pm). Tap water and mouse chow diet were given *ad libitum.* Mice were stereotaxically implanted with a chronic guide cannula aimed to the third ventricle (intracerebroventricular) one week prior to this experiment.

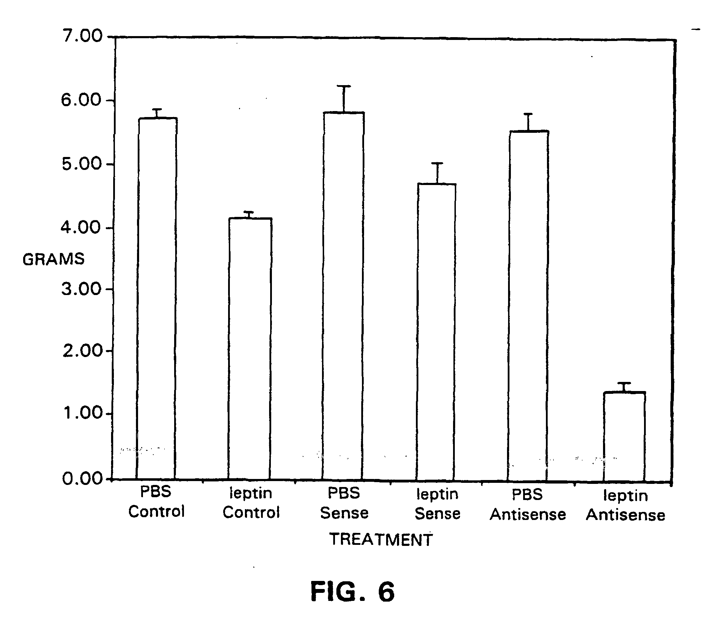

The effect of LIG46 antisense treatment on leptin-induced decrease in food intake was studied on day 5. Therefore, mice were treated intracerebroventricularly on days 1 and 3 with 18 µg LIG46 antisense oligodeoxyribonucleotide, 18 µg sense (control) oligodeoxyribonucleotide or 2 µl RNAse-free water. Intracerebroventricular injections were performed at 3 pm Control and oligodeoxyribonucleotide pre-treatments were followed by an intraperitoneal injection of 1 mg/kg leptin or phosphate-buffered saline (vehicle), performed at 5 pm on day 5 and food intake was measured each four hour after leptin or vehicle application. The results of this study are shown in Fig. 6. The leptin-induced decrease in food intake was far greater in the presence of LIG46 antisense oligonucleotide than LIG46 sense nucleotide or PBS control.

### Example 7: The Effect of LIG46 Antisence

### Oligodeoxynucleotides on Feeding of Lean Male Mice

For this study, a phosphothioate-protected antisense oligodeoxynucleotide and its respective control sequence (sense) were synthesized. The antisense oligodeoxynucleotide targets the LIG46 start codon mRNA at position 39. Male lean C57BL/6J (24 g) mice were individually housed in macrolon cages (22±2° C; 12:12 h light/dark cycle with lights off at 6 pm). Tap water and mouse chow diet were given *ad libitum.* Mice were stereotaxically implanted with a chronic guide cannula aimed to the third ventricle (intracerebroventricular) one week prior to this experiment.

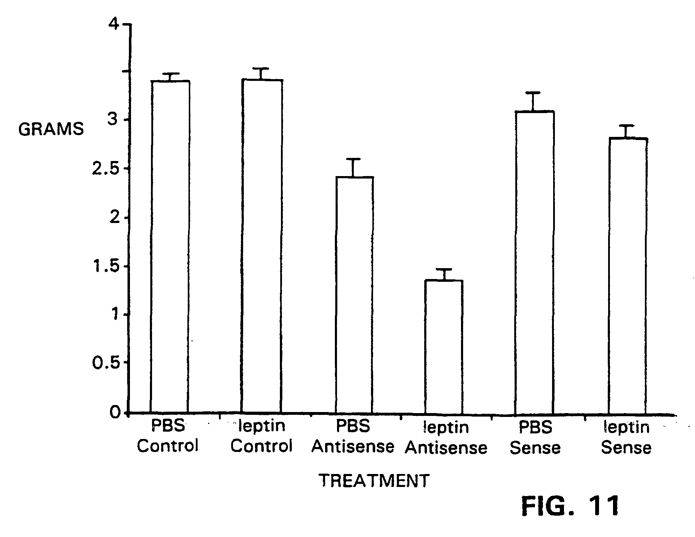

The effect of LIG46 antisense treatment on leptin-induced decrease in food intake was studied on day 5. Therefore, mice were treated intracerebroventricularly on days 1 and 3 with 18 µg LIG46 antisense oligodeoxyribonucleotide, 18 µg sense (control) oligodeoxyribonucleotide or 2 µl RNAse-free water. Intracerebroventricular injections were performed at 3 pm Control and oligodeoxyribonucleotide pre-treatments were followed by an intraperitoneal injection of 1 mg/kg leptin or phosphate-buffered saline (vehicle), performed at 5 pm on day 5 and food intake was measured each four hour after leptin or vehicle application. The results of this study are shown in Fig. 11. The LIG46 antisense-induced decrease in food intake was far greater in the presence of leptin than PBS control. Thus, food intake can be decreased in lean mice by decreasing LIG46 protein expression. Moreover, this decrease in food intake is increased when leptin is administered, demonstrating that leptin can sensitize lean mice to the effects of a LIG46 antagonist.

### Example 8: Characterization of LIG56 cDNA and Protein

The full-length LIG56 cDNA isolated as described above (SEQ ID NO:5) is shown in Figure 4. This cDNA has a 1200 nucleotide open reading frame (nucleotides 1 - 1200 of SEQ ID NO:5; SEQ ID NO:7) which encodes a 400 amino acid protein (SEQ ID NO:6).

The LIG56 polypeptide sequence of SEQ ID NO:6 includes potential N-glycosylation sites at amino acids 252-255; potential protein kinase C phosphorylation sites at amino acids 67-69, 75-77, 203-205, 218-220, 295-297, and 299-301; potential casein kinase II phosphorylation sites at amino acids 126-129, 170-173, 203-206, 256-259, 291-294, 341-344, and 345-349; a potential tyrosine kinase phosphorylation site at amino acids 233-241; potential N-myristlation sites at amino acids 66-71, 85-90, 116-121, and 308-313; and a potential amidation site at amino acids 63-70.

LIG56 may be a GTP-binding protein. Portions of LIG56 protein are to similar to one or more murine GTP-binding proteins (Genbank Accession Numbers: L38444; U15636; M63630; U19119; and U53219).

LIG56 protein possesses a GTP-binding protein-like domain (amino acids 12 to 283 of SEQ ID NO:6) and an LRG-47-like domain (amino acids 24-177 of SEQ ID NO:6).

Figure 5 is a hydropathy plot of LIG56. Relative hydrophobicity is shown above the dotted line, and relative hydrophilicity is shown below the dotted line.

### Example 9: Distribution of LIG56 mRNA

The expression of LIG56 in murine tissue was analyzed using Northern blot hybridization. Analysis of total tissue blots revealed that LIG56 is expressed at the highest level in heart followed by liver, then kidney, then lung, then skeletal muscle, then spleen. Analysis of LIG56 expression in murine brain revealed that LIG56 is expressed at least in the hippocampus (including, at least, the dentate gyrus).

### Example 10: Characterization and mRNA Distribution of Clone 50 (Tgtp)

Sequence analysis of clone 50 identified in the microarray described above revealed that the clone encodes murine Tgtp (Genbank Accession Number L38444), a T cell-specific guanine nucleotide triphosphate-binding protein (Carlow et al. (1994) *J. Immunol*. 154:1724-34).

The expression of clone 50 in murine tissue was analyzed using Northern blot hybridization. Analysis of total tissue blots revealed that clone 50 is expressed at the highest level in heart followed by kidney, then lung and skeletal muscle, then liver. Analysis of clone 50 expression in murine brain revealed that clone 50 is expressed at least in the choroid plexus.

### Example 11: Characterization and mRNA Distribution of Clone 44 (LRG-47)

Sequence analysis of clone 44 identified in the microarray described above revealed that the clone encodes murine LRG-47 (Genbank Accession Number U19119), a protein that is induced by LPS, IFN-γ, and IFN-α/β and has some homology to GTP-binding proteins (Sorace et al. (1995) *J. Leukocyte Biol.* 58:477-84).

The expression of clone LRG-47 mRNA in murine tissue was analyzed using Northern blot hybridization. Analysis of total tissue blots revealed that LRG-47 is expressed at the highest level in heart followed by kidney, then liver, then skeletal muscle, then lung, then spleen. Analysis of LRG-47 mRNA expression in murine brain revealed that LRG-47 is expressed at least in the cortex, the hippocampus, the choroid plexus, the medial habenuclear nucleus, and the hypothalamus (including at least: the arcuate nucleus and the paraventicular nucleus).

### Example 12: LRG-47 is Induced by Leptin in vivo

C57BL6 ob/ob mice were injected (IP) with 100 ul of either PBS or PBS supplemented with 100 µg leptin (R&D Systems Inc.). After 60 min, the animals were euthanized by CO₂ asphyxiation, the brains harvested, sliced, and the hypothalamus analyzed by *in-situ* hybridization using a 762 base pair radiolabeled antisense probe against sequences in the 5' untranslated region of the LRG-47 transcript. Comparative analysis of hypothalamic slices from sham injecting and leptin injected animals indicates that the LRG-47 transcript is induced in the arcuate nucleus by leptin, demonstrating that the LRG-47 transcript is a leptin-induced gene *in vivo*.

### Example 13: Characterization and mRNA Distribution of Clone 10 (RC10-11)

Sequence analysis of clone 10 identified in the microarray described above revealed that the clone encodes murine RC10-II (Genbank Accession Number D21800), a subunit of the 20S proteasome of rat embryonic brain (Nishimura et al. (1993) *FEBS Lett.* 336:462-66). It has been suggested that RC10-II is a proteasomal subunit that is required for expression of tryptic activity (Nishimura et al., *supra*).

The expression of clone 10 mRNA in murine tissue was analyzed using Northern blot hybridization. Analysis of total tissue blots revealed that clone 10 is expressed at the highest level in heart, liver, skeletal muscle, and kidney, followed by brain, lung, and testis. Analysis of clone 10 mRNA expression in murine brain revealed that clone 10 is expressed at least in the cortex, hippocampus, habenular nucleus, thalamus, and hypothalamus (including the arcuate nucleus and ventromedial hypothalamus).

### Example 14: Characterization and mRNA Distribution of Clone 67 (Stra13)

Sequence analysis of clone 67 identified in the microarray described above revealed that the clone encodes Stra13 (Genbank Accession Number AP010305), retinoic acid-inducible helix-loop-helix protein (Boudjelal et al. (1997) *Genes Dev.* 11:2052-65). Stra13 may act as a repressor of activated transcription and is thought to play a role in neuronal differentiation (Boudjelal et al., *supra*).

The expression of clone 67 mRNA in murine tissue was analyzed using Northern blot hybridization. Analysis of total tissue blots revealed that clone 10 is expressed at the highest level in liver followed by heart, then skeletal muscle, then brain, then kidney. Analysis of clone 67 mRNA expression in murine brain revealed that clone 67 is expressed at least in the cortex, hippocampus (CA1, CA2 and dentate gyrus), lateral thalamus, hypothalamus (arcuate nucleus).

### Example 15: Stra13 is Induced by Leptin in vivo

C57BL6 ob/ob mice were injected IP with 100 µl of either PBS or PBS supplemented with 100 µg leptin (R&D Systems, Inc.). After 60 min, the animals were euthanized by CO₂ asphyxiation, the brains harvested, sliced, and the hypothalamus analyzed by in situ hybridization using 328 base pair radiolabeled antisense probe against sequences in the 5' untranslated region of the LRG-47 transcript. Comparative analysis of hypothalamic slices from sham injected and leptin injected animals indicates that the Stra13 transcript is induced in the arcuate nucleus by leptin, supporting the claim that the Stra13 transcript is a leptin-induced gene *in vivo*.

### Example 16: Assays for LIG46 Activity

Because LIG46 appears to be a galactosyltransferase, it may be possible to identify modulators of LIG46 activity using assays that are used to measure the activity of members of the glycosyltransferase and galactosyltransferase family (see, e.g., Hennet et al. (1998) J. Biol Chem 1998 273:58-65; Current Protocols in Molecular Biology (1993) Ausbel et al., eds., Wiley Interscience (New York); and Wandall et al. (1997) J. Biol Chem 272(38):23503-14). Of course, the donor and acceptor substrates utilized by LIG46 may differ from those described in the literature previously. Those skilled in the art can adapt assays used for other members of the glycosyltransferase and galactosyltransferase family for use with LIG46.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method for determining whether a compound is a candidate compound for use to modulate body weight, the method comprising:
a) providing a cell containing one or more genes selected from the group consisting of the LIG46 gene, which encodes a polypeptide comprising the amino acid sequence of SEQ ID NO: 2 or the sequence shown in figure 8, the LIG56 gene, which encodes a polypeptide comprising the amino acid sequence of SEQ ID NO: 6, the Tgtp gene, the LRG-47 gene, the RC10-II gene, and the Stra13 gene;
b) measuring the expression level of the selected one or more genes in the presence and absence of a test compound;
c) comparing the expression level of the selected one or more genes in the presence of the test compound to the expression level of the selected one or more genes in the absence of the test compound; and
d) identifying the test compound as a candidate compound for use in modulating body weight if the expression level of the selected one or more genes in the presence of the test compound differs from the expression level of the selected one or more genes in the absence of the test compound.

2. The method of claim 1 wherein the expression level of the selected one or more genes is measured by measuring mRNA or protein.

3. The method of claim 1 or 2 wherein the cell is a neuronal cell and/or a cultured cell.

4. The method of any preceding claim wherein the cell expresses Ob receptor.

5. The method of any preceding claim wherein expression is measured in the presence of leptin.

6. The method of any preceding claim wherein the candidate compound is useful for increasing or decreasing food intake.

7. The method of any preceding claim, further comprising:
e) administering the candidate compound identified in step d) to a non-human test mammal;
f) monitoring the effect of the compound on the feeding behaviour of the non-human test mammal; and
g) identifying the compound as a compound for modulating the body weight of a mammal if the compound alters the feeding behaviour of the non-human test mammal.

8. The method of claim 7 wherein the non-human test mammal does not express leptin and/or the non-human test mammal is exposed to exogenous leptin.

9. The method of claim 7 or 8 wherein the non-human test mammal is a mouse.

10. The method of claim 9 wherein the mouse is an ob/ob mouse and/or wherein the mouse is a transgenic mouse expressing a UG-46 gene encoding the amino acid sequence shown in figure 8.

11. The method of any one of claims 7 to 10 wherein the non-human test mammal is fed or starved prior to administering the candidate compound.

12. A method for determining whether a compound is a candidate compound for use to modulate body weight, the method comprising:
a) providing a sample comprising one or more proteins selected from the group consisting of a LIG46 polypeptide comprising the amino acid sequence of SEQ ID NO: 2 or the sequence shown in figure 8, Tgtp, LRG-47, RC10-II, and Stra13;
b) measuring the activity of the selected one or more proteins in the presence and absence of a test compound;
c) comparing the activity of the selected one or more proteins in the presence of the test compounds to the activity of the selected one or more proteins in the absence of the test compound; and
d) identifying the test compound as a candidate compound useful for modulating body weight when the activity of the selected one or more proteins in the presence of the test compound differs from the activity of the selected one or more protein in the absence of the test compound.

13. The method of claim 12, wherein the selected protein is LIG46 and the activity measured is a galactosyltransferase activity.

14. The method of claim 12 or 13, wherein the sample comprises a cell expressing one of the proteins selected from the group consisting of a LIG46 polypeptide comprising the amino acid sequence of SEQ ID NO: 2 or the sequence shown in figure 8, Tgtp, LRG-47, RC10-II, and Stra13.

15. The method of claim 14, wherein the cell is a neuronal cell and/or a cultured cell.

16. The method of claim 14 or 15 wherein the cell expresses Ob receptor.

17. The method of any one of claims 12 to 16 wherein activity is measured in the presence of leptin.

18. The method of any one of claims 12 to 17, further comprising:
e) administering the candidate compound identified in step d) to a non-human test mammal;
f) monitoring the effect of the compound on the feeding behaviour of the non-human test mammal; and
g) identifying the compound as a compound for modulating the body weight of a mammal if the compound alters the feeding behaviour of the non-human test mammal.

19. The method of claim 18 wherein the non-human test mammal does not express leptin and/or the non-human test mammal is exposed to exogenous leptin.

20. The method of claim 18 or 19 wherein the non-human test mammal is a mouse.

21. The method of claim 20 wherein the mouse is an ob/ob mouse and/or wherein the mouse is a transgenic mouse expressing a LIG-46 gene encoding the amino acid sequence shown in figure 8.

22. The method of any one of claims 18 to 21 wherein the non-human test mammal is fed or starved prior to administering the candidate compound.

23. A method for determining whether a compound is a candidate compound for use to modulate body weight, the method comprising:
a) measuring expression level of one or more genes selected from the group consisting of the LIG46 gene, which encodes a polypeptide comprising the amino acid sequence of SEQ ID NO: 2 or the sequence shown in figure 8, the LIG56 gene, which encodes a polypeptide comprising the amino acid sequence of SEQ ID NO: 6, the Tgtp gene, the LRG-47 gene, the RC10-II gene, and the Stra13 gene in sample of cells is isolated from a mammal treated with the compound and in a sample of cells isolated from an untreated mammal; and
b) identifying the test compound as a candidate compound useful for modulating body weight when the expression level of the selected one or more genes in the sample of cells isolated from the treated mammal differs from the expression of the selected one or more genes in the sample of cells isolated from the untreated mammal.

24. The method of claim 23 wherein the cells in the sample are neuronal cells.

25. The method of claim 23 or 24 wherein the mammal is a mouse.

26. A method for determining whether a compound is a candidate compound for use to modulate body weight, the method comprising:
a) measuring activity level of one or more proteins selected from the group consisting of a LIG46 polypeptide comprising the amino acid sequence of SEQ ID NO: 2 or the sequence shown in figure 8, a LIG56 polypeptide comprising the amino acid sequence of SEQ ID NO: 6, Tgtp, LRG-47, RC10-II, and Stra13 in a sample of cells isolated from a mammal treated with a test compound and in a sample of cells isolated from an untreated mammal; and
b) identifying the test compound as a candidate compound useful for modulating body weight when the activity level of the selected one or more proteins in the sample of cells isolated from the treated mammal differs from the activity level of the one or more selected proteins in the sample of cells isolated from the untreated mammal.

27. The method of claim 26 wherein the cells in the sample are neuronal cells.

28. The method of claim 26 or 27 wherein said mammal is a mouse.

29. A method for determining whether a compound is a candidate compound for use to modulate body weight, the method comprising:
a) combining a test compound with a sample comprising one or more proteins selected from the group consisting of a LIG46 polypeptide comprising the amino acid sequence of SEQ ID NO: 2 or the sequence shown in figure 8, a LIG56 polypeptide comprising the amino acid sequence of SEQ ID NO: 6, Tgtp, LRG-47, RC10-II, and Stra13, under conditions suitable for binding;
b) measuring binding of the selected one or more proteins in the sample to the test compound; and
c) identifying the test compound as a candidate compound useful for modulating body weight when the test compound binds to the selected one or more proteins.

30. The method of claim 29 wherein the selected one or more proteins is bound to a solid support.

31. The method of claim 29 wherein the sample comprises a cell expressing the selected one or more proteins.

32. The method of claim 31 wherein the cell is a cultured cell and/or a neuronal cell.

33. The method of any one of claims 29 to 32 wherein the test compound is detectably labelled.

34. The method of any one of claims 29 to 33 wherein binding is measured in the presence of leptin.

## Patentansprüche

1. Verfahren zur Bestimmung, ob eine Verbindung eine zur Verwendung bei der Änderung des Körpergewichts geeignete Verbindung darstellt, welches umfasst:
a) Bereitstellen einer Zelle, die ein oder mehrere Gene enthält, ausgewählt aus der Gruppe bestehend aus dem LIG46 Gen, das ein die Aminosäuresequenz von SEQ ID NO: 2 oder die in Fig. 8 gezeigte Sequenz umfassendes Polypeptid kodiert, dem LIG56 Gen, das ein die Aminosäuresequenz von SEQ ID NO: 6 umfassendes Polypeptid kodiert, dem Tgtp Gen, dem LRG-47 Gen, dem RC10-II Gen und dem Stra13 Gen;
b) Bestimmen des Expressionsniveaus der ausgewählten einen oder mehreren Gene in der Anwesenheit und Abwesenheit einer Testverbindung;
c) Vergleichen des Expressionsniveaus der ausgewählten einen oder mehreren Gene in der Anwesenheit der Testverbindung mit dem Expressionsniveau der ausgewählten einen oder mehreren Gene in der Abwesenheit der Testverbindung; und
d) Ermitteln der Testverbindung als eine geeignete Verbindung zur Verwendung beim Verändern des Körpergewichts, falls sich das Expressionsniveau der ausgewählten einen oder mehreren Gene in der Anwesenheit der Testverbindung von dem Expressionsniveau der ausgewählten einen oder mehreren Gene in der Abwesenheit der Testverbindung unterscheidet.

2. Verfahren nach Anspruch 1, wobei das Expressionsniveau der ausgewählten einen oder mehreren Gene durch Bestimmen von mRNA oder Protein bestimmt wird.

3. Verfahren nach Anspruch 1 oder 2, wobei die Zelle eine Nervenzelle und/oder eine gezüchtete Zelle ist.

4. Verfahren nach einem der vorstehenden Ansprüche, wobei die Zelle Ob Rezeptor exprimiert.

5. Verfahren nach einem der vorstehenden Ansprüche, wobei die Expression in Anwesenheit von Leptin bestimmt wird.

6. Verfahren nach einem der vorstehenden Ansprüche, wobei die geeignete Verbindung zum Erhöhen oder Senken der Nahrungsaufnahme nützlich ist.

7. Verfahren nach einem der vorstehenden Ansprüche, weiter umfassend:
e) Verabreichen der in Schritt d) ermittelten, geeigneten Verbindung an einen nicht-menschlichen Test-Säuger;
f) Beobachten des Effekts der Verbindung auf das Fressverhalten des nicht-menschlichen Test-Säugers; und
g) Ermitteln der Verbindung als eine Verbindung zum Verändern des Körpergewichts eines Säugers, falls die Verbindung das Fress-Verhalten des nicht-menschlichen Test-Säugers ändert.

8. Verfahren nach Anspruch 7, wobei der nicht-menschliche Test-Säuger Leptin nicht exprimiert und/oder der nicht-menschliche Test-Säuger exogenem Leptin ausgesetzt ist.

9. Verfahren nach Anspruch 7 oder 8, wobei der nicht-menschliche Test-Säuger eine Maus ist.

10. Verfahren nach Anspruch 9, wobei die Maus eine ob/ob Maus ist und/oder wobei die Maus eine transgene Maus ist, die ein LIG-46 Gen exprimiert, das die in Fig. 8 gezeigte Aminosäuresequenz kodiert.

11. Verfahren nach einem der Ansprüche 7 bis 10, wobei der nicht-menschliche Säuger vor der Verabreichung der geeigneten Verbindung gefüttert oder hungern gelassen wird.

12. Verfahren zu Bestimmung, ob eine Verbindung eine zur Verwendung beim Verändern des Körpergewichts geeignete Verbindung darstellt, welches umfasst:
a) Bereitstellen einer Probe, die ein oder mehrere Proteine enthält, ausgewählt aus der Gruppe bestehend aus einem LIG46 Polypeptid, das die Aminosäuresequenz von SEQ ID NO: 2 oder die in Fig. 8 gezeigte Sequenz umfasst, Tgtp, LRG-47, RC10-II und Stra13;
b) Bestimmen der Aktivität der ausgewählten einen oder mehreren Proteine in der Anwesenheit oder Abwesenheit einer Testverbindung;
c) Vergleichen der Aktivität der ausgewählten einen oder mehreren Proteine in der Anwesenheit der Testverbindungen mit der Aktivität der ausgewählten einen oder mehreren Proteine in der Abwesenheit der Testverbindung; und
d) Ermitteln der Testverbindung als eine geeignete Verbindung, die zum Verändern des Körpergewichts nützlich ist, falls sich die Aktivität der ausgewählten einen oder mehreren Proteine in der Anwesenheit der Testverbindung von der Aktivität der ausgewählten einen oder mehreren Proteine in der Abwesenheit der Testverbindung unterscheidet.

13. Verfahren nach Anspruch 12, wobei das ausgewählte Protein LIG46 ist und die bestimmte Aktivität eine Galactosyltransferase-Aktivität ist.

14. Verfahren nach Anspruch 12 oder 13, wobei die Probe eine Zelle umfasst, die eines der Proteine exprimiert, die ausgewählt sind aus der Gruppe bestehend aus einem LIG46 Polypeptid, das die Aminosäuresequenz von SEQ ID NO:2 oder die in Fig. 8 gezeigte Sequenz umfasst, Tgtp, LRG-47, RC10-II und Stra-13.

15. Verfahren nach Anspruch 14, wobei die Zelle eine Nervenzelle und/oder eine gezüchtete Zelle ist.

16. Verfahren nach Anspruch 14 oder 15, wobei die Zelle Ob Rezeptor exprimiert.

17. Verfahren nach einem der Ansprüche 12 bis 16, wobei eine Aktivität in Anwesenheit von Leptin bestimmt wird.

18. Verfahren nach einem der Ansprüche 12 bis 17, weiterhin umfassend:
e) Verabreichen der in Schritt d) ermittelten geeigneten Verbindung an einen nicht-menschlichen Test-Säuger;
f) Beobachten des Effekts der Verbindung auf das Fress-Verhalten des nicht-menschlichen Test-Säugers; und
g) Ermitteln der Verbindung als eine Verbindung zum Verändern des Körpergewichts eines Säugers, falls die Verbindung das Fress-Verhalten des nicht-menschlichen Test-Säugers ändert.

19. Verfahren nach Anspruch 18, wobei der nicht-menschliche Test-Säuger Leptin nicht exprimiert und/oder der nicht-menschliche Test-Säuger exogenem Leptin ausgesetzt ist.

20. Verfahren nach Anspruch 18 oder 19, wobei der nicht-menschliche Säuger eine Maus ist.

21. Verfahren nach Anspruch 20, wobei die Maus eine ob/ob Maus ist und/oder, wobei die Maus eine transgene Maus ist, die ein LIG-46 Gen exprimiert, das die in Fig. 8 gezeigte Aminosäuresequenz kodiert.

22. Verfahren nach einem der Ansprüche 18 bis 21, wobei der nicht-menschliche Säuger vor der Verabreichung der geeigneten Verbindung gefüttert oder hungern gelassen wird.

23. Verfahren zur Bestimmung, ob eine Verbindung eine zur Verwendung beim Verändern des Körpergewichts geeignete Verbindung darstellt, welches umfasst:
a) Bestimmen des Expressionsniveaus eines oder mehrerer Gene ausgewählt aus der Gruppe bestehend aus dem LIG46 Gen, das ein die Aminosäuresequenz von SEQ ID NO: 2 oder die in Fig. 8 gezeigte Sequenz umfassendes Polypeptid kodiert, dem LIG56 Gen, das ein die Aminosäuresequenz von SEQ ID NO: 6 umfassendes Polypeptid kodiert, dem Tgtp Gen, dem LRG-47 Gen, dem RC10-II Gen und dem Stra13 Gen in einer von einem Säuger isolierten Zellprobe, der mit der Verbindung behandelt wurde, und in einer von einem nicht-behandelten Säuger isolierten Zellprobe; und
b) Ermitteln der Testverbindung als eine geeignete Verbindung zur Verwendung beim Verändern des Körpergewichts, falls sich das Expressionsniveau der ausgewählten einen oder mehreren Gene in der von dem behandelten Säuger isolierten Zellprobe von der Expression der ausgewählten einen oder mehreren Gene in der von dem nicht-behandelten Säugetier isolierten Zellprobe unterscheidet.

24. Verfahren nach Anspruch 23, wobei die Zellen in der Probe Nervenzellen sind.

25. Verfahren nach Anspruch 23 oder 24, wobei das Säugetier eine Maus ist.

26. Verfahren zur Bestimmung, ob eine Verbindung eine zur Verwendung beim Verändern des Körpergewichts geeignete Verbindung darstellt, welches umfasst:
a) Bestimmen des Aktivitätsniveaus eines oder mehrerer Proteine ausgewählt aus der Gruppe bestehend aus einem LIG46 Polypeptid, das die Aminosäuresequenz von SEQ ID NO: 2 oder die in Fig. 8 gezeigte Sequenz umfasst, einem LIG56 Polypeptid, das die Aminosäuresequenz von SEQ ID NO: 6 umfasst, Tgtp, LRG-47, RC10-II und Stra13 Gen in einer von einem Säuger isolierten Zellprobe, der mit der Verbindung behandelt wurde, und in einer von einem nicht-behandelten Säuger isolierten Zellprobe; und
b) Ermitteln der Testverbindung als eine geeignete Verbindung, die zum Verändern des Körpergewichts nützlich ist, falls das Aktivitätsniveau der ausgewählten einen oder mehreren Proteine in der von dem behandelten Säugetier isolierten Zellprobe sich von dem Aktivitätsniveau der einen oder mehreren ausgewählten Proteine in der von dem nicht-behandelten Säugetier isolierten Zellprobe unterscheidet.

27. Verfahren nach Anspruch 26, wobei die Zellen in der Probe Nervenzellen sind.

28. Verfahren nach Anspruch 26 oder 27, wobei das Säugetier eine Maus ist.

29. Verfahren zur Bestimmung, ob eine Verbindung eine zur Verwendung beim Verändern des Körpergewichts geeignete Verbindung darstellt, welches umfasst:
a) Kombinieren einer Testverbindung mit einer Probe enthaltend ein oder mehrere Proteine ausgewählt aus der Gruppe bestehend aus einem LIG46 Polypeptid, das die Aminosäuresequenz von SEQ ID NO: 2 oder die in Fig. 8 gezeigte Sequenz umfasst, einem LIG56 Polypeptid, das die Aminosäuresequenz von SEQ ID NO: 6 umfasst, Tgtp, LRG-47, RC10-II und Stra13 Gen unter zum Binden geeigneten Bedingungen; und
b) Bestimmen eines Bindens der ausgewählten einen oder mehreren Proteine in der Probe an die Testverbindung; und
b) Ermitteln der Testverbindung als eine geeignete Verbindung, die zum Verändern des Körpergewichts nützlich ist, falls die Testverbindung an die ausgewählten einen oder mehreren Proteine bindet.

30. Verfahren nach Anspruch 29, wobei die ausgewählten einen oder mehreren Proteine an einen festen Träger gebunden sind.

31. Verfahren nach Anspruch 29, wobei die Probe eine Zelle umfasst, die die ausgewählten ein oder mehreren Proteine exprimiert.

32. Verfahren nach Anspruch 31, wobei die Zelle eine gezüchtete Zelle und/oder eine Nervenzelle ist.

33. Verfahren nach einem der Ansprüche 29 bis 32, wobei die Testverbindung nachweisbar markiert ist.

34. Verfahren nach einem der Ansprüche 29 bis 33, wobei ein Binden in der Anwesenheit von Leptin bestimmt wird.

## Revendications

1. Procédé pour déterminer le fait de savoir si un composé est un composé candidat à utiliser pour moduler le poids corporel, le procédé comprenant le fait de :
a) procurer une cellule contenant un ou plusieurs gènes choisis parmi le groupe constitué par le gène LIG46 qui encode un polypeptide comprenant la séquence d'acides aminés SEQ ID NO : 2 ou la séquence représentée en figure 8, le gène LIG56 qui encode un polypeptide comprenant la séquence d'acides aminés SEQ ID NO : 6, le gène Tgtp, le gène LRG-47, le gène RC10-II, et le gène Stra13 ;
b) mesurer le taux d'expression desdits un ou plusieurs gènes sélectionnés en présence et en l'absence d'un composé d'essai ;
c) comparer le taux d'expression desdits un ou plusieurs gènes sélectionnés en présence du composé d'essai au taux d'expression desdits un ou plusieurs gènes sélectionnés en l'absence du composé d'essai ; et
d) identifier le composé d'essai comme étant un composé candidat à utiliser dans la modulation du poids corporel lorsque le taux d'expression desdits un ou plusieurs gènes sélectionnés en présence du composé d'essai diffère du taux d'expression desdits un ou plusieurs gènes sélectionnés en l'absence du composé d'essai.

2. Procédé selon la revendication 1, dans lequel le taux d'expression desdits un ou plusieurs gènes sélectionnés est mesuré en mesurant l'ARN ou la teneur en protéines.

3. Procédé selon la revendication 1 ou 2, dans lequel la cellule est une cellule neurale et/ou une cellule cultivée.

4. Procédé selon l'une quelconque des revendications précédentes, dans lequel la cellule exprime le récepteur Ob.

5. Procédé selon l'une quelconque des revendications précédentes, dans lequel l'expression est mesurée en présence de leptine.

6. Procédé selon l'une quelconque des revendications précédentes, dans lequel le composé candidat est utile pour augmenter ou diminuer la ration alimentaire.

7. Procédé selon l'une quelconque des revendications précédentes, comprenant en outre le fait de :
e) administrer le composé candidat identifié à l'étape d) à un mammifère d'essai non humain ;
f) surveiller l'effet du composé sur le comportement alimentaire du mammifère d'essai non humain ; et
g) identifier le composé comme étant un composé destiné à moduler le poids corporel d'un mammifère lorsque le composé modifie le comportement alimentaire du mammifère d'essai non humain.

8. Procédé selon la revendication 7, dans lequel le mammifère d'essai non humain n'exprime pas la leptine et/ou le mammifère d'essai non humain est exposé à de la leptine exogène.

9. Procédé selon la revendication 7 ou 8, dans lequel le mammifère d'essai non humain est une souris.

10. Procédé selon la revendication 9, dans lequel la souris est une souris ob/ob et/ou dans lequel la souris est une souris transgénique exprimant un gène LIG46 encodant la séquence d'acides aminés représentée en figure 8.

11. Procédé selon l'une quelconque des revendications 7 à 10, dans lequel le mammifère d'essai non humain est alimenté ou privé de nourriture avant l'administration du composé candidat.

12. Procédé pour déterminer le fait de savoir si un composé est un composé candidat à utiliser pour moduler le poids corporel, le procédé comprenant le fait de :
a) procurer un échantillon comprenant une ou plusieurs protéines choisies parmi le groupe constitué par un polypeptide LIG46 comprenant la séquence d'acides aminés SEQ ID NO: 2 ou la séquence représentée en figure 8, Tgtp, LRG-47, RC10-II et Stra13 ;
b) mesurer l'activité desdites une ou plusieurs protéines sélectionnées en présence et en l'absence d'un composé d'essai ;
c) comparer l'activité desdites une ou plusieurs protéines sélectionnées en présence du composé d'essai à l'activité desdites une ou plusieurs protéines sélectionnées en l'absence du composé d'essai ; et
d) identifier le composé d'essai comme étant un composé candidat à utiliser dans la modulation du poids corporel lorsque l'activité desdites une ou plusieurs protéines sélectionnées en présence du composé d'essai diffère de l'activité desdites une ou plusieurs protéines sélectionnées en l'absence du composé d'essai.

13. Procédé selon la revendication 12, dans lequel la protéine sélectionnée est la protéine LIG46 et l'activité mesurée est l'activité de galactosyltransférase.

14. Procédé selon la revendication 12 ou 13, dans lequel l'échantillon comprend une cellule exprimant une des protéines choisies parmi le groupe constitué par un polypeptide LIG46 comprenant la séquence d'acides aminés SEQ ID NO: 2 ou la séquence représentée en figure 8, Tgtp, LRG-47, RC10-II et Stra13.

15. Procédé selon la revendication 14, dans lequel la cellule est une cellule neurale et/ou une cellule cultivée.

16. Procédé selon la revendication 14 ou 15, dans lequel la cellule exprime le récepteur Ob.

17. Procédé selon l'une quelconque des revendications 12 à 16, dans lequel l'activité est mesurée en présence de leptine.

18. Procédé selon l'une quelconque des revendications 12 à 17, comprenant en outre le fait de :
e) administrer le composé candidat identifié à l'étape d) à un mammifère d'essai non humain ;
f) surveiller l'effet du composé sur le comportement alimentaire du mammifère d'essai non humain ; et
g) identifier le composé comme étant un composé destiné à moduler le poids corporel d'un mammifère lorsque le composé modifie le comportement alimentaire du mammifère d'essai non humain.

19. Procédé selon la revendication 18, dans lequel le mammifère d'essai non humain n'exprime pas la leptine et/ou le mammifère d'essai non humain est exposé à de la leptine exogène.

20. Procédé selon la revendication 18 ou 19, dans lequel le mammifère d'essai non humain est une souris.

21. Procédé selon la revendication 20, dans lequel la souris est une souris ob/ob et/ou dans lequel la souris est une souris transgénique exprimant un gène LIG46 encodant la séquence d'acides aminés représentée en figure 8.

22. Procédé selon l'une quelconque des revendications 18 à 21, dans lequel le mammifère d'essai non humain est alimenté ou privé de nourriture avant l'administration du composé candidat.

23. Procédé pour déterminer le fait de savoir si un composé est un composé candidat à utiliser pour moduler le poids corporel, le procédé comprenant le fait de :
a) mesurer le taux d'expression d'un ou de plusieurs gènes choisis parmi le groupe constitué par le gène LIG46 qui encode un polypeptide comprenant la séquence d'acides aminés SEQ ID NO : 2 ou la séquence représentée en figure 8, le gène LIG56 qui encode un polypeptide comprenant la séquence d'acides aminés SEQ ID NO : 6, le gène Tgtp, le gène LRG-47, le gène RC10-II, et le gène Stra13 dans un échantillon de cellules isolées d'un mammifère traité avec le composé et dans un échantillon de cellules isolées d'un mammifère non traité ; et
b) identifier le composé d'essai comme étant un composé candidat utile pour la modulation du poids corporel lorsque le taux d'expression desdits un ou plusieurs gènes sélectionnés dans l'échantillon de cellules isolées du mammifère traité diffère de l'expression desdits un ou plusieurs gènes sélectionnés dans l'échantillon de cellules isolées du mammifère non traité.

24. Procédé selon la revendication 23, dans lequel les cellules dans l'échantillon sont des cellules neurales.

25. Procédé selon un revendication 23 ou 24, dans lequel le mammifère est une souris.

26. Procédé pour déterminer le fait de savoir si un composé est un composé candidat à utiliser pour moduler le poids corporel, le procédé comprenant le fait de :
a) mesurer le taux d'activité d'une ou de plusieurs protéines choisies parmi le groupe constitué par un polypeptide LIG46 comprenant la séquence d'acides aminés SEQ ID NO : 2 ou la séquence représentée en figure 8, un polypeptide LIG56 comprenant la séquence d'acides aminés SEQ ID NO : 6, Tgtp, LRG-47, RC10-II, et Stra13 dans un échantillon de cellules isolées d'un mammifère traité avec un composé d'essai et dans un échantillon de cellules isolées d'un mammifère non traité ; et
b) identifier le composé d'essai comme étant un composé candidat utile pour la modulation du poids corporel lorsque le taux d'activité desdites une ou plusieurs protéines sélectionnées dans l'échantillon de cellules isolées du mammifère traité diffère du taux d'activité desdites une ou plusieurs protéines sélectionnées dans l'échantillon de cellules isolées du mammifère non traité.

27. Procédé selon la revendication 26, dans lequel les cellules dans l'échantillon sont des cellules neurales.

28. Procédé selon un revendication 26 ou 27, dans lequel ledit mammifère est une souris.

29. Procédé pour déterminer le fait de savoir si un composé est un composé candidat à utiliser pour moduler le poids corporel, le procédé comprenant le fait de :
a) combiner un composé d'essai avec un échantillon comprenant une ou plusieurs protéines choisies parmi le groupe constitué par un polypeptide LIG46 comprenant la séquence d'acides aminés SEQ ID NO : 2 ou la séquence représentée en figure 8, un polypeptide LIG56 comprenant la séquence d'acides aminés SEQ ID NO : 6, Tgtp, LRG-47, RC10-II, et Stra13, dans des conditions appropriées pour une liaison ;
b) mesurer la liaison desdites une ou plusieurs protéines sélectionnées dans l'échantillon au composé d'essai ; et
c) identifier le composé d'essai comme étant un composé candidat utile pour la modulation du poids corporel lorsque le composé d'essai se lie auxdites une ou plusieurs protéines sélectionnées.

30. Procédé selon la revendication 29, dans lequel lesdites une ou plusieurs protéines sélectionnées sont liées à un support solide.

31. Procédé selon la revendication 29, dans lequel l'échantillon comprend une cellule exprimant lesdites une ou plusieurs protéines sélectionnées.

32. Procédé selon la revendication 31, dans lequel la cellule est une cellule cultivée et/ou une cellule neurale.

33. Procédé selon l'une quelconque des revendications 29 à 32, dans lequel le composé d'essai est marqué pour être détecté.

34. Procédé selon l'une quelconque des revendications 29 à 33, dans lequel la liaison est mesurée en présence de leptine.
